(19) Europäisches Patentamt
European Patent Office
Office européen des brevets

(11) EP 4 174 064 A1

(12) EUROPEAN PATENT APPLICATION
published in accordance with Art. 153(4) EPC

(43) Date of publication:
**03.05.2023 Bulletin 2023/18**

(21) Application number: **21832182.6**

(22) Date of filing: **30.06.2021**

(51) International Patent Classification (IPC):
***C07D 413/12*** (2006.01) ***A61P 3/10*** (2006.01)
***A61P 35/00*** (2006.01) ***C07D 401/12*** (2006.01)
***C07D 405/14*** (2006.01) ***C07D 413/14*** (2006.01)
***A61K 31/428*** (2006.01) ***A61P 7/06*** (2006.01)
***C07D 405/12*** (2006.01) ***A61K 31/4439*** (2006.01)
***C07D 471/04*** (2006.01) ***C07D 471/08*** (2006.01)
***A61K 31/502*** (2006.01) ***A61K 31/517*** (2006.01)

(52) Cooperative Patent Classification (CPC):
**A61K 31/428; A61K 31/4439; A61K 31/502; A61K 31/517; A61P 3/10; A61P 7/06; A61P 35/00; C07D 401/12; C07D 405/12; C07D 405/14; C07D 413/12; C07D 413/14; C07D 471/04; C07D 471/08**

(86) International application number:
**PCT/CN2021/103597**

(87) International publication number:
**WO 2022/002142 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2020 CN 202010614735**
**19.03.2021 CN 202110296535**

(71) Applicant: **Jiangsu Simcere Pharmaceutical Co., Ltd.**
**Nanjing, Jiangsu 210042 (CN)**

(72) Inventors:
- **LIU, Lei**
  **Shanghai 201321 (CN)**
- **LIU, Yang**
  **Shanghai 201321 (CN)**
- **ZHAO, Chunyan**
  **Shanghai 201321 (CN)**
- **TANG, Renhong**
  **Shanghai 201321 (CN)**
- **REN, Jinsheng**
  **Nanjing, Jiangsu 210042 (CN)**

(74) Representative: **Grünecker Patent- und Rechtsanwälte**
**PartG mbB**
**Leopoldstraße 4**
**80802 München (DE)**

# (54) TETRAHYDROISOQUINOLINE COMPOUNDS AND USE THEREOF

(57) The present application describes tetrahydroisoquinoline compounds as PRMT5 inhibitors and pharmaceutically acceptable salts thereof. Said compounds have the structure of formula (I), and have substituents and structural features described in the present application. The present application also describes pharmaceutical compositions comprising the compounds of formula (I) or pharmaceutically acceptable salts thereof and use of the compounds of formula (I) or pharmaceutically acceptable salts thereof and the pharmaceutical composition comprising the same in the preparation of medicaments for preventing or treating diseases mediated by PRMT5.

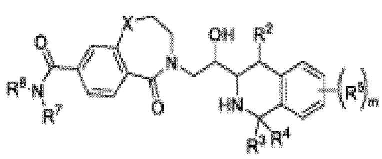

(I)

EP 4 174 064 A1

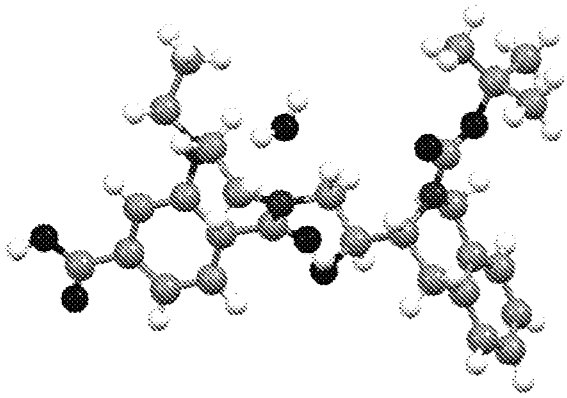

*Fig. 1*

## Description

The present application claims the priority of the following applications: patent application No. 202010614735.9 entitled "Tetrahydroisoquinoline Compounds and Use Thereof' and filed with the China National Intellectual Property Administration on June 30, 2020 and patent application No. 202110296535.8 entitled "Tetrahydroisoquinoline Compounds and Use Thereof' and filed with the China National Intellectual Property Administration on March 19, 2021, which are incorporated into the present application in their entireties by reference.

## Technical Field

The present disclosure relates to tetrahydroisoquinoline compounds as PRMT5 inhibitors, a preparation method thereof, pharmaceutical compositions comprising the compounds and the use thereof in the treatment of diseases mediated by PRMT5 or associated with PRMT5.

## Background

Epigenetic alterations are key mediators driving and maintaining the malignant phenotype of tumors. DNA methylation, histone acetylation and methylation, non-coding RNAs, and changes in post-translational modification, rather than changes in DNA sequences, are all epigenetic drivers of cancer development. Arginine methylation is an important class of post-translational modifications that affect cell growth and proliferation, apoptosis, angiogenesis and metastasis by regulating transcription and post-transcriptional RNA processing. There are three types of methylarginines: ω-NG-monomethylarginine (MMA), ω-NG,N'G-asymmetric dimethylarginine (ADMA), and ω-NG,N'G-symmetric dimethylarginine (SDMA). Such modifications are to transfer a methyl group from S-adenosylmethionine (AdoMet) to the arginine side chain of histone and nonhistone under the catalysis of the protein arginine methyltransferase (PRMT) family. Nine PRMT genes are annotated in the human genome and based on the methylarginine types produced, these genes are grouped into type I (PRMT1, 2, 3, 4, 6 and 8), type II (PRMT5 and PRMT9), and type III enzymes (PRMT7). PRMT5 is a major type II enzyme, which can catalyze the symmetric dimethylation of arginine. PRMT5 was first discovered in two-hybrid experiment for detecting proteins having interactions with the Janus tyrosine kinase (Jak2).

PRMT5 is a general transcriptional factor and can form complexes with other transcriptional factors, including BRG1 and hBRM, Blimp1 and Snail. PRMT5 is implicated with many different cellular biological processes through methylating many substrates within the cytoplasm and nucleus, including residue Arg3 of histone H4 (H4R3) and residue Arg8 of H3 (H3R8). H4R3 methylation is associated with transcriptional repression, while H3R8 methylation is considered to be associated with both transcriptional activation and transcriptional repression. In addition to directly inducing inhibitory histone labeling, the role of the enzyme PRMT5 in gene silencing is also mediated by forming multiple repressive protein complexes, including NuRD components, HDACs, MDB proteins, and DNA methyltranseferases. The substrate specificity of PRMT5 is affected through its interaction with some binding proteins. The core component of such protein complexes is MEP50. MEP50 is necessary to the enzymatic activity of PRMT5. Studies have found that PRMT5 can methylate proteins involved in RNA splicing, such as SmD3, which hence can be used to monitor the chemical activity of PRMT5 in cell organism.

PRMT5 plays an important role in tumorigenesis. Studies have found that the expression of PRMT5 is up-regulated in many tumors, including lymphoma, lung cancer, breast cancer and colorectal cancer. Furthermore, the expression of PRMT5 is increased in the samples from mantle cell lymphoma (MCL) patients, while PRMT5 knockout can inhibit the cell proliferation of MCL, indicating that PRMT5 plays an important role in MCL. PRMT5 over-expression promotes cell proliferation, and in cell lines of melanoma, breast cancer and lung cancer, PRMT5 knockout can inhibit the proliferation of the cells. Therefore, PRMT5 is a potential target for cancer treatment.

Loss of methylthioadenosine phosphorylase (MTAP) endows cells with selective dependence on PRMT5 and its binding protein WDR77. MTAP is often lost due to its proximity to the normally deleted tumor suppressor gene CDKN2A. Cells carrying MTAP deletion have an increased concentration of intracellular methylthioadenosine (MTA, a metabolite cleaved by MTAP). Furthermore, MTA specifically inhibits the enzyme activity of PRMT5. MTA or small-molecule inhibitors of PRMT5 significantly inhibit the cell viability of cancer cell lines having MTAP deletion relative to cells expressing MTAP.

Therefore, there is a need in the art to develop active small-molecule compounds that can inhibit the activity of PRMT5 and can treat various diseases associated with PRMT5.

## Summary

The present disclosure provides a compound of formula (I) or a pharmaceutically acceptable salt thereof,

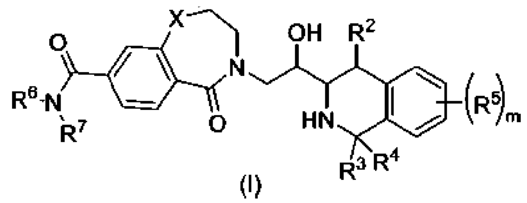

(I)

wherein

X is selected from $CR^8R^9$, $NR^1$, or O;

$R^1$ is selected from H, $C_1$-$C_{10}$ alkyl, $C_2$-$C_{10}$ alkenyl, $C_2$-$C_{10}$ alkynyl, $C_6$-$C_{10}$ aryl, 5- to 10-membered heteroaryl, $C_3$-$C_{10}$ cycloalkyl, or 3- to 10-membered heterocyclyl, wherein $C_1$-$C_{10}$ alkyl, $C_2$-$C_{10}$ alkenyl, $C_2$-$C_{10}$ alkynyl, $C_6$-$C_{10}$ aryl, 5- to 10-membered heteroaryl, $C_3$-$C_{10}$ cycloalkyl, or 3- to 10-membered heterocyclyl is optionally substituted with one or more $R^a$;

$R^2$ and $R^5$ are independently selected from H, halogen, CN, or the following groups which are optionally substituted with one or more $R^b$: $NH_2$, $C_1$-$C_{10}$ alkyl, $C_1$-$C_{10}$ alkoxy, $C_3$-$C_{10}$ cycloalkyl, or 3- to 10-membered heterocyclyl;

$R^3$ and $R^4$ are independently selected from H, deuterium, halogen, CN, or the following groups which are optionally substituted with one or more $R^b$: $NH_2$, $C_1$-$C_{10}$ alkyl, $C_1$-$C_{10}$ alkoxy, $C_3$-$C_{10}$ cycloalkyl, or 3- to 10-membered heterocyclyl;

or, $R^3$ and $R^4$ together with the C to which they are attached form $C_3$-$C_{10}$ cycloalkyl or 3-to 10-membered heterocyclyl, wherein the $C_3$-$C_{10}$ cycloalkyl or 3- to 10-membered heterocyclyl is optionally substituted with one or more $R^e$;

$R^8$ and $R^9$ are independently selected from H, OH, CN, $NO_2$, $C_1$-$C_{10}$ alkyl, $C_1$-$C_{10}$ alkoxy, $C_3$-$C_{10}$ cycloalkyl, or 3- to 10-membered heterocyclyl, wherein the $C_1$-$C_{10}$ alkyl, $C_1$-$C_{10}$ alkoxy, $C_3$-$C_{10}$ cycloalkyl, or 3- to 10-membered heterocyclyl is optionally substituted with one or more $R^f$;

m is selected from 0, 1, 2, 3 or 4;

$R^a$ is selected from halogen, =O, OH, CN, $NO_2$, $C_1$-$C_{10}$ alkyl, $C_1$-$C_{10}$ alkoxy, $C_6$-$C_{10}$ aryl, 5- to 10-membered heteroaryl, $C_3$-$C_{10}$ cycloalkyl, or 3- to 10-membered heterocyclyl;

$R^b$ is selected from halogen, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;

$R^e$ and $R^f$ are independently selected from halogen, =O, OH, CN, $NO_2$, $C_1$-$C_{10}$ alkyl, or $C_1$-$C_{10}$ alkoxy;

$R^6$ and $R^7$ are independently selected from H, $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl, wherein the $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl is optionally substituted with one or more $R^c$;

$R^c$ is selected from halogen, OH, CN, =O, $C_1$-$C_{10}$ alkoxy, $C_6$-$C_{10}$ aryl, $C_6$-$C_{10}$ aryloxy, 5- to 10-membered heteroaryl, 5- to 10-membered heteroaryloxy, $C_3$-$C_{10}$ cycloalkyl, $C_3$-$C_{10}$ cycloalkyloxy, 3- to 10-membered heterocyclyl, or 3- to 10-membered heterocyclyloxy, wherein the $C_1$-$C_{10}$ alkoxy, $C_6$-$C_{10}$ aryl, $C_6$-$C_{10}$ aryloxy, 5- to 10-membered heteroaryl, 5-to 10-membered heteroaryloxy, $C_3$-$C_{10}$ cycloalkyl, $C_3$-$C_{10}$ cycloalkyloxy, 3- to 10-membered heterocyclyl, or 3- to 10-membered heterocyclyloxy is optionally substituted with one or more $R^{c1}$;

$R^{c1}$ is selected from halogen, OH, =O, CN, $NO_2$, $C_1$-$C_{10}$ alkyl, or $C_1$-$C_{10}$ alkoxy;

or, $R^6$ and $R^7$ together with the N to which they are attached form 6- to 13-membered spiroheterocycloalkyl, 6- to 13-membered fused heterocycloalkyl, 6- to 13-membered bridged heterocycloalkyl, 3- to 8-membered monocyclic heterocycloalkyl, or 5- to 10-membered heteroaryl, wherein the 6- to 13-membered spiroheterocycloalkyl, 6- to 13-membered fused heterocycloalkyl, 6- to 13-membered bridged heterocycloalkyl, 3- to 8-membered monocyclic heterocycloalkyl, or 5- to 10-membered heteroaryl is optionally substituted with one or more $R^d$;

$R^d$ is selected from halogen, OH, =O, CN, $NO_2$, $C_1$-$C_{10}$ alkyl, $C_1$-$C_{10}$ alkoxy, $C_1$-$C_{10}$ alkyl-NH-, $(C_1$-$C_{10}$ alkyl$)_2$-N-, $C_6$-$C_{10}$ aryl, 5- to 10-membered heteroaryl, $C_3$-$C_{10}$ cycloalkyl, $C_3$-$C_{10}$ cycloalkyloxy, $C_3$-$C_{10}$ cycloalkyl-NH-, $(C_3$-$C_{10}$ cycloalkyl$)_2$-N-, or 3- to 10-membered heterocyclyl, wherein the $C_1$-$C_{10}$ alkyl, $C_1$-$C_{10}$ alkoxy, $C_1$-$C_{10}$ alkyl-NH-, $(C_1$-$C_{10}$ alkyl$)_2$-N-, $C_6$-$C_{10}$ aryl, 5- to 10-membered heteroaryl, $C_3$-$C_{10}$ cycloalkyl, $C_3$-$C_{10}$ cycloalkyloxy, $C_3$-$C_{10}$ cycloalkyl-NH-, $(C_3$-$C_{10}$ cycloalkyl$)_2$-N-, or 3- to 10-membered heterocyclyl is optionally substituted with one or more $R^{d1}$;

$R^{d1}$ is selected from deuterium, halogen, OH, =O, CN, $NO_2$, or $C_1$-$C_{10}$ alkoxy.

In some embodiments, $R^6$ and $R^7$ are independently selected from H, $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl, wherein the $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl is optionally substituted with one or more $R^c$; $R^c$ is selected from halogen, OH, CN, =O, $C_1$-$C_{10}$ alkoxy, $C_6$-$C_{10}$ aryl, $C_6$-$C_{10}$ aryloxy, 5- to 10-membered

heteroaryl, 5- to 10-membered heteroaryloxy, $C_3$-$C_{10}$ cycloalkyl, $C_3$-$C_{10}$ cycloalkyloxy, 3- to 10-membered heterocyclyl, or 3- to 10-membered heterocyclyloxy, wherein the $C_1$-$C_{10}$ alkoxy, $C_6$-$C_{10}$ aryl, $C_6$-$C_{10}$ aryloxy, 5- to 10-membered heteroaryl, 5-to 10-membered heteroaryloxy, $C_3$-$C_{10}$ cycloalkyl, $C_3$-$C_{10}$ cycloalkyloxy, 3- to 10-membered heterocyclyl, or 3- to 10-membered heterocyclyloxy is optionally substituted with one or more $R^{c1}$; $R^{c1}$ is selected from halogen, OH, =O, CN, $NO_2$, $C_1$-$C_{10}$ alkyl, or $C_1$-$C_{10}$ alkoxy; or, $R^6$ and $R^7$ together with the N to which they are attached form 6- to 13-membered spiroheterocycloalkyl, 6- to 13-membered bridged heterocycloalkyl, 3- to 8-membered monocyclic heterocycloalkyl, or 5- to 10-membered heteroaryl, wherein the 6-to 13-membered spiroheterocycloalkyl, 6- to 13-membered bridged heterocycloalkyl, 3- to 8-membered monocyclic heterocycloalkyl, or 5- to 10-membered heteroaryl is optionally substituted with one or more $R^d$; $R^d$ is selected from halogen, OH, =O, CN, $NO_2$, $C_1$-$C_{10}$ alkyl, $C_1$-$C_{10}$ alkoxy, $C_1$-$C_{10}$ alkyl-NH-, $(C_1$-$C_{10}$ alkyl$)_2$-N-, $C_6$-$C_{10}$ aryl, 5- to 10-membered heteroaryl, $C_3$-$C_{10}$ cycloalkyl, $C_3$-$C_{10}$ cycloalkyloxy, $C_3$-$C_{10}$ cycloalkyl-NH-, $(C_3$-$C_{10}$ cycloalkyl$)_2$-N-, or 3- to 10-membered heterocyclyl, wherein the $C_1$-$C_{10}$ alkyl, $C_1$-$C_{10}$ alkoxy, $C_6$-$C_{10}$ aryl, 5- to 10-membered heteroaryl, $C_3$-$C_{10}$ cycloalkyl, or 3- to 10-membered heterocyclyl is optionally substituted with one or more $R^{d1}$; $R^{d1}$ is selected from halogen, OH, =O, CN, $NO_2$, or $C_1$-$C_{10}$ alkoxy.

In some embodiments, $R^1$ is selected from H, $C_1$-$C_{10}$ alkyl, $C_6$-$C_{10}$ aryl, 5- to 10-membered heteroaryl, $C_3$-$C_{10}$ cycloalkyl or 3- to 10-membered heterocyclyl, wherein the $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl is optionally substituted with one or more $R^a$.

In some embodiments, $R^1$ is selected from H, and $C_1$-$C_{10}$ alkyl optionally substituted with one or more $R^a$.

In some embodiments, $R^1$ is selected from H, $CH_3$, or $CH_2CH_3$.

In some embodiments, $R^1$ is selected from $CH_3$ or $CH_2CH_3$.

In some embodiments, $R^2$, $R^3$, $R^4$ and $R^5$ are independently selected from H, halogen, CN, or the following groups which are optionally substituted with one or more $R^b$: $NH_2$, $C_1$-$C_{10}$ alkyl, $C_1$-$C_{10}$ alkoxy, $C_3$-$C_{10}$ cycloalkyl, or 3- to 10-membered heterocyclyl; or, $R^3$ and $R^4$ together with the C to which they are attached form $C_3$-$C_{10}$ cycloalkyl or 3- to 10-membered heterocyclyl, wherein the $C_3$-$C_{10}$ cycloalkyl or 3- to 10-membered heterocyclyl is optionally substituted with one or more $R^e$.

In some embodiments, $R^2$, $R^3$, $R^4$ and $R^5$ are independently selected from H, halogen, CN, $C_1$-$C_{10}$ alkyl, $C_1$-$C_{10}$ alkoxy, $C_3$-$C_{10}$ cycloalkyl, or 3- to 10-membered heterocyclyl, wherein the $C_1$-$C_{10}$ alkyl, $C_1$-$C_{10}$ alkoxy, $C_3$-$C_{10}$ cycloalkyl, or 3- to 10-membered heterocyclyl is optionally substituted with one or more $R^b$.

In some embodiments, $R^2$, $R^3$, $R^4$ and $R^5$ are independently selected from H, halogen, CN, $C_1$-$C_{10}$ alkyl, or $C_1$-$C_{10}$ alkoxy.

In some embodiments, $R^2$, $R^3$, $R^4$ and $R^5$ are all H.

In some embodiments, $R^8$ and $R^9$ are independently selected from H, OH, CN, $NO_2$, $C_1$-$C_{10}$ alkyl, or $C_1$-$C_{10}$ alkoxy.

In some embodiments, X is selected from $NR^1$ or O.

In some embodiments, X is selected from $NCH_3$, $NCH_2CH_3$, or O.

In some embodiments, m is selected from 0, 1 or 2.

In some embodiments, m is selected from 0 or 1.

In some embodiments, m is selected from 0.

In some embodiments, $R^6$ and $R^7$ are independently selected from H, $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl, wherein the $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl is optionally substituted with one or more $R^c$.

In some embodiments, $R^6$ and $R^7$ are independently selected from H, $C_1$-$C_{10}$ alkyl, $C_3$-$C_{10}$ cycloalkyl, or 3- to 10-membered heterocyclyl, wherein the $C_1$-$C_{10}$ alkyl, $C_3$-$C_{10}$ cycloalkyl, or 3- to 10-membered heterocyclyl is optionally substituted with one or more $R^c$.

In some embodiments, $R^6$ and $R^7$ are independently selected from H, or $C_1$-$C_{10}$ alkyl optionally substituted with one or more $R^c$.

In some embodiments, $R^c$ is selected from halogen, OH, $C_6$-$C_{10}$ aryl, 5- to 10-membered heteroaryl, $C_3$-$C_{10}$ cycloalkyl, or 3- to 10-membered heterocyclyl, wherein the $C_6$-$C_{10}$ aryl, 5- to 10-membered heteroaryl, $C_3$-$C_{10}$ cycloalkyl, or 3- to 10-membered heterocyclyl is optionally substituted with one or more $R^{c1}$.

In some embodiments, $R^c$ is selected from halogen, OH, $C_6$-$C_{10}$ aryl, or 5- to 10-membered heteroaryl, wherein the $C_6$-$C_{10}$ aryl, or 5- to 10-membered heteroaryl is optionally substituted with one or more $R^{c1}$.

In some embodiments, $R^c$ is selected from $C_6$-$C_{10}$ aryl, or 5- to 10-membered heteroaryl, wherein the $C_6$-$C_{10}$ aryl, or 5- to 10-membered heteroaryl is optionally substituted with one or more $R^{c1}$.

In some embodiments, $R^c$ is selected from phenyl or pyridyl optionally substituted with one or more $R^{c1}$.

In some embodiments, $R^{c1}$ is selected from halogen, OH, =O, CN, or $C_1$-$C_{10}$ alkyl.

In some embodiments, $R^{c1}$ is selected from halogen.

In some embodiments, $R^{c1}$ is selected from F.

In some embodiments, $R^6$ and $R^7$ are independently selected from $CH_3$, $CH_2CH_3$, benzyl, 4-fluorophenethyl,

or pyridine-3-ethyl.

In some embodiments, $R^6$ and $R^7$ together with the N to which they are attached form 6- to 13-membered spiroheterocycloalkyl, 6- to 13-membered fused heterocycloalkyl, 6- to 13-membered bridged heterocycloalkyl, 3- to 8-membered monocyclic heterocycloalkyl, or 5- to 10-membered heteroaryl, wherein the 6- to 13-membered spiroheterocycloalkyl, 6- to 13-membered bridged heterocycloalkyl, 3- to 8-membered monocyclic heterocycloalkyl, or 5- to 10-membered heteroaryl is optionally substituted with one or more $R^d$.

In some embodiments, $R^d$ is selected from halogen, OH, =O, CN, $NO_2$, $C_1$-$C_{10}$ alkyl, $C_1$-$C_{10}$ alkoxy, $C_1$-$C_{10}$ alkyl-NH-, ($C_1$-$C_{10}$ alkyl)$_2$-N-, $C_6$-$C_{10}$ aryl, 5- to 10-membered heteroaryl, $C_3$-$C_{10}$ cycloalkyl, $C_3$-$C_{10}$ cycloalkyloxy, $C_3$-$C_{10}$ cycloalkyl-NH-, ($C_3$-$C_{10}$ cycloalkyl)$_2$-N-, or 3- to 10-membered heterocyclyl, wherein the $C_1$-$C_{10}$ alkyl, $C_1$-$C_{10}$ alkoxy, $C_6$-$C_{10}$ aryl, 5- to 10-membered heteroaryl, $C_3$-$C_{10}$ cycloalkyl, or 3- to 10-membered heterocyclyl is optionally substituted with one or more $R^{d1}$.

In some embodiments, $R^d$ is selected from halogen, OH, =O, $NO_2$, CN, $C_1$-$C_{10}$ alkyl, $C_1$-$C_{10}$ alkoxy, $C_1$-$C_{10}$ alkyl-NH-, or ($C_1$-$C_{10}$ alkyl)$_2$-N-, wherein the $C_1$-$C_{10}$ alkyl, $C_1$-$C_{10}$ alkoxy, $C_1$-$C_{10}$ alkyl-NH-, or ($C_1$-$C_{10}$ alkyl)$_2$-N- is optionally substituted with one or more $R^{d1}$.

In some embodiments, $R^d$ is selected from halogen, OH, $NO_2$, CN, $C_1$-$C_{10}$ alkyl, $C_1$-$C_{10}$ alkoxy, $C_1$-$C_{10}$ alkyl-NH-, or ($C_1$-$C_{10}$ alkyl)$_2$-N-, wherein the $C_1$-$C_{10}$ alkyl, $C_1$-$C_{10}$ alkoxy, $C_1$-$C_{10}$ alkyl-NH-, or ($C_1$-$C_{10}$ alkyl)$_2$-N- is optionally substituted with one or more $R^{d1}$.

In some embodiments, $R^d$ is selected from halogen, OH, =O, CN, $C_1$-$C_{10}$ alkyl, $C_1$-$C_{10}$ alkoxy, $C_1$-$C_{10}$ alkyl-NH-, or ($C_1$-$C_{10}$ alkyl)$_2$-N-, wherein the $C_1$-$C_{10}$ alkyl, $C_1$-$C_{10}$ alkoxy, $C_1$-$C_{10}$ alkyl-NH-, or ($C_1$-$C_{10}$ alkyl)$_2$-N- is optionally substituted with one or more $R^{d1}$.

In some embodiments, $R^d$ is selected from halogen, CN, $C_1$-$C_{10}$ alkyl, or $C_1$-$C_{10}$ alkoxy, wherein the $C_1$-$C_{10}$ alkyl, or $C_1$-$C_{10}$ alkoxy is optionally substituted with one or more $R^{d1}$.

In some embodiments, $R^{d1}$ is selected from halogen, OH, =O, CN, $NO_2$, or $C_1$-$C_{10}$ alkoxy.

In some embodiments, $R^{d1}$ is selected from halogen, OH, =O, CN, or $NO_2$.

In some embodiments, $R^{d1}$ is selected from halogen or =O.

In some embodiments, $R^{d1}$ is selected from deuterium or =O.

In some embodiments, $R^d$ is selected from halogen, OH, =O, $NO_2$, CN, $C_1$-$C_{10}$ alkyl, $C_1$-$C_{10}$ alkoxy, $C_1$-$C_{10}$ alkyl-NH-, or ($C_1$-$C_{10}$ alkyl)$_2$-N-.

In some embodiments, $R^d$ is selected from halogen, OH, $NO_2$, CN, $C_1$-$C_{10}$ alkyl, $C_1$-$C_{10}$ alkoxy, $C_1$-$C_{10}$ alkyl-NH-, or ($C_1$-$C_{10}$ alkyl)$_2$-N-.

In some embodiments, $R^d$ is selected from halogen, OH, =O, CN, $C_1$-$C_{10}$ alkyl, $C_1$-$C_{10}$ alkoxy, $C_1$-$C_{10}$ alkyl-NH-, or ($C_1$-$C_{10}$ alkyl)$_2$-N-.

In some embodiments, $R^d$ is selected from halogen, CN, $C_1$-$C_{10}$ alkyl, or $C_1$-$C_{10}$ alkoxy.

In some embodiments, $R^d$ is selected from F, =O, OH, $N(CH_3)_2$, CN, $C(O)CH_3$, $CH_2CH_3$, $OCH_3$, or $OCD_3$.

In some embodiments, $R^d$ is selected from F, =O, OH, $N(CH_3)_2$, CN, $C(O)CH_3$, $OCH_3$, or $OCD_3$.

In some embodiments, $R^d$ is selected from F, =O, OH, $N(CH_3)_2$, CN, $C(O)CH_3$, or $OCH_3$.

In some embodiments, $R^d$ is selected from F, =O, OH, $N(CH_3)_2$, CN, $CH_2CH_3$, or $OCH_3$.

In some embodiments, $R^d$ is selected from F, CN, $C(=O)CH_3$, $CH_2CH_3$, or $OCH_3$.

In some embodiments, $R^d$ is selected from F, CN, $CH_2CH_3$, or $OCH_3$.

In some embodiments, $R^6$ and $R^7$ together with the N to which they are attached form 6- to 13-membered spiroheterocycloalkyl, wherein the 6- to 13-membered spiroheterocycloalkyl is optionally substituted with one or more $R^d$.

In some embodiments, $R^6$ and $R^7$ together with the N to which they are attached form

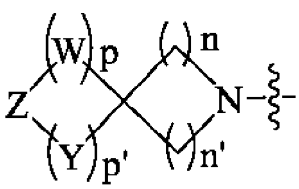

optionally substituted with one or more $R^d$, wherein:

n, n', p, and p' are independently selected from 1, 2, 3, or 4, and $n + n' + p + p' \leq 10$;
W and Y are independently selected from $CH_2$, NH, or O; and
Z is selected from $CH_2$, NH, O, or a bond.

In some embodiments, n, n', p, and p' are independently selected from 1, 2 or 3, and $n + n' + p + p' \leq 10$.

In some embodiments, n, n', p, and p' are independently selected from 1 or 2, and $n + n' + p + p' \leq 8$.

In some embodiments, W and Y are selected from $CH_2$.

In some embodiments, Z is selected from O, $CH_2$, or a bond.

In some embodiments, Z is selected from $CH_2$, or a bond.

In some embodiments, $R^6$ and $R^7$ together with the N to which they are attached form the following groups which are optionally substituted with one or more $R^d$:

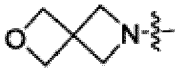, 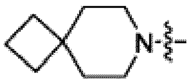, 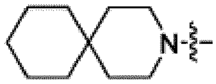, 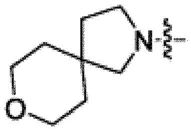, 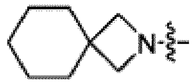, 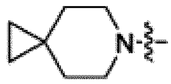.

or

In some embodiments, $R^6$ and $R^7$ together with the N to which they are attached form the following groups which are optionally substituted with one or more $R^d$:

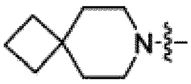, 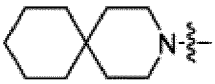, 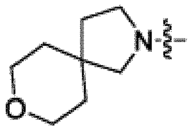, 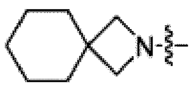, or 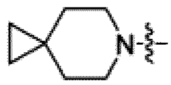.

In some embodiments, $R^6$ and $R^7$ together with the N to which they are attached form the following groups:

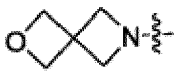, 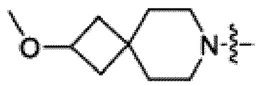,

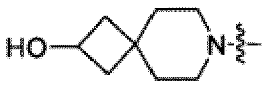, 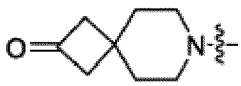, 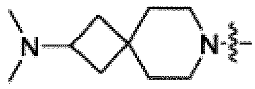, 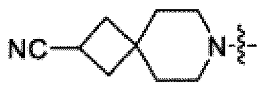,

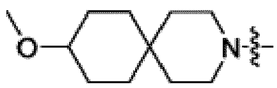, 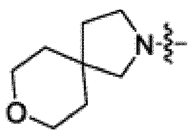, 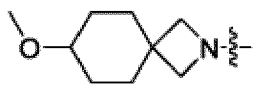, 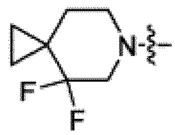,

or

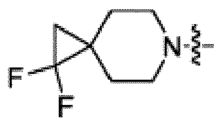.

In some embodiments, $R^6$ and $R^7$ together with the N to which they are attached form the following groups:

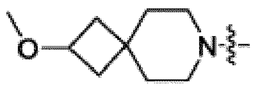, 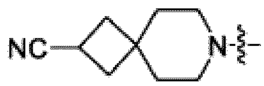,

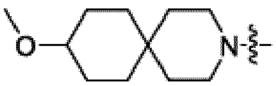, 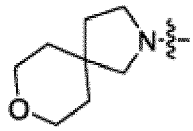, 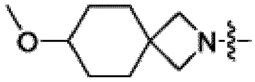, 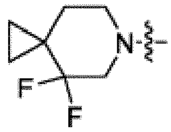,

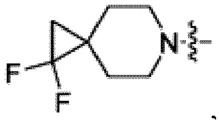, or 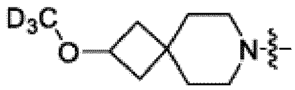.

In some embodiments, $R^6$ and $R^7$ together with the N to which they are attached form the following groups:

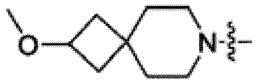, 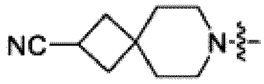,

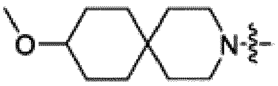, 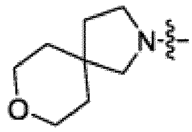, 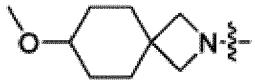, 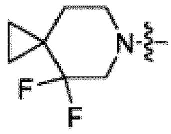,

or

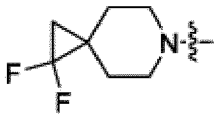

In some embodiments, $R^6$ and $R^7$ together with the N to which they are attached form 6- to 13-membered bridged heterocycloalkyl, wherein the 6- to 13-membered bridged heterocycloalkyl is optionally substituted with one or more $R^d$.

In some embodiments, $R^6$ and $R^7$ together with the N to which they are attached form

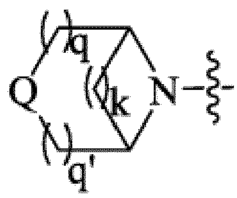

optionally substituted with one or more $R^d$, wherein:

q, q', and k are independently selected from 1, 2 or 3; and
Q is selected from $CH_2$, NH, O, or a bond.

In some embodiments, q and q' are independently selected from 1 or 2, and k is selected from 1, 2 or 3.

In some embodiments, q, q' and k are independently selected from 1 or 2.

In some embodiments, Q is selected from $CH_2$ or O.

In some embodiments, $R^6$ and $R^7$ together with the N to which they are attached form the following groups which are optionally substituted with one or more $R^d$:

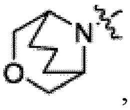, 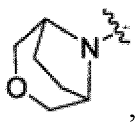, or 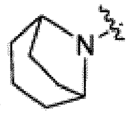.

In some embodiments, $R^6$ and $R^7$ together with the N to which they are attached form the following groups which are optionally substituted with one or more $R^d$:

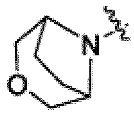 or 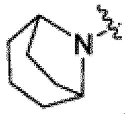.

In some embodiments, $R^6$ and $R^7$ together with the N to which they are attached form

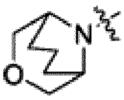, 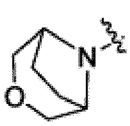, 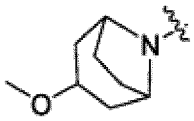, 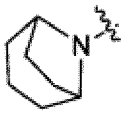, or 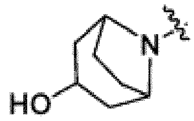.

In some embodiments, $R^6$ and $R^7$ together with the N to which they are attached form

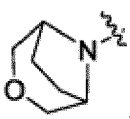, 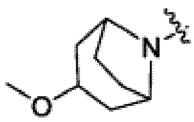, or 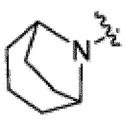.

In some embodiments, $R^6$ and $R^7$ together with the N to which they are attached form 6- to 13-membered fused heterocycloalkyl, 3- to 8-membered monocyclic heterocycloalkyl, or 5- to 10-membered heteroaryl optionally substituted with one or more $R^d$.

In some embodiments, $R^6$ and $R^7$ together with the N to which they are attached form 3- to 8-membered monocyclic heterocycloalkyl or 5- to 10-membered heteroaryl optionally substituted with one or more $R^d$.

In some embodiments, $R^6$ and $R^7$ together with the N to which they are attached form 5- to 6-membered monocyclic heterocycloalkyl or 5- to 6-membered heteroaryl optionally substituted with one or more $R^d$.

In some embodiments, $R^6$ and $R^7$ together with the N to which they are attached form 6- to 13-membered fused heterocycloalkyl or 5- to 6-membered monocyclic heterocycloalkyl optionally substituted with one or more $R^d$.

In some embodiments, $R^6$ and $R^7$ together with the N to which they are attached form 5- to 6-membered monocyclic heterocycloalkyl optionally substituted with one or more $R^d$.

In some embodiments, $R^6$ and $R^7$ together with the N to which they are attached form

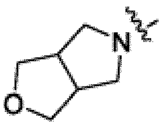,

a morpholine ring or a piperazine ring optionally substituted with one or more $R^d$.

In some embodiments, $R^6$ and $R^7$ together with the N to which they are attached form a morpholine ring or a piperazine ring optionally substituted with one or more $R^d$.

In some embodiments, $R^6$ and $R^7$ together with the N to which they are attached form

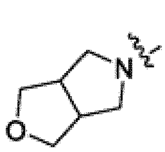
,
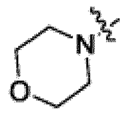
, or
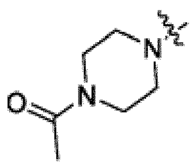
.

In some embodiments, $R^6$ and $R^7$ together with the N to which they are attached form

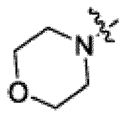
or
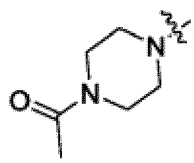
.

In some embodiments, the compound of formula (I) or the pharmaceutically acceptable salt thereof according to the present disclosure is selected from a compound of formula (II), or a pharmaceutically acceptable salt thereof:

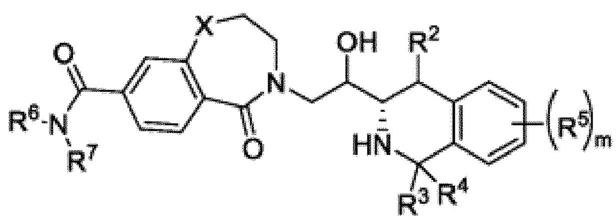

(II)

wherein X, $R^2$, $R^3$, $R^4$, $R^5$, $R^6$, $R^7$, and m are as defined above.

In some embodiments, the compound of formula (I) or the pharmaceutically acceptable salt thereof according to the present disclosure is selected from a compound of formula (III), or a pharmaceutically acceptable salt thereof:

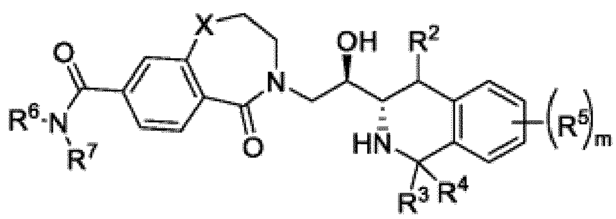

(III)

wherein X, $R^2$, $R^3$, $R^4$, $R^5$, $R^6$, $R^7$, and m are as defined above.

In some embodiments, the compound of formula (I) or the pharmaceutically acceptable salt thereof according to the present disclosure is selected from the following compounds or pharmaceutically acceptable salts thereof:

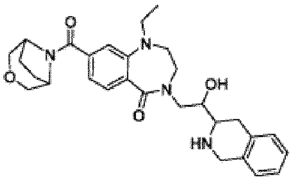
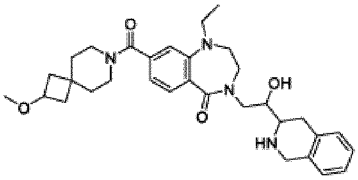
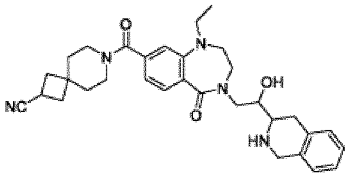

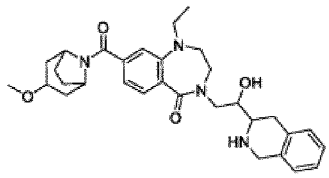
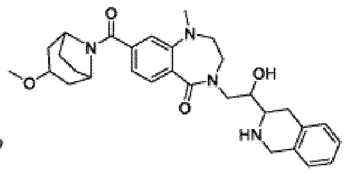
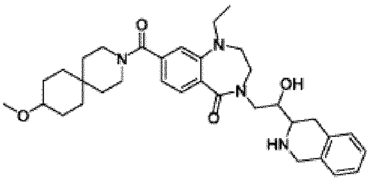

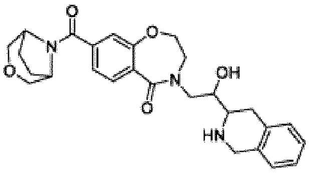
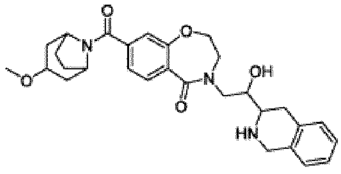
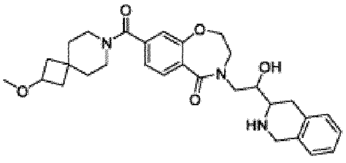

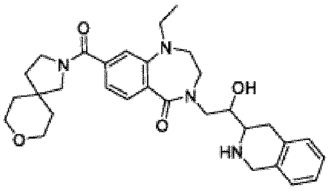
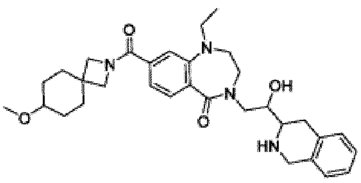
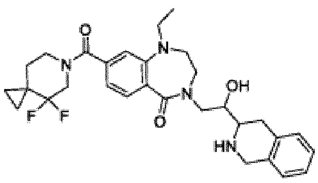

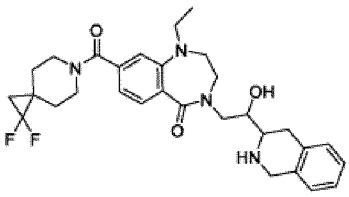
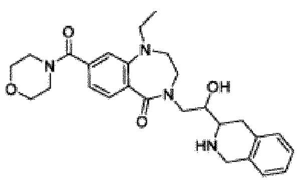
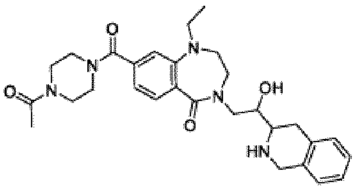

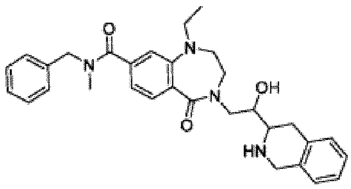
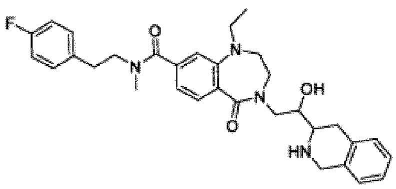

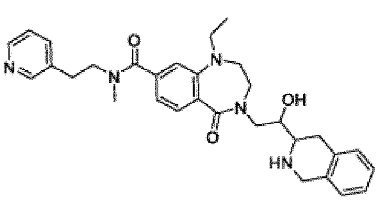
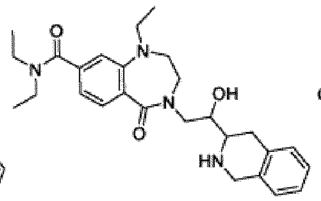
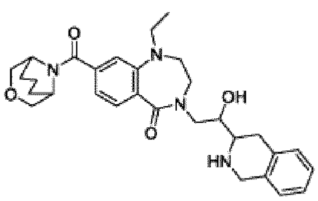

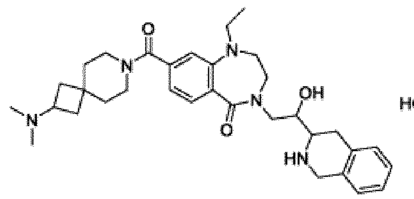
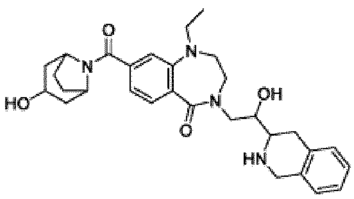
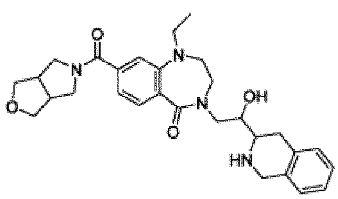

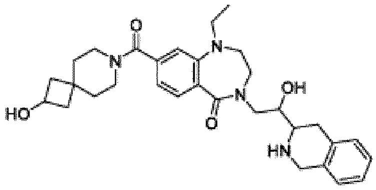
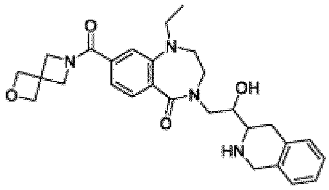
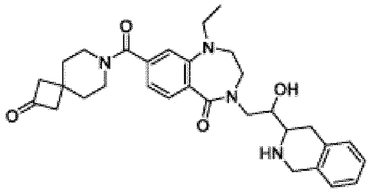

or

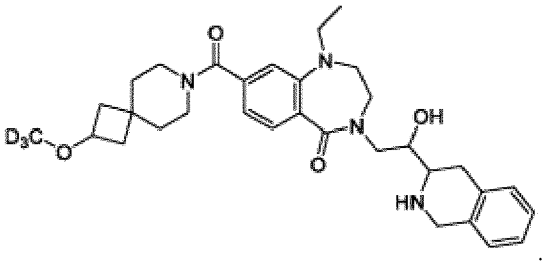

In some embodiments, the compound of formula (I) or the pharmaceutically acceptable salt thereof according to the present disclosure is selected from the following compounds or pharmaceutically acceptable salts thereof:

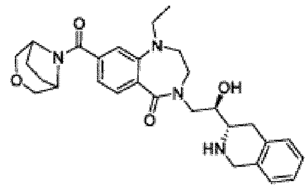
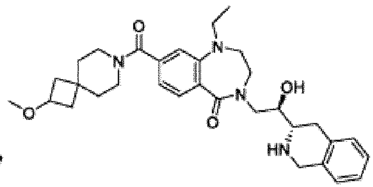
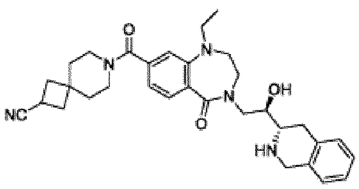

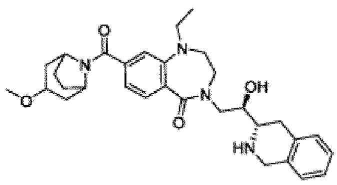
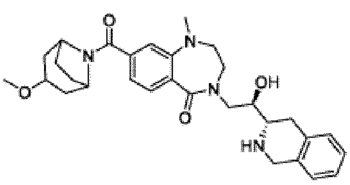
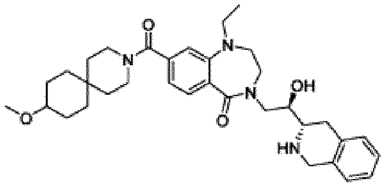

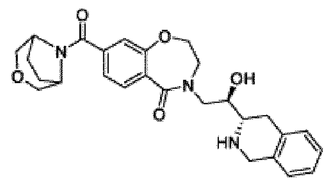
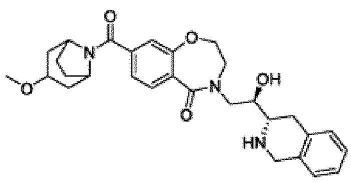
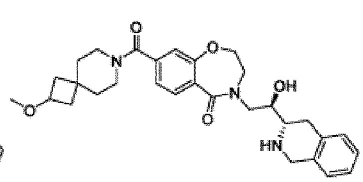

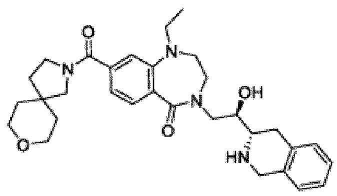
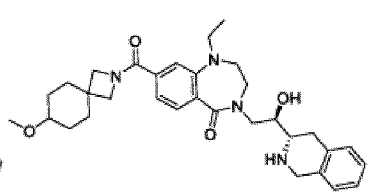
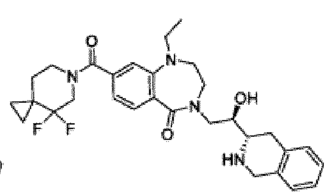

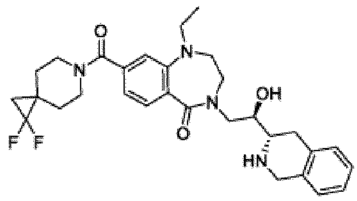
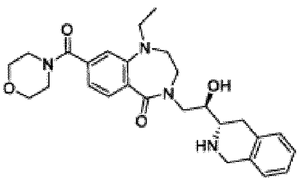
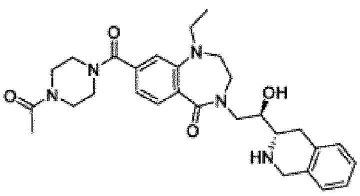

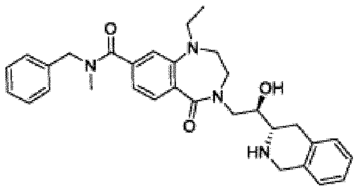
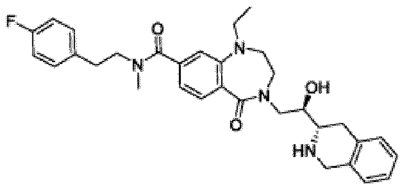

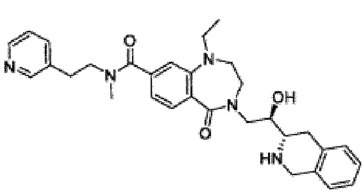
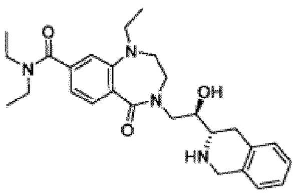
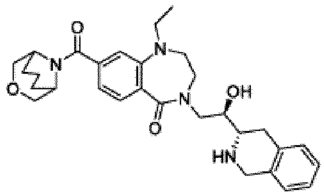

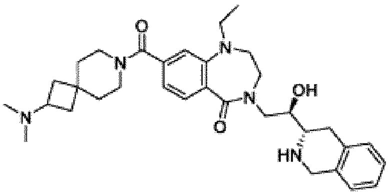
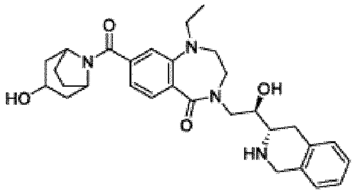
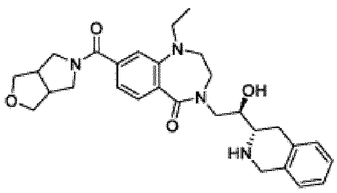

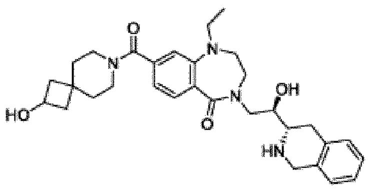
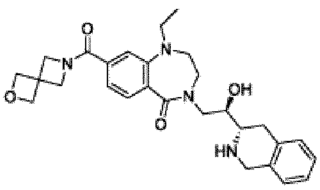
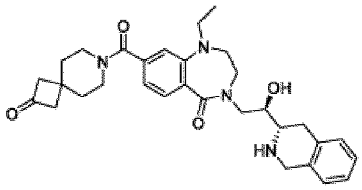

or

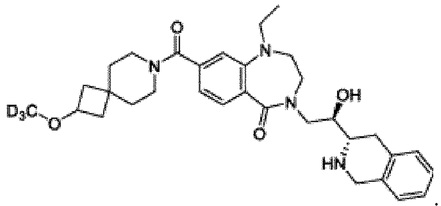

In some embodiments, the pharmaceutically acceptable salt of the compound of formula (I) is selected from a hydrochloride.

In another aspect, the present disclosure relates to a pharmaceutical composition comprising the compound of formula (I) or the pharmaceutically acceptable salt thereof according to the present disclosure.

In some embodiments, the pharmaceutical composition of the present disclosure also comprises a pharmaceutically acceptable adjuvant.

In another aspect, the present disclosure relates to a method of treating a disease mediated by PRMT5 in a mammal, comprising administering a therapeutically effective amount of a compound of formula (I) or a pharmaceutically

acceptable salt thereof or a pharmaceutical composition comprising the same to a mammal, preferably a human in need of the treatment.

In another aspect, the present disclosure relates to the use of the compound of formula (I) or the pharmaceutically acceptable salt thereof or the pharmaceutical composition comprising the same in the preparation of a medicament for preventing or treating a disease mediated by PRMT5.

In another aspect, the present disclosure relates to the use of the compound of formula (I) or the pharmaceutically acceptable salt thereof or the pharmaceutical composition comprising the same in the prevention or treatment of a disease mediated by PRMT5.

In another aspect, the present disclosure relates to the compound of formula (I) or the pharmaceutically acceptable salt thereof or the pharmaceutical composition comprising the same for preventing or treating a disease mediated by PRMT5.

In another aspect, the present disclosure relates to the compound of formula (I) or the pharmaceutically acceptable salt thereof or the pharmaceutical composition comprising the same that prevents or treats a disease mediated by PRMT5.

In some embodiments, the disease mediated by PRMT5 is cancer.

Definition and Description of Terminology

Unless otherwise stated, the following terms used in the present disclosure have the following meanings. A specific term without specifical definition should not be considered uncertain or unclear, and it should be understood in its ordinary meaning in the art. When a trade name appears herein, it is intended to refer to the corresponding commodity or an active ingredient thereof.

Unless otherwise stated, the definitions of groups and terms described in the specification and claims of the present disclosure, including their definitions as examples, exemplary definitions, preferred definitions, definitions listed in tables, and definitions of specific compounds in the examples, etc., may be arbitrarily combined or incorporated with one another. The group definitions and compound structures derived from such combinations and incorporations should fall within the scope described in the specification of the present disclosure.

In the present disclosure, "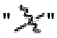" denotes a linking site.

The term "substituted" means that any one or more hydrogen atoms on the designated atom are replaced with a substituent, provided that the valence state of the designated atom is normal, and the substituted compound is stable. When the substituent is oxo (i.e., =O), it means that two hydrogen atoms are substituted, which would not occur on aromatic groups.

The term "optional" or "optionally" means that the subsequently described event or circumstance may or may not occur, and the description includes the occurrence and the non-occurrence of the event or circumstance. For example, the expression "ethyl is optionally substituted with halogen" means that ethyl may be unsubstituted ($CH_2CH_3$), monosubstituted (such as $CH_2CH_2F$), polysubstituted (such as $CHFCH_2F$, $CH_2CHF_2$), or completely substituted ($CF_2CF_3$). With respect to any group containing one or more substituents, it will be understood by those skilled in the art that any substitution or substitution patterns that are sterically impractical and/or synthetically non-feasible are not intended to be introduced into such groups.

$C_m$-$C_n$ herein means that the moiety has an integer number of carbon atoms, wherein the integer number is within the range m-n. For example, "$C_1$-$C_{10}$" means that the group may have 1, 2, 3, 4, 5, 6, 7, 8, 9, or 10 carbon atoms.

Where any variable (such as R) appears more than once in the composition or structure of a compound, its definition in each case is independent. For example, if a group is substituted with two Rs, then each R has an independent option.

When the bonds of a substituent are cross-linked to two atoms on a ring, the substituent can be bonded with any atom on the ring. For example, the structural unit

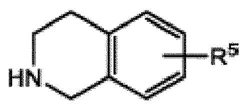

means that the substitution with $R^5$ may occur on any position of the phenyl ring.

The term "halo" or "halogen" refers to fluoro, chloro, bromo and iodo.

The term "hydroxy" refers to -OH group.

The term "cyano" refers to -CN group.

The term "amino" refers to $-NH_2$ group.

The term "alkyl" refers to a hydrocarbyl group of general formula $C_nH_{2n+1}$. The alkyl group can be linear or branched. For example, the term "$C_1$-$C_{10}$ alkyl" is to be understood as denoting a linear or branched, saturated monovalent hydrocarbyl having 1, 2, 3, 4, 5, 6, 7, 8, 9, or 10 carbon atoms. Said alkyl is such as methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, and the like; and the term "$C_{1-6}$ alkyl" means alkyl that contains 1 to 6 carbon atoms (such as methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). Similarly, the alkyl moiety (i.e., alkyl) in alkoxy, alkylamino, and dialkylamino has the same definition as that described above.

The term "alkoxy" refers to -O-alkyl. Preferably, "$C_1$-$C_{10}$ alkoxy" may include "$C_1$-$C_6$ alkoxy".

The term "alkylamino" refers to -NH-alkyl.

The term "dialkylamino" refers to -N(alkyl)$_2$.

The term "$C_2$-$C_{10}$ alkenyl" is to be understood as preferably meaning a linear or branched, monovalent hydrocarbyl, which contains one or more double bonds and has 2, 3, 4, 5, 6, 7, 8, 9 or 10 carbon atoms, preferably "$C_2$-$C_6$ alkenyl", further preferably "$C_2$-$C_4$ alkenyl", and more further preferably, $C_2$ alkenyl or $C_3$ alkenyl. It is to be understood that in the case in which the alkenyl group contains more than one double bond, then the double bonds may be isolated from, or conjugated with, each other. Said alkenyl is such as vinyl, allyl, (E)-2-methylvinyl, (Z)-2-methylvinyl, (E)-but-2-enyl, (Z)-but-2-enyl, (E)-but-1-enyl, (Z)-but-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl.

The term "$C_2$-$C_{10}$ alkynyl" is to be understood as preferably meaning a linear or branched, monovalent hydrocarbyl which contains one or more triple bonds and has 2, 3, 4, 5, 6, 7, 8, 9 or 10 carbon atoms, preferably "$C_2$-$C_6$ alkynyl", further preferably "$C_2$-$C_4$ alkynyl", and more further preferably, $C_2$ alkynyl or $C_3$ alkynyl. Said alkynyl is such as ethynyl, prop-1-ynyl, prop-2-ynyl, but-1-ynyl, but-2-ynyl, but-3-ynyl, 1-methylprop-2-ynyl.

The term "cycloalkyl" refers to a carbocyclic group that is fully saturated and can exist as a monocyclic ring, fused ring, bridged ring or spirocyclic ring. Unless otherwise indicated, the carbocyclic group is typically a 3- to 10-membered ring. Non-limiting examples of cycloalkyl include, but are not limited to cyclopropyl, cyclobutyl, cyclopentyl, cyclohexyl, norbornyl (bicyclo[2.2.1]heptyl), bicyclo[2.2.2]octyl, adamantyl, spiro[4.5]decane, and the like. Spirocycloalkyl refers to cycloalkyl that exists as a spirocyclic ring. Preferably, "$C_3$-$C_{10}$ cycloalkyl" is further preferably "$C_3$-$C_6$ cycloalkyl".

The term "cycloalkyloxy" can be understood as "cycloalkyl-O-", preferably, "$C_3$-$C_{10}$ cycloalkyloxy" may include "$C_3$-$C_6$ cycloalkyloxy".

The term "heterocycloalkyl" refers to a cyclic group that is fully saturated and can exist as a monocyclic ring, fused ring, bridged ring or spirocyclic ring. Unless indicated otherwise, the heterocycle is typically a 3 to 20-membered ring containing 1 to 3 heteroatoms (preferably 1 or 2 heteroatoms) independently selected from sulfur, oxygen and/or nitrogen. "3- to 8-membered monocyclic heterocycloalkyl" refers to a fully saturated cyclic group having 3, 4, 5, 6, 7, or 8 ring atoms, existing as a monocyclic ring, and containing 1 to 3 heteroatoms (preferably, 1 or 2 heteroatoms) independently selected from sulfur, oxygen and/or nitrogen. Examples of 3-membered heterocycloalkyl include but are not limited to oxiranyl, thiiranyl, and aziranyl; non-limiting examples of 4-membered heterocycloalkyl include but are not limited to azetidinyl, oxetanyl, and thietanyl; examples of 5-membered heterocycloalkyl include but are not limited to tetrahydrofuryl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include but are not limited to piperidyl, tetrahydropyranyl, tetrahydrothiopyranyl, morpholinyl, piperazinyl, 1,4-thioxanyl, 1,4-dioxanyl, thiomorpholinyl, 1,3-dithianyl, 1,4-dithianyl; and examples of 7-membered heterocycloalkyl include but are not limited to azepanyl, oxepanyl, and thiepanyl. Monocyclic heterocycloalkyl is preferably monocyclic heterocycloalkyl having 5 or 6 ring atoms. Spiroheterocycloalkyl refers to heterocycloalkyl that exists as a spirocyclic ring.

The term "6- to 13-membered spiroheterocycloalkyl" refers to a fully saturated cyclic group having 6, 7, 8, 9, 10, 11, 12, or 13 ring atoms, existing as a spirocyclic ring, and containing 1 to 3 heteroatoms (preferably, 1 or 2 heteroatoms) independently selected from sulfur, oxygen and/or nitrogen.

The term "6- to 13-membered fused heterocycloalkyl" refers to a fully saturated cyclic group having 6, 7, 8, 9, 10, 11, 12, or 13 ring atoms, existing as a fused ring/by means of fusing, and containing 1 to 3 heteroatoms (preferably, 1 or 2 heteroatoms) independently selected from sulfur, oxygen and/or nitrogen.

The term "6- to 13-membered bridged heterocycloalkyl" refers to a fully saturated cyclic group having 6, 7, 8, 9, 10, 11, 12, or 13 ring atoms, existing as a bridged ring, and containing 1 to 3 heteroatoms (preferably, 1 or 2 heteroatoms) independently selected from sulfur, oxygen and/or nitrogen.

The term "heterocyclyl" refers to a monocyclic heterocyclyl and fused heterocyclyl system, wherein a fused heterocycle includes fused heterocyclyl, spiro heterocyclyl, and bridged heterocyclyl, and can be saturated, partially saturated, or unsaturated but cannot be aromatic.

The term "3- to 10-membered heterocyclyl" means a saturated or partially saturated monovalent monocyclic

or bicyclic hydrocarbyl comprising 1-5, preferably 1-3 heteroatoms selected from N, O and S. In particular, the heterocyclyl can include but is not limited to: a 4-membered ring such as azetidinyl, and oxetanyl; a 5-membered ring such as tetrahydrofuryl, dioxolyl, pyrrolidinyl, imidazolidinyl, pyrazolidinyl, and pyrrolinyl; or a 6-membered ring such as tetrahydropyranyl, piperidyl, morpholinyl, dithianyl, thiomorpholinyl, piperazinyl, or trithianyl; or a partially saturated 6-membered ring such as tetrahydropyridyl; or a 7-membered ring such as diazepanyl. Optionally, the 3- to 10-membered heterocyclyl can be 8- to 10-membered benzo-fused heterocyclyl or 8- to 10-membered heteroaryl-fused heterocyclyl, including but not limited to such as benzopiperidinyl, pyridopiperidyl, or pyrimidopiperidyl, and the like; and the heterocyclyl, *i.e.,* 3- to 10-membered heterocyclyl can also include, but is not limited to a 5,5-membered ring, e.g., a hexahydrocyclopenta[c]pyrrol-2(1H)-yl, or a 5,6-membered bicyclic ring, e.g., a hexahydropyrrolo[1,2-a]pyrazin-2(1H)-yl. A nitrogen-containing ring can be partially unsaturated, i.e., it can contain one or more double bonds, such as, without being limited thereto, a 2,5-dihydro-1H-pyrrolyl, 4H-[1,3,4]thiadiazinyl, 4,5-dihydrooxazolyl, or 4H-[1,4]thiazinyl, or, it may be benzo-fused, such as, without being limited thereto, a dihydroisoquinolinyl. According to the present disclosure, the heterocyclyl is not aromatic.

The term "3- to 10-membered heterocyclyloxy" is to be understood as "3- to 10-membered heterocyclyl-O-".

The term "$C_6$-$C_{10}$ aryl" is understood to preferably denote a monovalent aromatic or partially aromatic monocyclic or bicyclic hydrocarbyl having 6 to 10 carbon atoms, especially a ring having 6 carbon atoms ("$C_6$ aryl"), such as phenyl; or a ring having 9 carbon atoms ("$C_9$ aryl"), such as indanyl or indenyl, or a ring having 10 carbon atoms ("$C_{10}$ aryl"), such as naphthyl.

The term "$C_6$-$C_{10}$ aryloxy" is to be understood as "$C_6$-$C_{10}$ aryl-O-".

The term "5- to 10-membered heteroaryl" is understood to include monovalent monocyclic or bicyclic aromatic ring systems having 5, 6, 7, 8, 9, or 10 ring atoms, especially 5 or 6 or 9 or 10 ring atoms, and containing 1-5, preferably 1-3, heteroatoms each independently selected from N, O and S, and in addition, may be benzo-fused in each case. In particular, the heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, etc. and the benzo derivatives thereof, such as benzofuryl, benzothienyl, benzothiazolyl, benzoxazolyl, benzisoxazolyl, benzoimidazolyl, benzotriazolyl, indazolyl, indolyl, isoindolyl, and the like; or pyridyl, pyridazinyl, pyrimidinyl, pyrazinyl, triazinyl, and the like, and the benzo derivatives thereof such as quinolyl, quinazolinyl, isoquinolinyl, and the like; or azocinyl, indolizinyl, purinyl, and the like and the benzo derivatives thereof; or cinnolinyl, phthalazinyl, quinazolinyl, quinoxalinyl, naphthyridinyl, pteridinyl, and the like.

The term "5- to 10-membered heteroaryloxy" is to be understood as "5- to 10-membered heteroaryl-O".

The term "treatment" refers to the administration of the compounds or preparations of the present disclosure for preventing, ameliorating or eliminating diseases or one or more symptoms associated with the diseases and comprises:

(i) prophylaxis of occurrence of diseases or conditions in mammals, particularly when the mammals are susceptible to the conditions, but have not been diagnosed with the conditions;
(ii) inhibition of diseases or conditions, i.e., restraining their development; or
(iii) relief of diseases or conditions, i.e., resolution of the diseases or conditions.

The term "therapeutically effective amount" means an amount of a compound of the present disclosure used to (i) treat or prevent a particular disease, condition, or disorder, (ii) attenuate, ameliorate, or eliminate one or more symptoms of a particular disease, condition, or disorder, or (iii) prevent or delay the onset of one or more symptoms of a particular disease, condition, or disorder described herein. The amount of a compound of the present disclosure which constitutes a "therapeutically effective amount" will vary depending on the compound, the conditions and their severity, the manner of administration, and the age of the mammal to be treated, but can be determined routinely by those skilled in the art according to their own knowledge and the present disclosure.

The term "pharmaceutically acceptable" refers to compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

As pharmaceutically acceptable salts, for example, the following examples may be mentioned: metal salts, ammonium salts, salts formed with organic bases, inorganic acids, organic acids, basic or acidic amino acids, and the like.

The term "pharmaceutical composition" refers to a mixture of one or more of the compounds or salts thereof according to the present disclosure and a pharmaceutically acceptable adjuvant. An object of the pharmaceutical composition is to facilitate administering the compound according to the present disclosure to an organism.

The term "pharmaceutically acceptable adjuvant" refers to those adjuvants which have no significant irritating effect on the organism and do not impair the bioactivity and properties of the active compound. Suitable adjuvants are well known to those skilled in the art, and are such as a carbohydrate, a wax, a water-soluble and/or water-swellable polymer, a hydrophilic or hydrophobic material, gelatin, an oil, a solvent, water, and the like.

The word "comprise" and its variants such as "comprises" or "comprising" is to be understood as an open, non-exclusive meaning, i.e., "including but not limited to".

The compounds and intermediates of the present disclosure may also exist in different tautomeric forms, and all such forms are embraced within the scope of the present disclosure. The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. A specific example of a proton tautomer is the imidazole moiety where the proton may migrate between the two ring nitrogens. Valence tautomers include interconversions by reorganization of some of the bonding electrons.

The present disclosure also includes isotopically-labeled compounds of the present disclosure which are identical to those recited herein, but have one or more atoms replaced by an atom having an atomic mass or mass number different from the atomic mass or mass number usually found in nature. Examples of isotopes that can be incorporated into compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, respectively.

Certain isotopically-labeled compounds of the present disclosure (e.g., those labeled with $^{3}H$ and $^{14}C$) are useful in tissue distribution assays of compounds and/or substrates. Tritiated (i.e., $^{3}H$) and carbon-14 (i.e., $^{14}C$) isotopes are particularly preferred for their ease of preparation and detectability. Positron emitting isotopes such as $^{15}O$, $^{13}N$, $^{11}C$, and $^{18}F$ are useful for positron emission tomography (PET) studies to determine substrate occupancy. The isotopically-labeled compounds of the present disclosure can generally be prepared according to following procedures analogous to those disclosed in the Schemes and/or in the Examples herein below by substituting a non-isotopically-labeled reagent with an isotopically-labeled reagent.

Furthermore, substitution with heavier isotopes (such as deuterium, i.e., $^{2}H$) may afford certain therapeutic advantages resulting from greater metabolic stability, for example, increased *in vivo* half-life or reduced dosage requirements, and hence may be preferred in some circumstances, wherein the deuterium substitution may be partial or complete, and partial deuterium substitution means that at least one hydrogen is substituted with at least one deuterium.

All isotopic variations of the compounds of the present disclosure, whether radioactive or not, are intended to be encompassed within the scope of the present disclosure.

The compound of the present disclosure may be asymmetric, such as, having one or more stereoisomers. Stereoisomer refers to isomers created by a different spatial arrangement of atoms in molecules; and unless otherwise stated, all stereoisomers are included, such as cis and trans isomers, conformational isomers, enantiomers and diastereomers. The compound comprising asymmetric carbon atoms of the present disclosure can be isolated in optically active-pure or racemic forms. The optically active-pure form can be resolved from the racemic mixture or synthesized by utilizing chiral raw materials or chiral reagents. The diagrammatic presentation of the racemate or enantiomerically pure compound herein is from Maehr, J. Chem. Ed. 1985, 62: 114-120. Unless otherwise stated, the wedge-shaped bond and the dashed bond

( 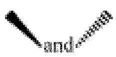 and )

are used to represent the absolute configuration of a stereocenter. When the compounds described herein contain olefinic double bonds or other centers of geometric asymmetry, and unless specified otherwise, it is intended that the compounds include both E and Z geometric isomers. Likewise, all tautomeric forms are encompassed within the scope of the present disclosure.

Non-limiting examples of stereoisomers include, but are not limited to:

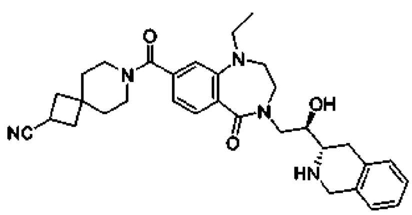

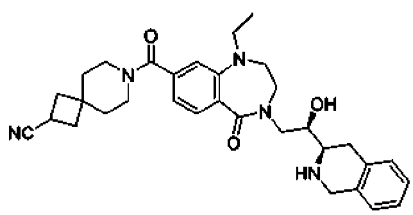

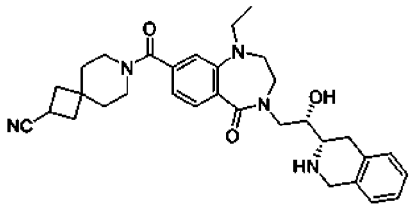

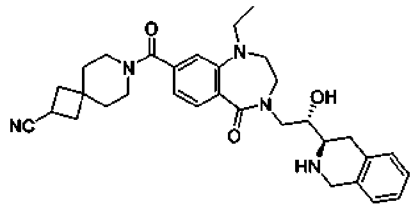

The pharmaceutical composition of the present disclosure may be prepared by combining the compound of the present disclosure with an appropriate pharmaceutically acceptable adjuvant. For example, the pharmaceutical composition of the present disclosure may be formulated into solid, semi-solid, liquid or gaseous preparations, such as tablets, pills, capsules, powders, granules, ointments, emulsions, suspensions, suppositories, injections, inhalants, gels, microspheres, and aerosols, and the like.

Typical administration routes of the compound or the pharmaceutically acceptable salt thereof, or the pharmaceutical composition thereof according to the present disclosure include, but are not limited to, oral administration, rectal administration, topical administration, administration by inhalation, parenteral administration, sublingual administration, intravaginal administration, intranasal administration, intraocular administration, intraperitoneal administration, intramuscular administration, subcutaneous administration, and intravenous administration.

The pharmaceutical composition of the present disclosure can be manufactured by using well-known methods in the art, such as conventional mixing method, dissolution method, granulation method, dragee manufacture method, grinding method, emulsification method, and freeze-drying method, and the like.

In some embodiments, the pharmaceutical composition is in oral form. For oral administration, the pharmaceutical composition may be formulated by mixing the active compound with a pharmaceutically acceptable adjuvant well-known in the art. Such adjuvants enable the compounds of the present disclosure to be formulated into tablets, pills, lozenges, dragees, capsules, liquids, gels, syrups, suspensions and the like, for oral administration to patients.

A solid oral composition can be prepared by a conventional mixing, filling or tabletting method. For example, it can be obtained by the following method: mixing the active compound with a solid adjuvant, optionally grinding the resulting mixture, adding other suitable adjuvants, if necessary, and then processing the mixture into granules to obtain cores of tablets or dragees. Suitable adjuvants include, but are not limited to, binders, diluents, disintegrants, lubricants, glidants, sweeting agents, or flavoring agents, and the like.

The pharmaceutical composition can also be suitable for parenteral administration, such as sterile solutions, suspensions or lyophilized products in a suitable unit dosage form.

The daily administration dose of the compound of general formula I in all the administration manners described herein is from 0.01 mg/kg body weight to 100 mg/kg body weight, preferably from 0.05 mg/kg body weight to 50 mg/kg body weight, and more preferably from 0.1 mg/kg body weight to 30 mg/kg body weight, in the form of a single dose or divided doses.

The compounds of the present disclosure can be prepared by various synthetic methods well known to those skilled in the art, including the specific embodiments listed below, the embodiments formed by the combination with other chemical synthesis methods, and equivalent alternative embodiments well known to those skilled in the art, wherein the preferred embodiments include but are not limited to the examples of the present disclosure.

The chemical reactions described in the specific embodiments of the present disclosure are completed in a suitable solvent, wherein the solvent must be suitable for the chemical changes of the present disclosure and the reagents and materials required thereby. In order to obtain the compounds of the present disclosure, sometimes a person skilled in the art needs to modify or select synthesis steps or reaction schemes based on the existing embodiments.

An important consideration in the design of a synthetic route in the art is the selection of a suitable protecting group for a reactive functional group, such as an amino group in the present disclosure. For example, reference may be made to Greene's Protective Groups in Organic Synthesis (4th Ed)., Hoboken, New Jersey: John Wiley & Sons, Inc. All references cited herein are incorporated into the present disclosure in their entireties.

In some embodiments, the compound of general formula (I) of the present disclosure may be prepared by a person skilled in the field of organic synthesis via route 1:

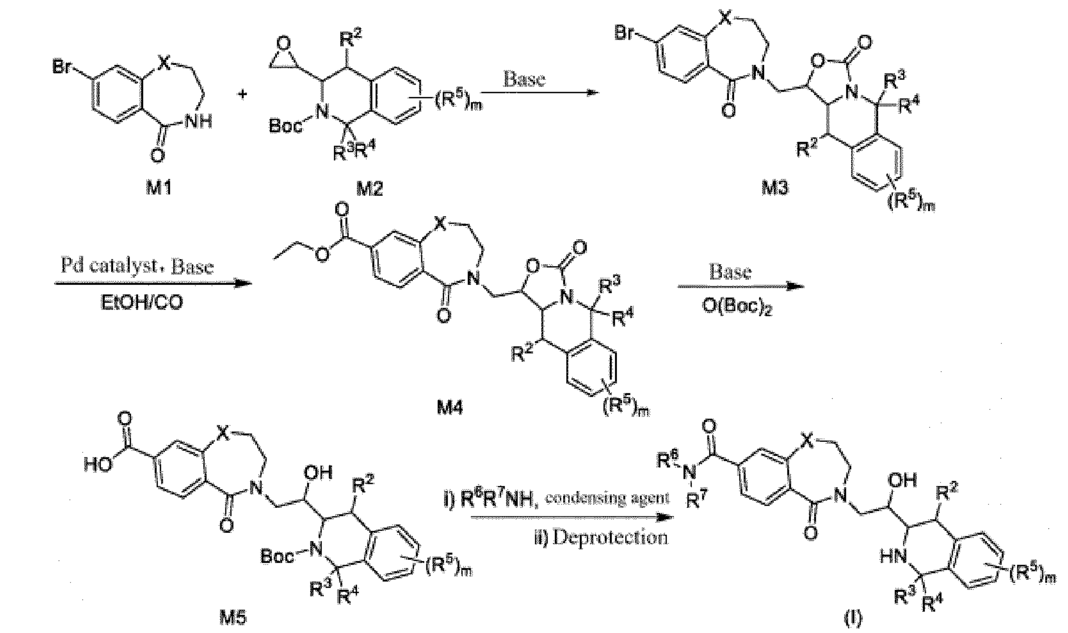


Route 1

reacting compounds M1 and M2 in the presence of a base to produce M3; reacting M3 in the presence of a palladium catalyst/base/ethanol/CO to produce M4; subjecting M4 to carboxyl deprotection in the presence of a base and producing M5 in the presence of Boc-anhydride; and reacting M5 and $R^6R^7NH$ in the presence of a condensing agent and further removing the amino protecting group to produce the compound of formula (I).

In some embodiments, the compound of general formula (I) of the present disclosure may be prepared by a person skilled in the field of organic synthesis via route 2:

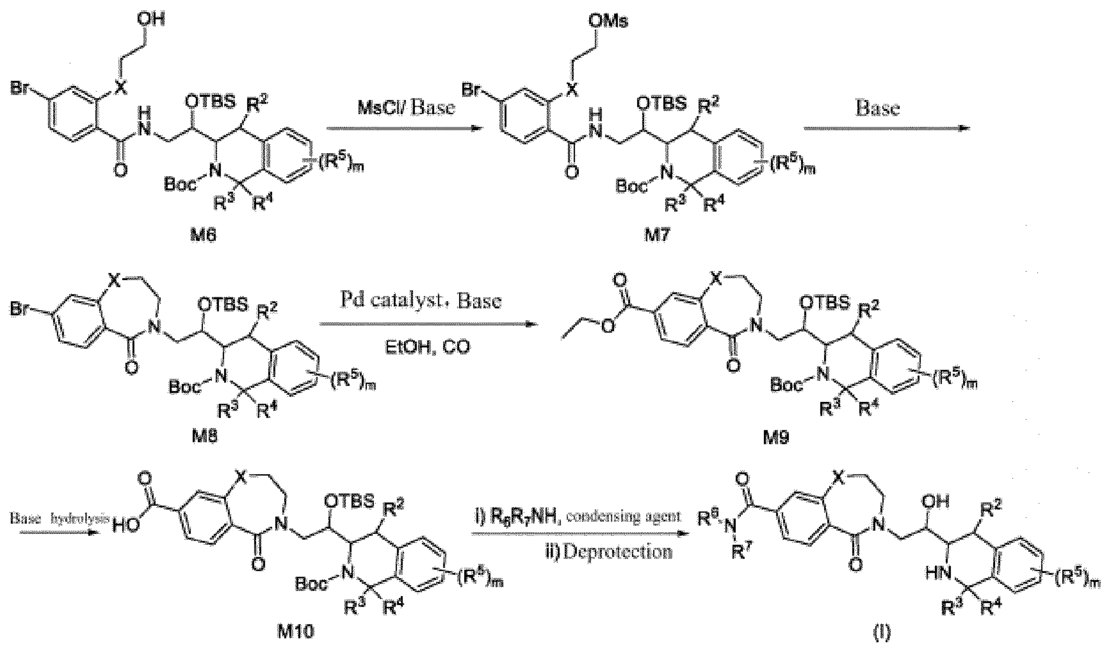


Route 2

reacting compound M6 in the presence of MsCl and a base to produce M7; reacting M7 in the presence of a base to produce M8; reacting M8 in the presence of a palladium catalyst/base/ethanol/CO to produce M9; subjecting M9 to a hydrolysis reaction in the presence of a base to obtain M10; and reacting M10 and $R^6R^7NH$ in the presence of a condensing agent and further removing the amino protecting group to produce the compound of formula (I).

**Brief Description of the Drawings**

FIG. 1 is the ball-and-stick representation of the single crystal of compound 1-8.
FIG. 2 is the tumor growth curve of mice receiving the test compound in Z-138 subcutaneous tumor models.
FIG. 3 is the body weight change curve of mice receiving the test compound in Z-138 subcutaneous tumor models.

**Detailed Description**

For clarity, the present disclosure is further illustrated by the following examples, but the examples are not intended to limit the scope of the present disclosure. All reagents used in the present disclosure are commercially available and can be used without further purification.

Example 1. Preparation of tert-butyl (S)-3-((S)-oxiran-2-yl)-3,4-dihydroisoquinoline-2(1H)-carboxylate (intermediate 1)

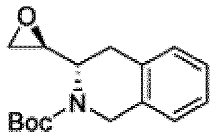

Intermediate 1

Preparation of compound b:

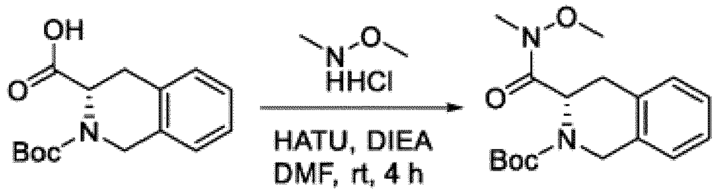


a b

At room temperature, (S)-2-(tert-butoxycarbonyl)-1,2,3,4-tetrahydroisoquinoline-3-carboxylic acid (a) (55 g, 200 mmol), dimethylhydroxylamine hydrochloride (29.4 g, 300 mmol), and 2-(7-azabenzotriazole)-N,N,N',N'-tetramethylurea hexafluorophosphate (91 g, 240 mmol) were added into a 1 L single-necked flask, and then anhydrous N,N-dimethylformamide (500 mL) was added. Under nitrogen, the reaction was cooled in ice bath and then N,N-diisopropylethylamine (104 mL, 600 mmol) was added dropwise. The reaction solution was reacted at room temperature for 4 hours. Upon completion of reaction, excess N,N-diisopropylethylamine and N,N-dimethylformamide were removed by rotary evaporation and then the resulting mixture was cooled under ice bath, diluted with saturated brine (1 L), and extracted with ethyl acetate (200 mL X 2). The organic phases were combined and washed with 5% aqueous sodium carbonate solution (500 mL X 2), and then washed with saturated brine (500 mL). The resulting mixture was dried over anhydrous sodium sulfate and filtered, and the filtrate was concentrated under reduced pressure and then purified by silica gel column chromatography (eluent gradient: petroleum ether/ethyl acetate = 2/1) to afford the target intermediate tert-butyl (S)-3-(methoxy(methyl)carbamoyl)-3,4-dihydroisoquinoline-2(1H)-carboxylate (b) (62 g, yield: 97%).

**LCMS:** Rt: 1.76 min; MS m/z (ESI): 321.3 [M+H].
**Chiral HPLC:** Rt: 3.159 min.

Preparation of compound c:

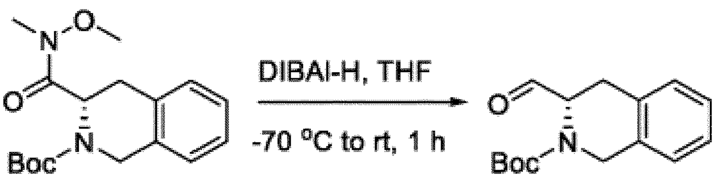


b c

At room temperature, tert-butyl (S)-3-(methoxy(methyl)carbamoyl)-3,4-dihydroisoquinoline-2(1H)-carboxylate (b) (20 g, 62.5 mmol) was weighed into a 500 mL three-necked flask, and anhydrous tetrahydrofuran (200 mL) was added. The mixture was cooled to -70°C and a solution of diisobutylaluminum hydride (DIBAL-H) in toluene (1.5 mol/L, 83 mL, 125 mmol) was slowly added dropwise. The reaction solution was stirred at -70°C for 1 hour. Upon completion of reaction, saturated ammonium chloride solution (100 mL) was slowly added at -70°C to quench the reaction and then 0.5 mol/L hydrochloric acid aqueous solution (200 mL) was added to dilute the reaction. After layering, the organic phase was washed with saturated sodium chloride aqueous solution (200 mL X 2), then dried over anhydrous sodium sulfate and filtered. The filtrate was concentrated under reduced pressure. The residue was purified by silica gel column chromatography (eluent gradient: petroleum ether/ethyl acetate = 8/1) to afford the intermediate tert-butyl (S)-3-formyl-3,4-dihydroisoquinoline-2(1H)-carboxylate (c) (15 g, yield: 92%).

**LCMS:** Rt: 1.93 min; MS m/z (ESI): 206.1 [M-56+H].
**Chiral HPLC:** Rt: 2.018 min.

Preparation of compound d:

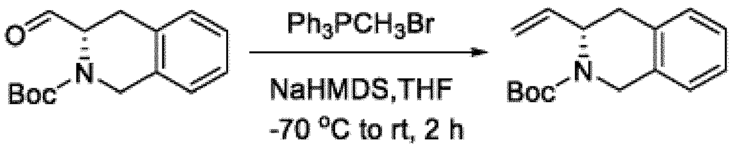


c d

Methyltriphenylphosphonium bromide (238 g, 0.67 mol) was dispersed into anhydrous tetrahydrofuran (1.5 L). Under nitrogen, the mixture was cooled to -70°C. Sodium bis(trimethylsilyl)amide (334 mL, 0.67 mol) was slowly added dropwise whilst the temperature was controlled below -50°C. After the addition was completed, the reaction was slowly warmed to room temperature and stirred for 2 hours. The reaction was cooled to -70°C again. A solution of tert-butyl (S)-3-formyl-3,4-dihydroisoquinoline-2(1H)-carboxylate (c) (87 g, 0.33 moL) in tetrahydrofuran solution (200 mL) was slowly added dropwise whilst the temperature was controlled below -50°C. After the addition was completed, the reaction was slowly warmed to room temperature overnight. After the reaction was complete as detected by TLC, the reaction was cooled to 0°C and quenched with saturated ammonium chloride solution. The 1 mol/L hydrochloric acid aqueous solution was slowly added to adjust the pH to 3-4, and ethyl acetate (500 mL) was added for extraction. The organic phase was washed with saturated brine (200 mL X 2), dried over anhydrous sodium sulfate and filtered. The filtrate was concentrated under reduced pressure. The residue was recrystallized by adding mixed solvent (ethyl acetate/petroleum ether = 1/4) and then filtered to remove precipitated triphenyl phosphine oxide. The filtrate was concentrated and then subjected to silica gel column chromatography (ethyl acetate/petroleum ether = 1/8) to afford the target product tert-butyl (S)-3-vinyl-3,4-dihydroisoquinoline-2(1H)-carboxylate (d) (85 g, yield: 98%).
**Chiral HPLC:** Rt: 1.883 min.

Preparation of compound e:

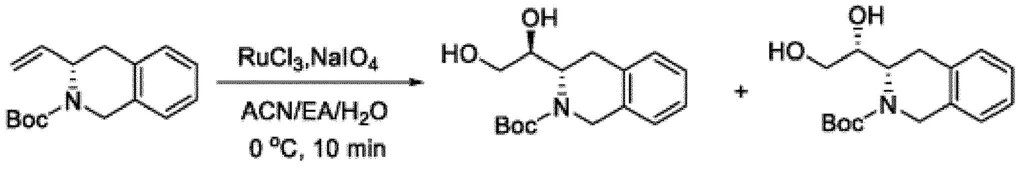


d e e-1

Tert-butyl (S)-3-vinyl-3,4-dihydroisoquinoline-2(1H)-carboxylate (d) (25.9 g, 0.1 mol) was dissolved in ethyl acetate/acetonitrile (500 mL/500 mL) solution and the mixture was cooled to 0°C. An aqueous solution of sodium periodate (32.1 g, 0.15 mol) and ruthenium trichloride hydrate (1.6 g, 7.7 mmol) was added within 10 minutes and the reaction solution was stirred at 0°C for 10 minutes. The reaction was complete as detected by TLC. Saturated sodium thiosulfate solution (150 mL) was added to quench the reaction. The resulting mixture was stirred for 30 minutes and layered, and the organic phase was washed with saturated brine (200 mL X 2), dried over anhydrous sodium sulfate and filtered. The filtrate was concentrated under reduced pressure and the residue was purified by silica gel column chromatography (eluents: ethyl acetate/petroleum ether = 1/2-1/1) to afford the product tert-butyl (S)-3-((S)-1,2-dihydroxyethyl)-3,4-dihydroisoquinoline-2(1H)-carboxylate (e) (less-polar product, 14 g, yield: 48%). The polarity of the eluent (ethyl acetate/petroleum ether = 2/1-1/0) was increased to afford the product tert-butyl (S)-3-((R)-1,2-dihydroxyethyl)-3,4-dihydroisoquinoline-2(1H)-carboxylate (e-1) (more-polar product, 8 g, yield: 28%).

Preparation of compound f:

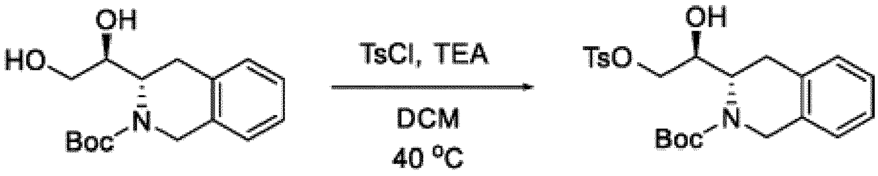


e f

Tert-butyl (S)-3-((S)-1,2-dihydroxyethyl)-3,4-dihydroisoquinoline-2(1H)-carboxylate (e) (14 g, 0.047 mol) was dissolved in dichloromethane (150 mL). Then p-toluenesulfonyl chloride (10.0 g, 0.052 mol) was added in batches under stirring following the addition of triethylamine (9.90 mL, 0.072 mol). The reaction solution was stirred at 40°C overnight. The reaction solution was cooled to room temperature and washed with saturated brine (100 mL X 2). The organic phase was dried over anhydrous sodium sulfate. After filtration, the filtrate was concentrated under reduced pressure. The residue was subjected to silica gel column chromatography (ethyl acetate/petroleum ether = 1/4) to afford the target product tert-butyl (S)-3-((S)-1-hydroxy-2-(tosyloxy)ethyl)-3,4-dihydroisoquinoline-2(1H)-carboxylate (f) (12.6 g, yield: 59%).

Preparation of compound intermediate 1:

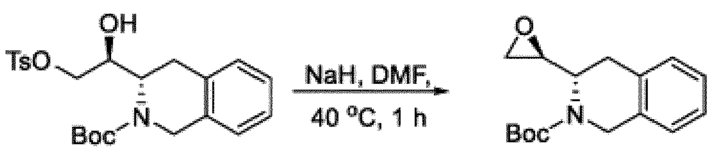


f Intermediate 1

Tert-butyl (S)-3-((S)-1-hydroxy-2-(tosyloxy)ethyl)-3,4-dihydroisoquinoline-2(1H)-carboxylate (f) (12.6 g, 28.2 mmol) was dissolved in N,N-dimethylformamide (150 mL). Under nitrogen, sodium hydride (1.70 g, 42.3 mmol) was added in batches, and the reaction solution was stirred at 40°C for 1 hour. The reaction was complete as detected by TLC. The reaction was cooled to 0°C and quenched by adding saturated brine dropwise. The reaction solution was directly purified using a reverse-phase silica gel chromatographic column (water/acetonitrile = 50/10). The resulting solution from the column chromatography was extracted with ethyl acetate (200 mL X 3). The organic phase was dried over anhydrous sodium sulfate and filtered. The filtrate was concentrated under reduced pressure to afford the intermediate tert-butyl (S)-3-((S)-oxiran-2-yl)-3,4-dihydroisoquinoline-2(1H)-carboxylate (intermediate 1) (6.5 g, yield: 72%).

Example 2. Preparation of 8-(3-oxa-8-azabicyclo[3.2.1]octane-8-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 001)

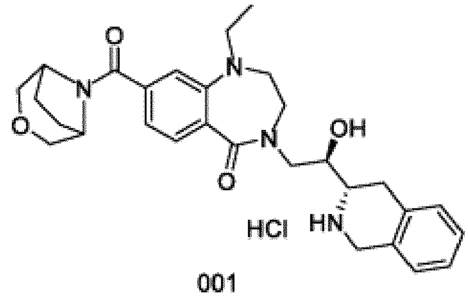

001

Preparation of compound 1-2:

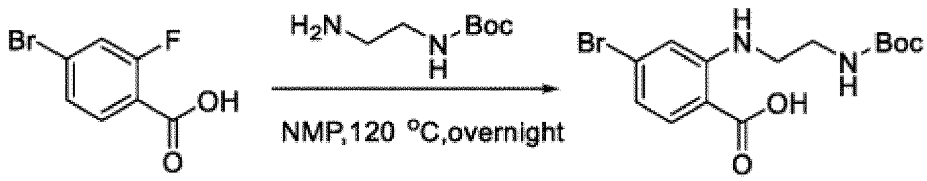


1-1 1-2

At room temperature, 4-bromo-2-fluorobenzoic acid (1-1) (7.5 g, 34.2 mmol) and N-tert-butoxycarbonyl-1,2-ethylenediamine (16.4 g, 102.7 mmol) were added to N-methyl pyrrolidone (30 mL). Upon completion of addition, the mixture was warmed to 120°C and reacted for 16 hours. Upon completion of reaction, the reaction solution was slowly added to water (150 mL) , adjusted with 2 mol/L dilute hydrochloric acid to the pH of 5-6, and then extracted with ethyl acetate (150 mL X 2). The organic phases were combined, washed with saturated brine (150 mL X 2), dried over anhydrous sodium sulfate and filtered. The filtrate was concentrated under reduced pressure to afford the target intermediate 4-bromo-2-((2-((tert-butoxycarbonyl)amino)ethyl)amino)benzoic acid (1-2) (crude, 11.0 g, yield: 89%).
**LCMS:** Rt: 1.836 min; MS m/z (ESI): 359.0 [M+H].

Preparation of compound 1-3:

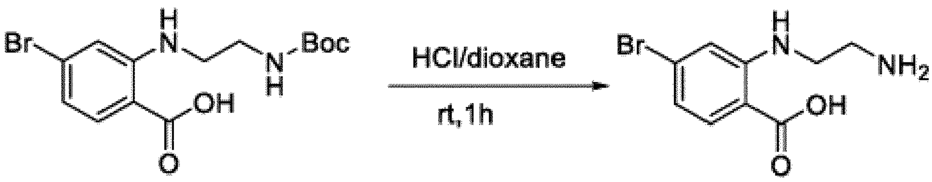


1-2 1-3

At room temperature, 4-bromo-2-((2-((tert-butoxycarbonyl)amino)ethyl)amino)benzoic acid (1-2) (11.0 g, 30.6 mmol) was added to 4 mol/L hydrochloric acid/dioxane solution (30 mL) and then the mixture was reacted at room temperature for 1 hour. Upon completion of reaction, the reaction solution was concentrated under reduced pressure to afford the target intermediate 2-((2-aminoethyl)amino)-4-bromobenzoic acid (1-3) (crude, 8 g, yield: 100%).
**LCMS:** Rt: 0.688 min; MS m/z (ESI): 259.0 [M+H].

Preparation of compound 1-4:

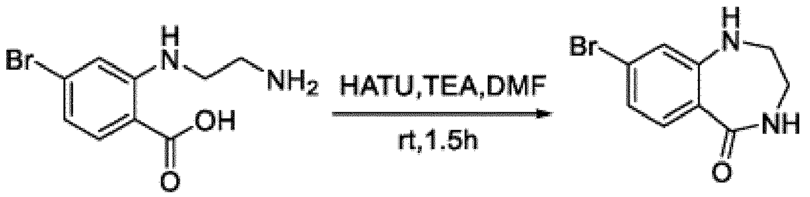


1-3 1-4

At room temperature, 2-((2-aminoethyl)amino)-4-bromobenzoic acid (1-3) (3 g, 11.6 mmol), 2-(7-azabenzotriazole)-N,N,N',N'-tetramethylurea hexafluorophosphate (8.8 g, 23.2 mmol) and triethylamine (4.7 g, 46.4 mmol) were added to ultradry N,N-dimethylformamide (20 mL) and the mixture was reacted at room temperature for 1.5 hours. Upon completion of reaction, saturated brine (100 mL) was added, and the resulting mixture was extracted with ethyl acetate (100 mL X 2). The organic phase was washed with saturated brine (50 mL X 2), dried over anhydrous sodium sulfate, and filtered. The filtrate was concentrated under reduced pressure. The residue was purified using a normal phase silica gel chromatographic column (eluent gradient: dichloromethane/methanol = 20/1) to afford the target intermediate 8-bromo-1,2,3,4-tetrahydro-5H-benzo[e][1,4]diazepin-5-one (1-4) (1.6 g, yield: 57%).
**LCMS:** Rt: 1.173 min; MS m/z (ESI): 241.0 [M+H].

Preparation of compound 1-5:

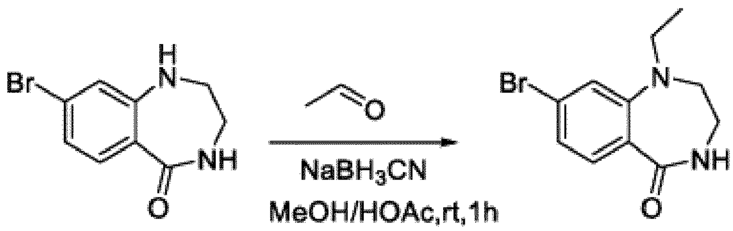


1-4 1-5

At room temperature, 8-bromo-1,2,3,4-tetrahydro-5H-benzo[e][1,4]diazepin-5-one (1-4) (1.6 g, 6.6 mmol), sodium cyanoborohydride (834 mg, 13.3 mmol) and acetaldehyde (584 mg, 13.3 mmol) were added to a mixed solution of acetic acid (2 mL) and methanol (20 mL). The mixture was reacted at room temperature for 1 hour. Upon completion of reaction, 2 mol/L dilute hydrochloric acid (1 mL) and water (50.0 mL) were added and the mixture was extracted with ethyl acetate (50.0 mL X 2). The organic phases were combined, washed with saturated brine (50 mL X 2), dried over anhydrous sodium sulfate, and filtered. The filtrate was concentrated under reduced pressure. The residue was purified using a normal phase silica gel chromatographic column (eluent gradient: petroleum ether/ethyl acetate = 1/2) to afford the target intermediate 8-bromo-1-ethyl-1,2,3,4-tetrahydro-5H-benzo[e][1,4]diazepin-5-one (1-5) (1.1 g, yield: 62%).
**LCMS:** Rt: 1.499 min; MS m/z (ESI): 269.0 [M+H].

Preparation of compound 1-6:

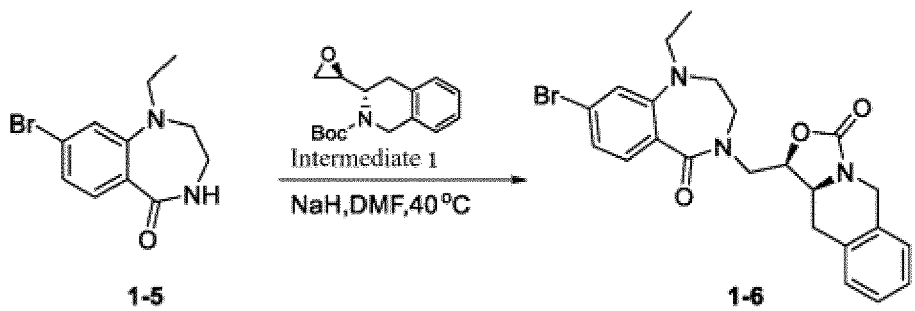


1-5

1-6

At room temperature, 8-bromo-1-ethyl-1,2,3,4-tetrahydro-5H-benzo[e][1,4]diazepin-5-one (1-5) (400 mg, 1.52 mmol) was added to ultradry N,N-dimethylformamide (20 mL) and then sodium hydride (90.9 mg, 2.27 mmol) was added. After the addition, the mixture was heated to 40°C and stirred for 1 h. Then intermediate 1 (500 mg, 1.82 mmol) prepared in example 1 was added, and the resulting mixture was reacted for 16 hours. Upon completion of reaction, water (100 mL) was added, and the mixture was extracted with ethyl acetate (100 mL X 2). The organic phases were combined, washed with saturated brine (50 mL X 2), dried over anhydrous sodium sulfate, and filtered. The filtrate was concentrated under reduced pressure. The residue was purified using a normal phase silica gel chromatographic column (eluent gradient: dichloromethane/methanol = 20/1) to afford the target intermediate (1R,10aS)-1-((8-bromo-1-ethyl-5-oxo-1,2,3,5-tetrahydro-4H-benzo[e][1,4]diazepin-4-yl)methyl)-1,5,10,10a-tetrahydro-3H-oxazolo[3,4-b]isoquinolin-3-one (1-6) (150 mg, yield: 21%). **LCMS:** Rt: 1.872 min; MS m/z (ESI): 470.1 [M+H].
Preparation of compound 1-7:

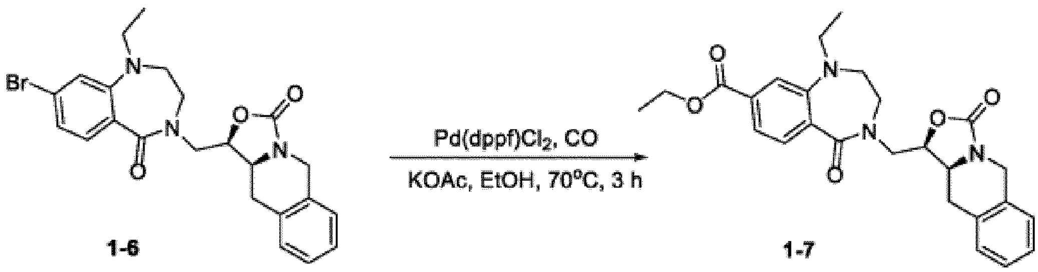


1-6

1-7

At room temperature, the intermediate (1R,10aS)-1-((8-bromo-1-ethyl-5-oxo-1,2,3,5-tetrahydro-4H-benzo[e][1,4]diazepin-4-yl)methyl)-1,5,10,10a-tetrahydro-3H-oxazolo[3,4-b]isoquinolin-3-one (1-6) (150 mg, 0.32 mmol), [1,1'-bis(diphenylphosphine)ferrocene]palladium dichloride (11.7 mg, 0.02 mmol) and potassium acetate (94 mg, 0.96 mmol) were added to anhydrous ethanol (10 mL). The mixture was subjected to CO replacement three times, heated to 70°C and reacted for 3.0 hours. Upon completion of reaction, the reaction was cooled to room temperature, and the reaction solution was concentrated under reduced pressure. Saturated brine (50 mL) was added and then the mixture was extracted with ethyl acetate (50 mL X 2). The organic phase was dried over anhydrous sodium sulfate and filtered. The filtrate was concentrated under reduced pressure. The residue was purified using a normal phase silica gel chromatographic column (eluent gradient: petroleum ether/ethyl acetate = 1/2) to afford the target intermediate ethyl 1-ethyl-5-oxo-4-(((1R,10aS)-3-carbonyl-1,5,10,10a-tetrahydro-3H-oxazolo[3,4-b]isoquinolin-1-yl)methyl)-2,3,4,5-tetrahydro-1H-benzo[e][1,4]diazepine-8-carboxylate (1-7) (130 mg, yield: 87%).
**LCMS:** Rt: 1.784 min; MS m/z (ESI): 464.1 [M+H].

Preparation of compound 1-8:

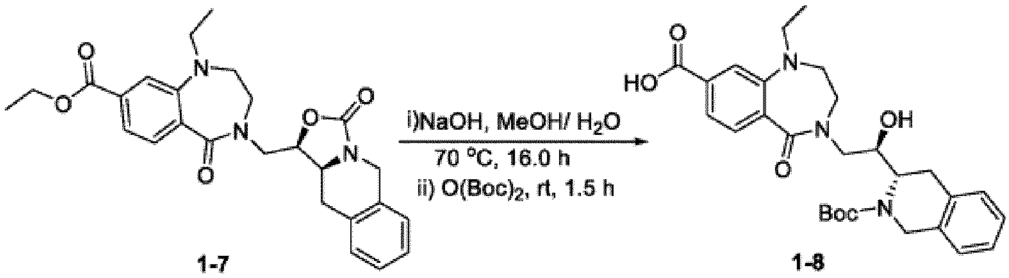


1-7 1-8

At room temperature, ethyl 1-ethyl-5-oxo-4-(((1R,10aS)-3-carbonyl-1,5,10,10a-tetrahydro-3H-oxazolo[3,4-b]isoquinolin-1-yl)methyl)-2,3,4,5-tetrahydro-1H-benzo[e][1,4]diazepine-8-carboxylate (1-7) (130 mg, 0.28 mmol) was added to a mixed solution of methanol (4 mL) and water (4 mL), and sodium hydroxide (90 mg, 2.24 mmol) was added then. The mixture was heated to 70°C and reacted for 16.0 hours. Upon completion of reaction, the reaction system was cooled to room temperature and Boc anhydride (122 mg, 0.56 mmol) was added. The resulting mixture was reacted for 1.5 hours. Upon completion of reaction, the reaction system was cooled to 0°C. The reaction solution was adjusted with 1 mol/L hydrochloric acid aqueous solution to the pH of 5.0 and then extracted with ethyl acetate (30 mL X 3). The organic phase was dried over anhydrous sodium sulfate and filtered. The filtrate was concentrated under reduced pressure. The residue was subjected to a reverse-phase silica gel chromatographic column (eluent gradient: acetonitrile/water = 43%) to afford the target intermediate 4-((R)-2-((S)-2-(tert-butoxycarbonyl)-1,2,3,4-tetrahydroisoquinolin-3-yl)-2-hydroxyethyl)-1-ethyl-5-oxo-2,3,4,5-tetrahydro-1H-benzo[e][1,4]diazepine-8-carboxylic acid (1-8) (crude, 60 mg, yield: 42%).

**LCMS:** Rt: 1.722 min; MS m/z (ESI): 510.3 [M+H].

Single crystal X-ray structure determination and single crystal X-ray analysis for compound 1-8:

Method for preparing the single crystal: Compound 1-8 (10.0 mg) was weighed and placed into a 3 mL screw neck glass bottle and methanol (2 mL) was added. After the mixture was stirred for 5 minutes, the solid was dissolved and the mixture was clarified. 0.5 mL of water was added into the glass bottle and the mixture was further stirred for 5 minutes. The solution was filtered through 0.22 μm microporous filter membrane into a 3 mL screw neck glass bottle and the glass bottle was covered with a preservative film. 8 small holes were made by a needle on the film covering the bottle. The bottle was placed at room temperature for 7 days to obtain the above-mentioned single crystal of the compound.

X-ray analysis was carried out on the single crystal sample obtained. The test results are shown in Table 1 and Figure 1.

Table 1 Single crystal sample of compound 1-8 and crystal data

| | |
|---|---|
| Chemical formula | $C_{28}H_{35}N_3O_6.H_2O$ |
| Chemical formula weight | 527.60 |
| Crystal system | Monoclinic form |
| Space group | P1 21 1 |
| a, Å | a = 6.0902(12) |
| b, Å | b = 9.601(2) |
| c, Å | c = 23.026(5) |
| $\alpha$, deg | 90 |
| $\beta$, deg | 90.334(6) |
| $\gamma$, deg | 90 |
| Volume, $Å^3$ | 1346.4(5) |
| Z | 2 |
| $D_{calc}$, g $cm^{-3}$ | 1.301 |
| temperature, K | 100(2) |
| Radiation (wavelength) | Mo K$\alpha$ (0.71073) |
| Instruments | BRUKER D8 VENTURE PHOTON II |

(continued)

| | |
|---|---|
| Chemical formula | $C_{28}H_{35}N_3O_6.H_2O$ |
| h, k, 1 ranges | -8< = h <= 7, -12 <= k <= 12, -30 <= 1 <= 30 |
| θ range | 2.298-28.383 |
| Program | SHELXL-2014 |
| Data acquired | 28356 |
| Unique data | 6667 |
| R ($F_o$) | 0.0676 |
| Rw ($F_o^2$) | 0.1957 |
| Goodness of fit | 1.018 |
| Absolute configuration determination | Flack parameter (0.0(2)) |

From the above-mentioned X-ray crystal diffraction experiment, it can be determined that the chemical structure and absolute configuration of compound 1-8 is as follows:

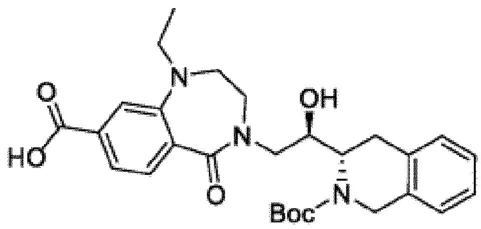

Preparation of compound 1-9:

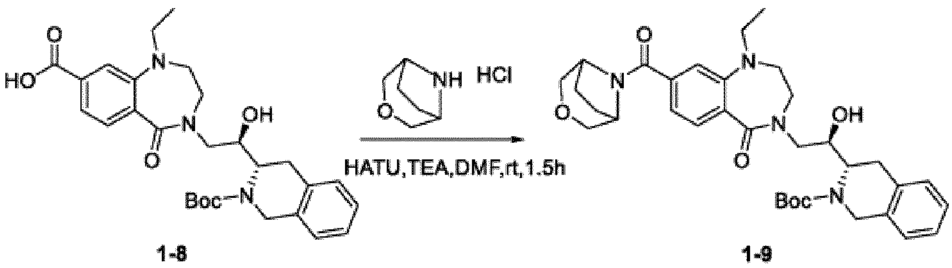


1-8 1-9

At room temperature, 4-((R)-2-((S)-2-(tert-butoxycarbonyl)-1,2,3,4-tetrahydroisoquinolin-3-yl)-2-hydroxyethyl)-1-ethyl-5-oxo-2,3,4,5-tetrahydro-1H-benzo[e][1,4]diazepine-8-carboxylic acid (1-8) (60 mg, 0.12 mmol), 2-(7-azabenzotriazole)-N,N,N',N'-tetramethylurea hexafluorophosphate (89.4 mg, 0.24 mmol) and triethylamine (47.5 mg, 0.47 mmol) were added to ultradry N,N-dimethylformamide (5.0 mL), then 3-oxa-8-azabicyclo[3.2.1]octane hydrochloride (26.3 mg, 0.18 mmol) was added, and the mixture was reacted at room temperature for 1.5 hours. Upon completion of reaction, saturated brine (30 mL) was added, and the resulting mixture was extracted with ethyl acetate (30 mL X 2). The organic phase was washed with saturated brine (50 mL), dried over anhydrous sodium sulfate and filtered. The filtrate was concentrated under reduced pressure. The residue was purified using a normal phase silica gel chromatographic column (eluent gradient: petroleum ether/ethyl acetate = 1/2) to afford the target intermediate tert-butyl (3S)-3-((1R)-2-(8-(3-oxa-8-azabicyclo[3.2.1]octane-8-carbonyl)-1-ethyl-5-oxo-1,2,3,5-tetrahydro-4H-benzo[e][1,4]diazepin-4-yl)-1-hydroxyethyl)-3,4-dihydroisoquinoline-2(1H)-carboxylate (1-9) (20 mg, yield: 28%). **LCMS:** Rt: 1.779 min; MS m/z (ESI): 605.2 [M+H].

Preparation of compound 001:

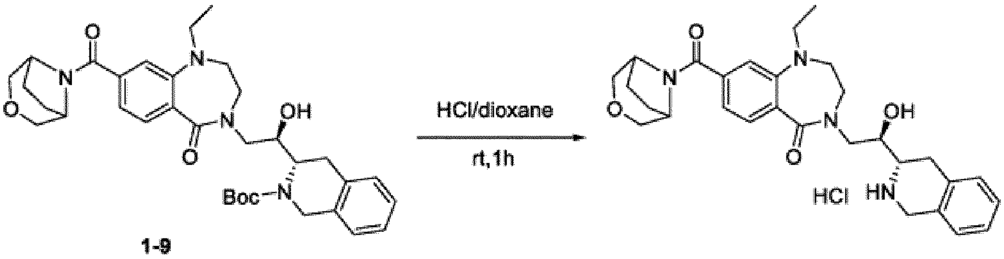


1-9

At 0°C, 4 mol/L hydrochloric acid/dioxane solution (1.0 mL) was added to tert-butyl (3S)-3-((1R)-2-(8-(3-oxa-8-azabicyclo[3.2.1]octane-8-carbonyl)-1-ethyl-5-oxo-1,2,3,5-tetrahydro-4H-benzo[e][1,4]diazepin-4-yl)-1-hydroxyethyl)-3,4-dihydroisoquinoline-2(1H)-carboxylate (1-9) (20 mg, 0.033 mmol), and the mixture was reacted for 1 hour. Upon completion of reaction, the reaction solution was concentrated under reduced pressure. The residue was purified by high-performance liquid preparative chromatography (eluent gradient:

| Instruments | Waters 2767 | | |
|---|---|---|---|
| Mobile phase | A: 0.1% HCl in $H_2O$, B: $CH_3CN$ | | |
| Chromatographic column | Waters sunfire C18 19*250 mm 10 μm | | |
| Injection volume | 500 μL | | |
| Table of running gradients | Time (min) | Mobile phase A (%) | Mobile phase B (%) |
| | 0 | 90 | 5 |
| | 1 | 80 | 20 |
| | 11 | 60 | 40 |
| | 11.2 | 5 | 95 |
| | 13 | 5 | 95 |
| | 13.2 | 95 | 5 |
| | 15 | 95 | 5 |

) to afford the target compound 8-(3-oxa-8-azabicyclo[3.2.1]octane-8-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 001) (3.65 mg, yield: 20%). **$^1$H NMR** (400 MHz, $CD_3OD$): $\delta$ 7.65-7.63 (m, 1 H), 7.31-7.16 (m, 6 H), 4.64 (s, 1 H), 4.50-4.36 (m, 2 H), 4.33-4.29 (m, 1 H), 3.99-3.96 (m, 2 H), 3.84-3.77 (m, 1 H), 3.79-3.57 (m, 8 H), 3.47-3.46 (m, 1 H), 3.34-3.30 (m, 2 H), 3.29-3.25 (m, 2 H), 2.05-1.99 (m, 4 H), 1.18-1.14 (m, 3 H).
**LCMS:** Rt: 0.798 min; MS m/z (ESI): 505.2 [M+H].

Example 3. Preparation of 1-ethyl-4-((R)-2-hydroxy-2-((S)-1,2,3,4-tetrahydroisoquinolin-3-yl)ethyl)-8-(2-methoxy-7-azaspiro[3.5]nonane-7-carbonyl)-1,2,3,4-tetrahydro-5H-benzo[e][1,4]diazepin-5-one hydrochloride (compound 002)

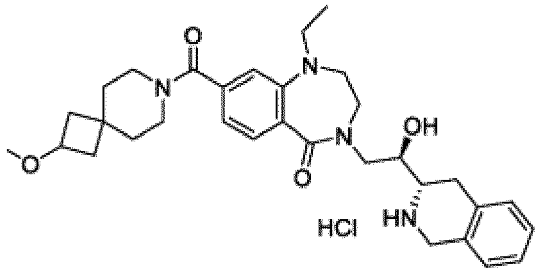

002

Preparation of compound 2-2:

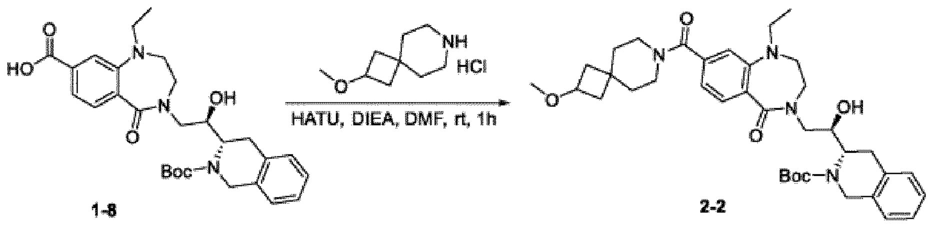


At room temperature, 4-((R)-2-((S)-2-(tert-butoxycarbonyl)-1,2,3,4-tetrahydroisoquinolin-3-yl)-2-hydroxyethyl)-1-ethyl-5-oxo-2,3,4,5-tetrahydro-1H-benzo[e][1,4]diazepine-8-carboxylic acid (1-8) (100 mg, 0.196 mmol), 2-(7-azabenzotriazole)-N,N,N',N'-tetramethylurea hexafluorophosphate (231 mg, 0.608 mmol) and N,N-diisopropylethylamine (157 mg, 1.216 mmol) were added to ultradry N,N-dimethylformamide (2.5 mL), then 2-methoxy-7-azaspiro[3.5]nonane hydrochloride (53 mg, 0.275 mmol) was added, and the mixture was stirred at room temperature for 1.0 hour. Upon completion of reaction, water (30 mL) was added and then the resulting mixture was extracted with ethyl acetate (30 mL X 3). The organic phases were combined, washed with saturated brine (50 mL), dried over anhydrous sodium sulfate and filtered. The filtrate was concentrated under reduced pressure. The residue was purified using a normal phase silica gel chromatographic column (eluents: dichloromethane/methanol = 20/1) to afford the target intermediate tert-butyl (S)-3-((R)-2-(1-ethyl-8-(2-methoxy-7-azaspiro[3.5]nonane-7-carbonyl)-5-oxo-1,2,3,5-tetrahydro-4H-benzo[e][1,4]diazepin-4-yl)-1 -hydroxyethyl)-3,4-dihydroisoquinoline-2(1H)-carboxylate (2-2) (110 mg, yield: 86.7%).
**LCMS:** Rt: 2.062 min; MS m/z (ESI): 647.3 [M+H].

Preparation of compound 002:

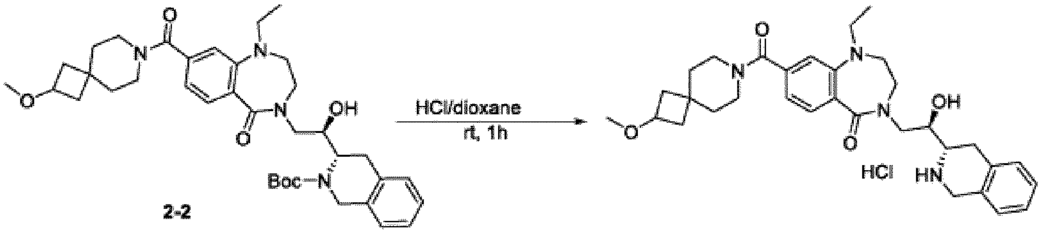


At room temperature, tert-butyl (S)-3-((R)-2-(1-ethyl-8-(2-methoxy-7-azaspiro[3.5]nonane-7-carbonyl)-5-oxo-1,2,3,5-tetrahydro-4H-benzo[e][1,4]diazepin-4-yl)-1-hydroxyethyl)-3,4-dihydroisoquinoline-2(1H)-carboxylate (2-2) (110 mg, 0.170 mmol) was added to a solution of 4 mol/L hydrochloric acid/dioxane (2.5 mL). The mixture was stirred and reacted at room temperature for 1.0 hour. Upon completion of reaction, the reaction solution was concentrated under reduced pressure and the residue was purified by high-performance liquid preparative chromatography (refer to example 2 for the eluents and gradient) to afford the target compound 1-ethyl-4-((R)-2-hydroxy-2-((S)-1,2,3,4-tetrahydroisoquinolin-3-yl)ethyl)-8-(2-methoxy-7-azaspiro[3.5]nonane-7-carbonyl)-1,2,3,4-tetrahydro-5H-benzo[e][1,4]diazepin-5-one hydrochloride (compound 002) (39.81 mg, yield: 42.8%).

**$^1$H NMR** (400 MHz, $CD_3OD$): $\delta$7.64 (d, $J$ = 7.6 Hz, 1 H), 7.35-7.22 (m, 4 H), 7.12 (d, $J$ = 8 Hz, 2 H), 4.51-4.36 (m, 2 H), 4.33-4.30 (m, 1 H), 4.04-3.89 (m, 2 H), 3.73-3.61 (m, 7 H), 3.51-3.50 (m, 1 H), 3.38-3.34 (m, 4 H), 3.31-3.30 (m, 2 H), 3.22 (s, 3 H), 2.29-2.23 (m, 2 H), 1.71-1.68 (m, 4 H),1.55 (s, 2H), 1.17 (t, $J$ = 7.0 Hz, 3 H).
**LCMS:** Rt: 1.355 min; MS m/z (ESI): 547.4 [M+H].

Example 4. Preparation of 7-(1-ethyl-4-((R)-2-hydroxy-2-((S)-1,2,3,4-tetrahydroisoquinolin-3-yl)ethyl)-5-oxo-2,3,4,5-tetrahydro-1H-benzo[e][1,4]diazepine-8-carbonyl)-7-azaspiro[3.5]nonane-2-carbonitrile (compound 003)

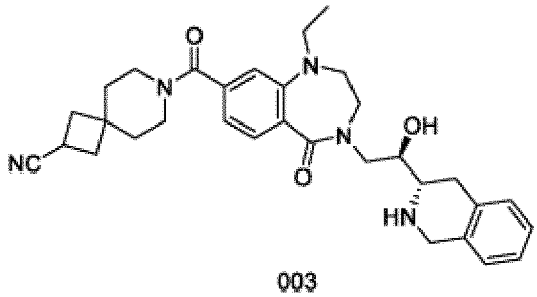

003

Preparation of compound 3-2:

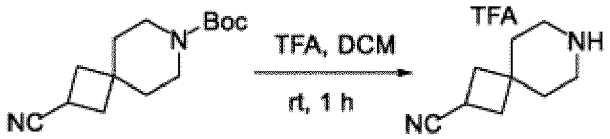


3-1 3-2

At room temperature, tert-butyl 2-cyano-7-azaspiro[3.5]nonane-7-carboxylate (3-1) (600 mg, 2.4 mmol) was dissolved in dichloromethane (2 mL), trifluoroacetic acid (1 mL) was slowly added, and the reaction solution was stirred at room temperature for 1 hour. Upon completion of reaction, the reaction solution was concentrated under reduced pressure to afford the intermediate 7-azaspiro[3.5]nonane-2-carbonitrile trifluoroacetate (3-2) (crude, 590 mg, yield: 100%).

Reference can be made to the synthetic method in example 2 for the other steps, and 7-azaspiro[3.5]nonane-2-carbonitrile trifluoroacetate (3-2) was used in place of 3-oxa-8-azabicyclo[3.2.1]octane hydrochloride. The final hydrochloride product prepared was dissolved in dichloromethane and neutralized and washed with saturated sodium bicarbonate aqueous solution. The organic phase was dried over anhydrous sodium sulfate and filtered. The filtrate was concentrated under reduced pressure to afford the target product 7-(1-ethyl-4-((R)-2-hydroxy-2-((S)-1,2,3,4-tetrahydroisoquinolin-3-yl)ethyl)-5-oxo-2,3,4,5-tetrahydro-1H-benzo[e][1,4]diazepine-8-carbonyl)-7-azaspiro[3.5]nonane-2-carbonitrile (compound 003).

**$^1$H NMR** (400 MHz, $CD_3OD$): $\delta$7.55 (d, $J$ = 7.7 Hz, 1 H), 7.13-7.09 (m, 3 H), 7.04-7.02 (m, 1 H), 6.98-6.95 (m, 2 H), 4.06-3.87 (m, 4 H), 3.80-3.75 (m, 1 H), 3.65-3.60 (m, 4 H), 3.51-3.49 (m, 1 H), 3.37-3.31 (m, 3 H), 3.29-3.18 (m, 3 H), 3.02-2.83 (m, 3 H), 2.34-2.32 (m, 2 H), 2.19-2.16 (m, 2 H), 1.75-1.62 (m, 4H), 1.11 (t, $J$ = 7.2 Hz, 3 H).
**LCMS:** Rt: 1.324 min; MS m/z (ESI): 542.3 [M+H].

Example 5. Preparation of 1-ethyl-4-((R)-2-hydroxy-2-((S)-1,2,3,4-tetrahydroisoquinolin-3-yl)ethyl)-8-(3-methoxy-8-azabicyclo[3.2.1]octane-8-carbonyl)-1,2,3,4-tetrahydro-5H-benzo[e][1,4]diazepin-5-one hydrochloride (compound 004)

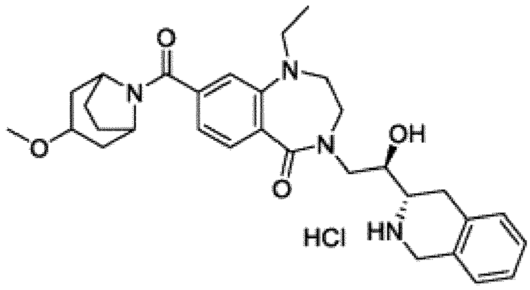

Reference can be made to the synthetic method in example 2 and 3-methoxy-8-azabicyclo[3.2.1]octane hydrochloride was used in place of 3-oxa-8-azabicyclo[3.2.1]octane hydrochloride to afford the target product 1-ethyl-4-((R)-2-hydroxy-2-((S)-1,2,3,4-tetrahydroisoquinolin-3-yl)ethyl)-8-(3-methoxy-8-azabicyclo[3.2.1]octane-8-carbonyl)-1,2,3,4-tetrahydro-5H-benzo[e][1,4]diazepin-5-one hydrochloride (compound 004).

**$^1$H NMR** (400 MHz, $CD_3OD$): $\delta$ 7.68 (d, *J* = 7.6 Hz, 1 H), 7.30-7.22 (m, 6 H), 4.68 (s, 1 H), 4.51-4.37 (m, 2 H), 4.33-4.31 (m, 1 H), 4.03-3.98 (m, 2 H), 3.74-3.62 (m, 5 H), 3.60-3.50 (m, 2 H), 3.44-3.32 (m, 2 H), 3.30 (s, 3 H), 3.28-3.22 (m, 2 H), 2.25-2.07 (m, 4 H), 2.01-1.89 (m, 4 H), 1.19 *(t, J* = 6.8 Hz, 3 H).
**LCMS:** Rt: 1.347 min; MS m/z (ESI): 533.2 [M+H].

Example 6. Preparation of 4-((R)-2-hydroxy-2-((S)-1,2,3,4-tetrahydroisoquinolin-3-yl)ethyl)-8-(3-methoxy-8-azabicyclo[3.2.1]octane-8-carbonyl)-1-methyl-1,2,3,4-tetrahydro-5H-benzo[e][1,4]diazepin-5-one hydrochloride (compound 005)

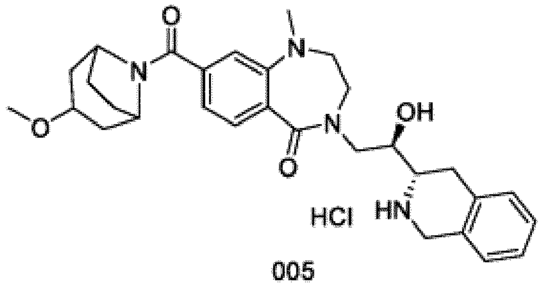

005

Preparation of compound 5-2:

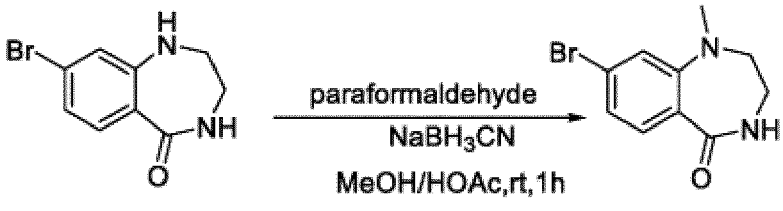


1-4 5-2

At room temperature, 8-bromo-1,2,3,4-tetrahydro-5H-benzo[e][1,4]diazepin-5-one (1-4) (500 mg, 2.07 mmol), sodium cyanoborohydride (260.7 mg, 4.15 mmol) and paraformaldehyde (125 mg, 4.15 mmol) were added to a mixed solution of acetic acid (1 mL) and methanol (10 mL). The mixture was reacted at room temperature for 1 hour. Upon completion of reaction, 2 mol/L dilute hydrochloric acid (1 mL) and water (50 mL) were added and the mixture was extracted with ethyl acetate (50 mL X 2). The organic phases were combined, washed with saturated brine (40 mL X 3), dried over anhydrous sodium sulfate, and filtered. The filtrate was concentrated under reduced pressure. The residue

was purified using a normal phase silica gel chromatographic column (eluent gradient: petroleum ether/ethyl acetate = 1/2) to afford the target intermediate 8-bromo-1-methyl-1,2,3,4-tetrahydro-5H-benzo[e][1,4]diazepin-5-one (5-2) (400 mg, yield: 76%).

**LCMS:** Rt: 1.359 min; MS m/z (ESI): 255.0 [M+H].

Preparation of compound 5-3:

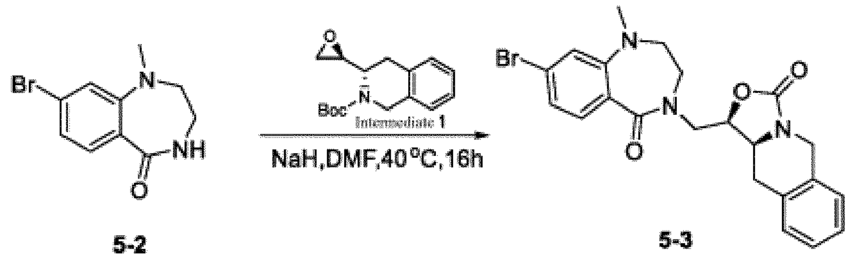


5-2 5-3

At room temperature, 8-bromo-1-methyl-1,2,3,4-tetrahydro-5H-benzo[e][1,4]diazepin-5-one (5-2) (400 mg, 1.57 mmol) was added to ultradry N,N-dimethylformamide (40 mL) and then sodium hydride (75.3 mg, 1.88 mmol) was added. After the addition, the mixture was heated to 40°C and stirred for 1 hour. Then tert-butyl (S)-3-((S)-oxiran-2-yl)-3,4-dihydroisoquinoline-2(1H)-carboxylate (intermediate 1) (518 mg, 1.88 mmol) was added, and the resulting mixture was reacted for 16 hours. Upon completion of reaction, water (120 mL) was added, and the reaction was extracted with ethyl acetate (100 mL X 2). The organic phases were combined, washed with saturated brine (50 mL X 2), dried over anhydrous sodium sulfate, and filtered. The filtrate was concentrated under reduced pressure. The residue was purified using normal phase silica gel chromatographic column (eluent gradient: petroleum ether/ethyl acetate = 1/1) to afford the target intermediate (1R,10aS)-1-((8-bromo-1-methyl-5-oxo-1,2,3,5-tetrahydro-4H-benzo[e][1,4]diazepin-4-yl)methyl)-1,5,10,10a-tetrahydro-3H-oxazolo[3,4-b]isoquinolin-3-one (5-3) (520 mg, yield: 73%).
**LCMS:** Rt: 1.749 min; MS m/z (ESI): 456.0 [M+H].

Preparation of compound 5-4:

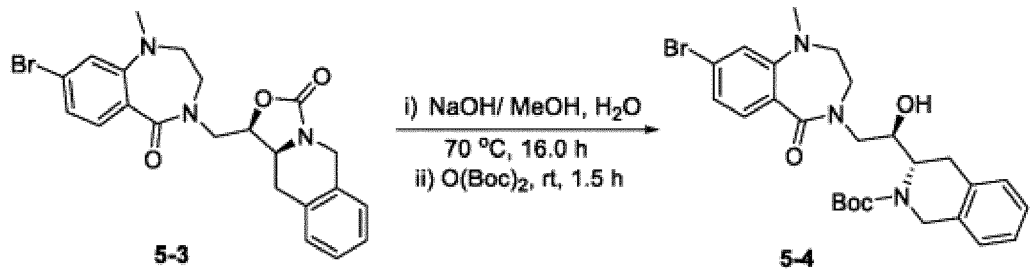


5-3 5-4

At room temperature, (1R,10aS)-1-((8-bromo-1-methyl-5-oxo-1,2,3,5-tetrahydro-4H-benzo[e][1,4] diazepin-4-yl)methyl)-1, 5,10,10a-tetrahydro-3H-oxazolo[3,4-b]isoquinolin-3-one (5-3) (520 mg, 1.14 mmol) was added to a mixed solution of methanol (5 mL) and water (5 mL), and sodium hydroxide (274 mg, 6.84 mmol) was added. The mixture was heated to 70°C and reacted for 16.0 hours. Upon completion of reaction, the reaction system was cooled to room temperature and Boc anhydride (497 mg, 2.28 mmol) was added. The resulting mixture was reacted for 1.5 hours. Upon completion of reaction, the mixture was extracted with ethyl acetate (30 mL X 3), and the organic phase was dried over anhydrous sodium sulfate and filtered. The filtrate was concentrated under reduced pressure. The residue was purified using a normal phase silica gel chromatographic column (eluent gradient: petroleum ether/ethyl acetate = 1/1) to afford the target intermediate tert-butyl (S)-3-((R)-2-(8-bromo-1-methyl-5-oxo-1,2,3,5-tetrahydro-4H-benzo[e][1,4]diazepin-4-yl)-1-hydroxyethyl)-3,4-dihydroisoquinoline-2(1H)-carboxylate (5-4) (530 mg, yield: 88%).
**LCMS:** Rt: 2.088 min; MS m/z (ESI): 530.1 [M+H].

Preparation of compound 5-5:

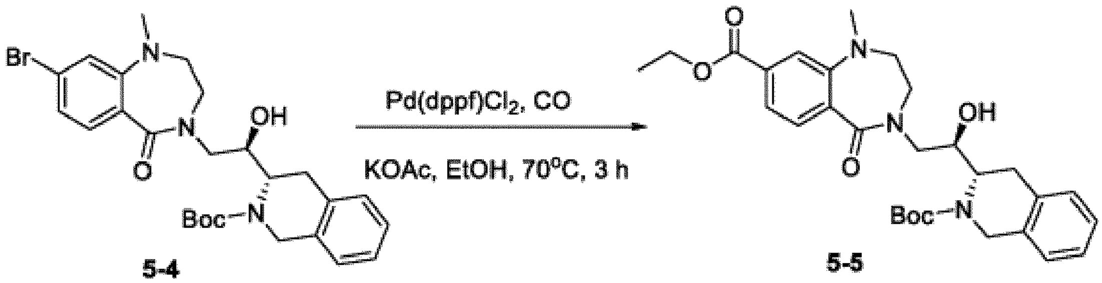


5-4 5-5

At room temperature, tert-butyl (S)-3-((R)-2-(8-bromo-1-methyl-5-oxo-1,2,3,5-tetrahydro-4H-benzo[e][1,4]diazepin-4-yl)-1-hydroxyethyl)-3,4-dihydroisoquinoline-2(1H)-carboxylate (5-4) (530 mg, 1 mmol), [1,1'-bis(diphenylphosphine)ferrocene]palladium dichloride dichloromethane complex (38 mg, 0.05 mmol) and potassium acetate (294 mg, 3 mmol) were added to anhydrous ethanol (15 mL). The mixture was subjected to CO replacement three times, heated to 70°C and reacted for 3.0 hours. Upon completion of reaction, the reaction was cooled to room temperature, and the reaction solution was concentrated under reduced pressure. Saturated brine (50 mL) was added and then the mixture was extracted with ethyl acetate (50 mL X 2). The organic phase was dried over anhydrous sodium sulfate and filtered, and the filtrate was concentrated under reduced pressure. The residue was purified using a normal phase silica gel chromatographic column (eluent gradient: petroleum ether/ethyl acetate = 1/1) to afford the target intermediate ethyl 4-((R)-2-((S)-2-(tert-butoxycarbonyl)-1,2,3,4-tetrahydroisoquinolin-3-yl)-2-hydroxyethyl)-1-methyl-5-oxo-2,3,4,5-tetrahydro-1H-benzo[e][1,4]diazepine-8-carboxylate (5-5) (500 mg, yield: 96%).
**LCMS:** Rt: 1.965 min; MS m/z (ESI): 524.2 [M+H].

Preparation of compound 5-6:

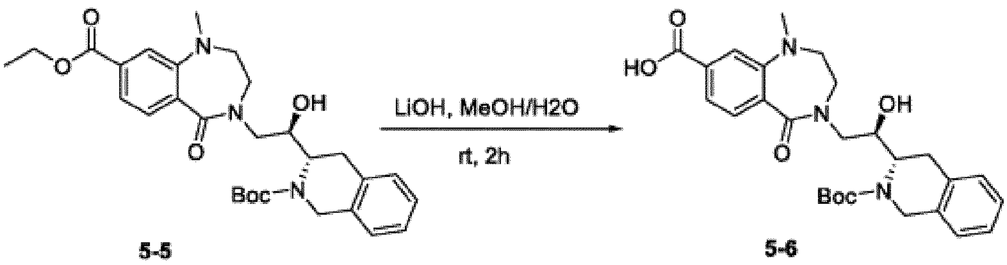


5-5 5-6

At room temperature, the intermediate ethyl 4-((R)-2-((S)-2-(tert-butoxycarbonyl)-1,2,3,4-tetrahydroisoquinolin-3-yl)-2-hydroxyethyl)-1-methyl-5-oxo-2,3,4,5-tetrahydro-1H-benzo[e][1,4]diazepine-8-carboxylate (5-5) (500 mg, 0.96 mmol) was added to a mixed solution of methanol (5 mL) and water (5 mL), and lithium hydroxide monohydrate (161 mg, 3.82 mmol) was added. The mixture was reacted at room temperature for 2 hours. Upon completion of reaction, the reaction solution was adjusted with 1 mol/L hydrochloric acid aqueous solution to the pH of 5.0 and extracted with ethyl acetate (30 mL X 3), and the organic phase was dried over anhydrous sodium sulfate and filtered. The filtrate was concentrated under reduced pressure to afford the target intermediate 4-((R)-2-((S)-2-(tert-butoxycarbonyl)-1,2,3,4-tetrahydroisoquinolin-3-yl)-2-hydroxyethyl)-1-methyl-5-oxo-2,3,4,5-tetrahydro-1H-benzo[e][1,4]diazepine-8-carboxylic acid (5-6) (420 mg, yield: 88%).
**LCMS:** Rt: 1.686 min; MS m/z (ESI): 496.2 [M+H].

Preparation of compound 5-7:

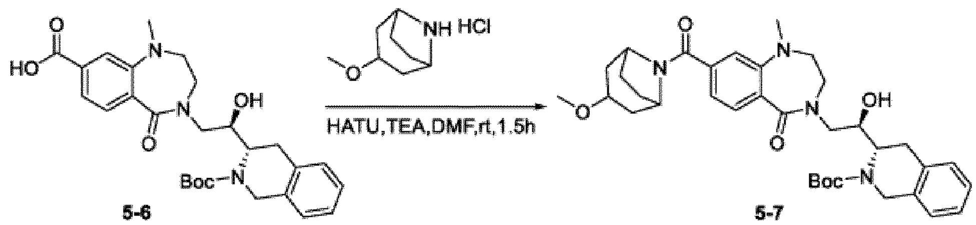


5-6 5-7

At room temperature, 4-((R)-2-((S)-2-(tert-butoxycarbonyl)-1,2,3,4-tetrahydroisoquinolin-3-yl)-2-hydroxyethyl)-1-methyl-5-oxo-2,3,4,5-tetrahydro-1H-benzo[e][1,4]diazepine-8-carboxylic acid (5-6) (70 mg, 0.14 mmol), 2-(7-azabenzotriazole)-N,N,N',N'-tetramethylurea hexafluorophosphate (106 mg, 0.28 mmol) and triethylamine (57 mg, 0.56 mmol) were added to ultradry N,N-dimethylformamide (5.0 mL), and 3-methoxy-8-azabicyclo[3.2.1]octane hydrochloride (30 mg, 0.17 mmol) was added. The mixture was reacted at room temperature for 1.5 hours. Upon completion of reaction, saturated brine (30 mL) was added, and the resulting mixture was extracted with ethyl acetate (30 mL X 2). The organic phase was washed with saturated brine (30 mL), dried over anhydrous sodium sulfate and filtered. The filtrate was concentrated under reduced pressure. The residue was purified using a normal phase silica gel chromatographic column (eluent: ethyl acetate) to afford the target intermediate tert-butyl (3S)-3-((1R)-1-hydroxyl-2-(8-(3-methoxy-8-azabicyclo[3.2.1]octane-8-carbonyl)-1-methyl-5-oxo-1,2,3,5-tetrahydro-4H-benzo[e][1,4]diazepin-4-yl)ethyl)-3,4-dihydroisoquinoline-2(1H)-carboxylate (5-7) (65 mg, yield: 75%).
**LCMS:** Rt: 1.831 min; MS m/z (ESI): 619.3 [M+H].

Preparation of compound 005:

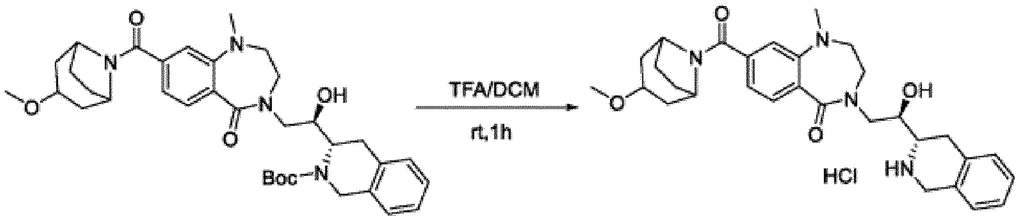


5-7

At room temperature, tert-butyl (3S)-3-((1R)-1-hydroxyl-2-(8-(3-methoxy-8-azabicyclo[3.2.1]octane-8-carbonyl)-1-methyl-5-oxo-1,2,3,5-tetrahydro-4H-benzo[e][1,4]diazepin-4-yl)ethyl)-3,4-dihydroisoquinoline-2(1H)-carboxylate (5-7) (65 mg, 0.1 mmol) was added to dichloromethane (2 mL), then trifluoroacetic acid (1 ml) was added. The mixture was reacted at room temperature for 1 hour. Upon completion of reaction, the reaction solution was concentrated under reduced pressure. The residue was purified by high-performance liquid preparative chromatography (referring to example 2 for the eluents and gradient) to afford the target compound 4-((R)-2-hydroxy-2-((S)-1,2,3,4-tetrahydroisoquinolin-3-yl)ethyl)-8-(3-methoxy-8-azabicyclo[3.2.1]octane-8-carbonyl)-1-methyl-1,2,3,4-tetrahydro-5H-benzo[e][1,4]diazepin-5-one hydrochloride (compound 5) (20.33 mg, yield: 37%).

**$^1$H NMR** (400 MHz, $CD_3OD$): $\delta$7.67-7.65 (m, 1 H), 7.31-7.19 (m, 6 H), 4.69 (s, 1 H), 4.50-4.33 (m, 3 H), 4.05-4.00 (m, 2 H), 3.70-3.60 (m, 4 H), 3.57-3.52 (m, 3 H), 3.35-3.32 (m, 1 H), 3.28-3.22 (m, 4 H), 2.98 (s, 3 H), 2.23-2.06 (m, 4 H), 2.00-1.90 (m, 4 H). **LCMS:** Rt: 1.271 min; MS m/z (ESI): 519.2 [M+H].

Example 7. Preparation of 1-ethyl-4-((R)-2-hydroxy-2-((S)-1,2,3,4-tetrahydroisoquinolin-3-yl)ethyl)-8-(9-methoxy-3-azaspiro[5.5]undecane-3-carbonyl)-1,2,3,4-tetrahydro-5H-benzo[e][1,4]diazepin-5-one hydrochloride (compound 006)

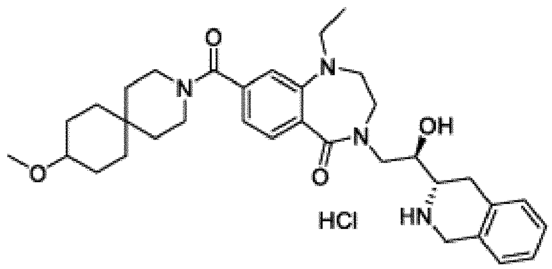

Preparation of compound 6-2:

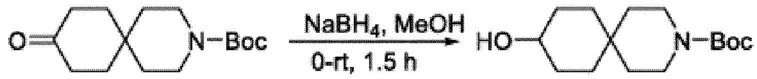


6-1 6-2

At room temperature, tert-butyl 9-oxo-3-azaspiro[5.5]undecane-3-carboxylate (6-1) (400 mg, 1.5 mmol) was added to methanol (10 mL), and at 0°C, sodium borohydride (171 mg, 4.5 mmol) was added. The mixture was reacted at room temperature for 1.5 hours. The reaction was quenched by adding an aqueous ammonium chloride solution (10 mL) and the reaction solution was extracted with ethyl acetate (50 mL X 2). The organic phases were combined, washed with saturated brine (50 mL), dried over anhydrous sodium sulfate, and filtered. The filtrate was concentrated under reduced pressure. The residue was purified using a normal phase silica gel chromatographic column (petroleum ether/ethyl acetate = 2/1) to afford tert-butyl 9-hydroxy-3-azaspiro[5.5]undecane-3-carboxylate (6-2) (305 mg, yield: 76%). **LCMS: Rt:** 1.570 min; MS m/z (ESI): 270.0 [M+H].

Preparation of compound 6-3:

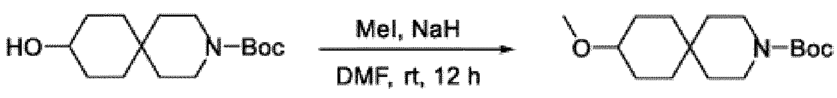


6-2 6-3

Tert-butyl 9-hydroxy-3-azaspiro[5.5]undecane-3-carboxylate (6-2) (540 mg, 2 mmol) was dissolved in N,N-dimethylformamide (10 mL) and the mixture was cooled to 0°C. Sodium hydride (320 mg, 8 mmol) was added in batches and then the mixture was reacted at 0°C for 1.0 hour. After iodomethane (568 mg, 4 mmol) was added, the resulting reaction solution was stirred at room temperature overnight. Upon completion of reaction, the reaction was quenched by adding saturated ammonium chloride solution (10 mL). The resulting reaction solution was diluted with saturated brine (50 mL) and extracted with ethyl acetate (20 mL X 2). The organic phase was washed with saturated brine (30 mL X 2), dried over anhydrous sodium sulfate and filtered. The filtrate was concentrated under reduced pressure to afford the target intermediate tert-butyl 9-methoxy-3-azaspiro[5.5]undecane-3-carboxylate (6-3) (crude, 560 mg, yield: 100%).

Preparation of compound 6-4:

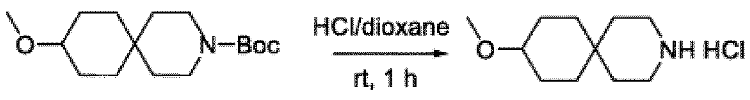


6-3 6-4

Tert-butyl 9-methoxy-3-azaspiro[5.5]undecane-3-carboxylate (6-3) (560 mg, 2 mmol) was dissolved in dioxane (1 mL) and the mixture was cooled to 0°C. 4 mol/L hydrochloric acid/dioxane solution (4 mL) was added and the mixture was reacted at 0°C for 1.0 hour. Upon completion of reaction, the reaction solution was concentrated under reduced pressure. The residue was slurried with methyl tert-butyl ether and then filtered to afford the target intermediate 9-methoxy-3-azaspiro[5.5]undecane hydrochloride (6-4) (192 mg, yield: 44%).
**LCMS:** Rt: 0.415 min; MS m/z (ESI): 184.2 [M+H].
Reference can be made to the synthetic method in example 2 for the other steps and 9-methoxy-3-azaspiro[5.5]undecane hydrochloride (6-4) was used in place of 3-oxa-8-azabicyclo[3.2.1]octane hydrochloride to afford the target product 1-ethyl-4-((R)-2-hydroxy-2-((S)-1,2,3,4-tetrahydroisoquinolin-3-yl)ethyl)-8-(9-methoxy-3-azaspiro[5.5]undecane-3-carbonyl)-1,2,3,4-tetrahydro-5H-benzo[e][1,4]diazepin-5-one hydrochloride (compound 006).

**$^1$H NMR(400** MHz, $CD_3OD$): $\delta$ 7.62-7.60 (m, 1 H), 7.31-7.21 (m, 4 H), 7.08-7.03 (m, 2 H), 4.47-4.38 (m, 2 H), 4.30-4.27 (m, 1 H), 3.99-3.95 (m, 1 H), 3.74-3.56 (m, 7 H), 3.43-3.38 (m, 3 H), 3.32-3.31 (m, 3 H), 3.27-3.22 (m, 5 H), 1.78-1.70 (m, 4 H), 1.63-1.57 (m, 1 H), 1.51-1.38 (m, 5 H), 1.32-1.23 (m, 2 H), 1.15 (t, $J$ = 7 Hz, 3 H).
**LCMS:** Rt: 1.347 min; MS m/z (ESI): 575.3 [M+H].

Example 8. Preparation of 8-(3-oxa-8-azabicyclo[3.2.1]octane-8-carbonyl)-4-((R)-2-hydroxy-2-((S)-1,2,3,4-tetrahydroisoquinolin-3-yl)ethyl)-3,4-dihydrobenzo[f][1,4]oxazepin-5(2H)-one (compound 007)

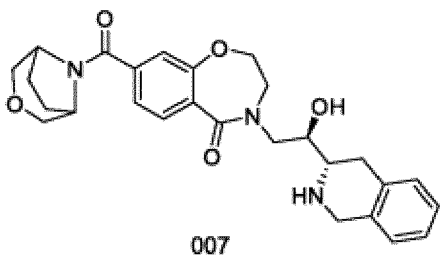

007

Preparation of compound 7-2:

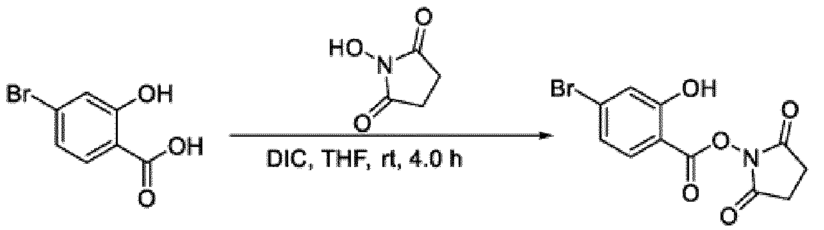


7-1

7-2

At room temperature, 4-bromo-2-hydroxybenzoic acid (7-1) (2.5 g, 11.5 mmol) and N-hydroxybutyldicarboxamide (2.65 g, 23.0 mmol) were added to ultradry tetrahydrofuran (75.0 mL), and then N,N-diisopropylcarbodiimide (2.9 g, 23.0 mmol) was added. The mixture was reacted at room temperature for 4.0 hours. Upon completion of reaction, the reaction solution was concentrated under reduced pressure. The residue was purified using a normal phase silica gel chromatographic column (eluent gradient: petroleum ether/ethyl acetate = 2/1) to afford the target intermediate 2,5-dicarbonylpyrrolidin-1-yl 4-bromo-2-hydroxybenzoate (7-2) (3.45 g, yield: 96%). **LCMS:** Rt: 1.546 min; MS m/z (ESI): 311.8[M-H].

Preparation of compound 7-4:

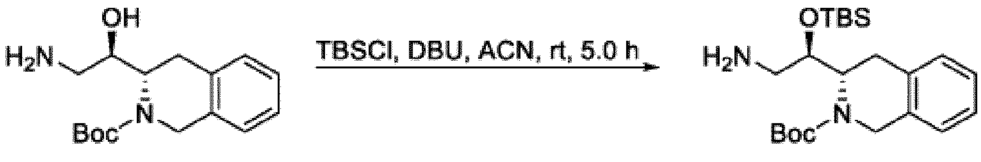


7-3 7-4

At room temperature, tert-butyl (S)-3-((R)-2-amino-1-hydroxyethyl)-3,4-dihydroisoquinoline-2(1H)-carboxylate (7-3, referring to US 10494376 B2 for the synthetic method) (1.0 g, 3.42 mmol) and 1,8-diazabicycloundec-7-ene (2.1 g, 13.68 mmol) were added to acetonitrile (30.0 mL), then at 0°C, tert-butyldimethylsilyl chloride (1.03 g, 6.85 mmol) was added. The mixture was reacted at room temperature for 5.0 hours. Upon completion of reaction, water (50 mL) was added, and the mixture was extracted with ethyl acetate (50 mL X 2). The organic phase was washed with saturated brine (30 mL X 3), dried over anhydrous sodium sulfate and filtered. The filtrate was concentrated under reduced pressure. The residue was purified using a normal phase silica gel chromatographic column (eluent gradient: dichloromethane/methanol = 50/1) to afford the target intermediate tert-butyl (S)-3-((R)-2-amino-1-((tert-butyldimethylsilyl)oxy)ethyl)-3,4-dihydroisoquinoline-2(1H)-carboxylate (7-4) (1.21 g, yield: 87%).
**LCMS:** Rt: 1.321 min; MS m/z (ESI): 407.1 [M+H].

Preparation of compound 7-5:

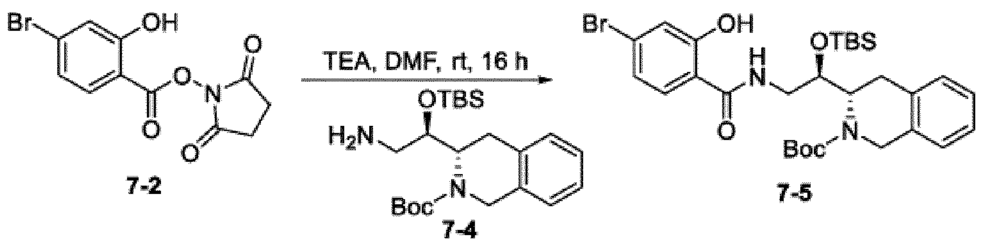


7-2 7-4 7-5

At room temperature, 2,5-dicarbonylpyrrolidin-1-yl 4-bromo-2-hydroxybenzoate (7-2) (773.0 mg, 2.46 mmol) and tert-butyl (S)-3-((R)-2-amino-1-((tert-butyldimethylsilyl)oxy)ethyl)-3,4-dihydroisoquinoline-2(1H)-carboxylate (7-4) (1.1 g, 2.71 mmol) were added to ultradry N,N-dimethylformamide (15.0 mL), and then triethylamine (1.8 mL) was added. The mixture was reacted at room temperature for 16.0 hours. Upon completion of reaction, water (100 mL) was added, and the mixture was extracted with ethyl acetate (100 mL X 2). The organic phase was washed with saturated brine, dried over anhydrous sodium sulfate and filtered. The filtrate was concentrated under reduced pressure. The residue was purified using a normal phase silica gel chromatographic column (eluent gradient: petroleum ether/ethyl acetate = 5/1) to afford the target intermediate tert-butyl (S)-3-((R)-2-(4-bromo-2-hydroxybenzamido)-1-((tert-butyl dimethylsilyl)oxy)ethyl)-3,4-dihydroisoquinoline-2(1H)-carboxylate (7-5) (1.1 g, yield: 74%).
**LCMS:** Rt: 1.814 min; MS m/z (ESI): 605.1 [M+H].

Preparation of compound 7-6:

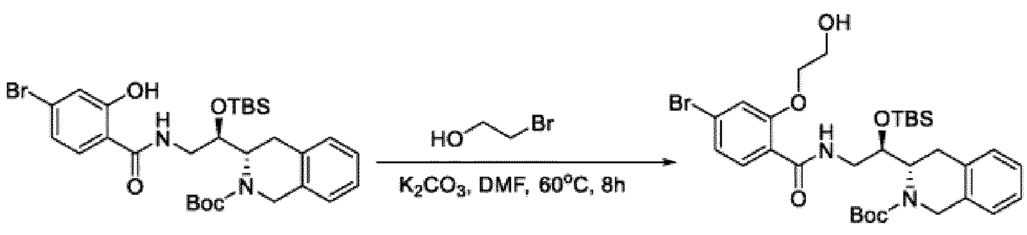


7-5 7-6

At room temperature, tert-butyl (S)-3-((R)-2-(4-bromo-2-hydroxybenzamido)-1-((tert-butyl dimethylsilyl)oxy)ethyl)-3,4-dihydroisoquinoline-2(1H)-carboxylate (7-5) (500.0 mg, 0.825 mmol) and 2-bromoethanol (231.0 mg, 1.85 mmol) were added to N,N-dimethylformamide (8.0 mL), and then potassium carbonate (340.0 mg, 2.46 mmol) was added. The mixture was warmed to 60°C and reacted for 8.0 hours. Upon completion of reaction, water (50 mL) was added, and the mixture was extracted with ethyl acetate (50 mL X 2). The organic phase was washed with saturated brine (30 mL X 3), dried over anhydrous sodium sulfate and filtered. The filtrate was concentrated under reduced pressure. The residue was purified using a normal phase silica gel chromatographic column (eluent gradient: petroleum ether/ethyl acetate = 3/1) to afford the target intermediate (S)-3-((R)-2-(4-bromo-2-(2-hydroxyethoxy)benzamido)-1-((tert-butyl dimethylsilyl)oxy)ethyl)-3,4-dihydroisoquinoline-2(1H)-carboxylate (7-6) (454 mg, yield: 85%).
**LCMS:** Rt: 2.40 min; MS m/z (ESI): 649.1 [M+H].

Preparation of compound 7-7:

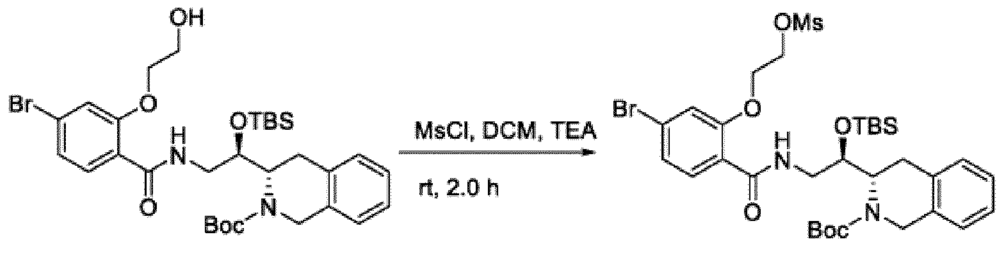


7-6 7-7

At room temperature, (S)-3-((R)-2-(4-bromo-2-(2-hydroxyethoxy)benzamido)-1-((tert-butyl dimethylsilyl)oxy)ethyl)-3,4-dihydroisoquinoline-2(1H)-carboxylate (7-6) (454 mg, 0.7 mmol) and triethylamine (212.0 mg, 2.1 mmol) were added to dichloromethane (10.0 mL), and the mixture was cooled to 0°C. Then methyl sulfonyl chloride (160.0 mg, 1.4 mmol) was added and the mixture was reacted at room temperature for 2.0 hours. Upon completion of reaction, water (50 mL) was added, and the mixture was extracted with dichloromethane (50 mL X 3). The organic phase was washed with saturated brine (50 mL), dried over anhydrous sodium sulfate and filtered. The filtrate was concentrated under reduced pressure. The residue was purified using a normal phase silica gel chromatographic column (eluent gradient: petroleum ether/ethyl acetate = 2/1) to afford the target intermediate tert-butyl (S)-3-((R)-2-(4-bromo-2-(2-((methanesulfonyl)oxy)ethoxy)benzamido)-1-((tert-butyl dimethylsilyl)oxy)ethyl)-3,4-dihydroisoquinoline-2(1H)-carboxylate (7-7) (510 mg, yield: 100%).
**LCMS:** Rt: 2.123 min; MS m/z (ESI): 727.1 [M+H].

Preparation of compound 7-8:

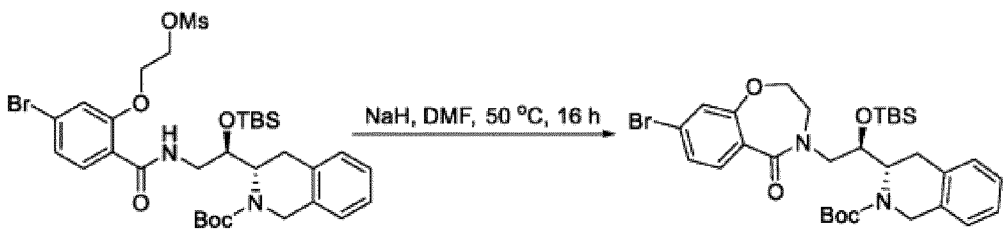


7-7 7-8

At room temperature, tert-butyl (S)-3-((R)-2-(4-bromo-2-(2-((methanesulfonyl)oxy)ethoxy)benzamido)-1-((tert-butyl dimethylsilyl)oxy)ethyl)-3,4-dihydroisoquinoline-2(1H)-carboxylate (7-7) (510 mg, 0.7 mmol) was added to ultradry N,N-dimethylformamide (10.0 mL), and then sodium hydride (36.4 mg, 0.91 mmol) was added. The mixture was reacted at 50°C for 16.0 hours. Upon completion of reaction, water (50 mL) was added, and the mixture was extracted with ethyl acetate (50 mL X 3). The organic phase was washed with saturated brine (50 mL X 3), dried over anhydrous sodium sulfate and filtered. The filtrate was concentrated under reduced pressure. The residue was purified using a normal phase silica gel chromatographic column (eluent gradient: petroleum ether/ethyl acetate = 10/1) to afford the target

intermediate tert-butyl (S)-3-((R)-2-(8-bromo-5-carbonyl-2,3-dihydrobenzo[f][1,4]oxazepin-4(5H)-yl)-1-((tert-butyl dimethylsilyl)oxy)ethyl)-3,4-dihydroisoquinoline-2(1H)-carboxylate (7-8) (341 mg, yield: 77%).
**LCMS:** Rt: 2.405 min; MS m/z (ESI): 631.1 [M+H].

Preparation of compound 7-9:

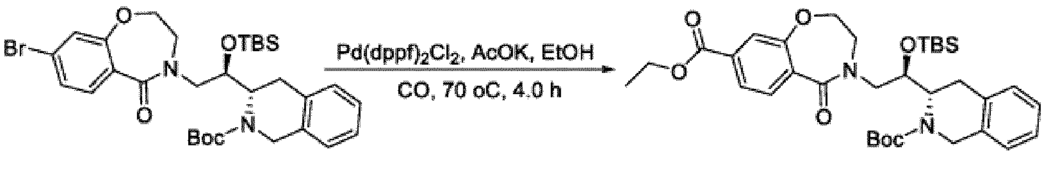


7-8 7-9

At room temperature, tert-butyl (S)-3-((R)-2-(8-bromo-5-carbonyl-2,3-dihydrobenzo[f][1,4]oxazepin-4(5H)-yl)-1-((tert-butyl dimethylsilyl)oxy)ethyl)-3,4-dihydroisoquinoline-2(1H)-carboxylate (7-8) (291 mg, 0.46 mmol), [1,1'-bis(diphenylphosphine)ferrocene]palladium dichloride (17 mg, 0.023 mmol) and potassium acetate (135 mg, 1.38 mmol) were added to anhydrous ethanol (17.0 mL). The mixture was subjected to CO replacement three times, heated to 70°C and reacted for 4.0 hours. Upon completion of reaction, the reaction solution was cooled to room temperature and concentrated under reduced pressure. The residue was purified using a normal phase silica gel chromatographic column (eluent gradient: petroleum ether/ethyl acetate = 5/1) to afford the target intermediate ethyl 4-((R)-2-((S)-2-(tert-butoxycarbonyl)-1,2,3,4-tetrahydroisoquinolin-3-yl)-2-((tert-butyl dimethylsilyl)oxy)ethyl)-5-oxo-2,3,4,5-tetrahydrobenzo[f][1,4]oxazepine-8-carboxylate (7-9) (261 mg, yield: 91%).
**LCMS:** Rt: 1.532 min; MS m/z (ESI): 625.0 [M+H].

Preparation of compound 7-10:

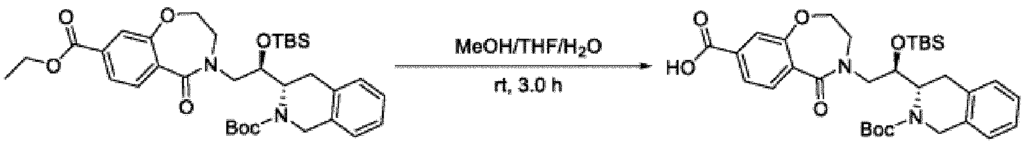


7-9 7-10

At room temperature, ethyl 4-((R)-2-((S)-2-(tert-butoxycarbonyl)-1,2,3,4-tetrahydroisoquinolin-3-yl)-2-((tert-butyl dimethylsilyl)oxy)ethyl)-5-oxo-2,3,4,5-tetrahydrobenzo[f][1,4]oxazepine-8-carboxylate (7-9) (261 mg, 0.42 mmol) was added to a mixed solution of methanol (5.0 mL), tetrahydrofuran (5.0 mL) and water (5.0 mL).Then lithium hydroxide monohydrate (140.0 mg, 3.34 mmol) was added, and the mixture was reacted at room temperature for 3.0 hours. Upon completion of reaction, the reaction system was cooled to 0°C and the reaction solution was adjusted with 1 N hydrochloric acid aqueous solution to the pH of 5.0 and then extracted with ethyl acetate (30 mL X 2). The organic phases were combined, washed with saturated brine, dried over anhydrous sodium sulfate, and filtered. The filtrate was concentrated under reduced pressure to afford the target intermediate 4-((R)-2-((S)-2-(tert-butoxycarbonyl)-1,2,3,4-tetrahydroisoquinolin-3-yl)-2-((tert-butyl dimethylsilyl)oxy)ethyl)-5-oxo-2,3,4,5-tetrahydrobenzo[f][1,4]oxazepine-8-carboxylic acid (7-10) (241 mg, yield: 97%).
**LCMS:** Rt: 0.788 min; MS m/z (ESI): 597.2 [M+H].

Preparation of compound 7-11:

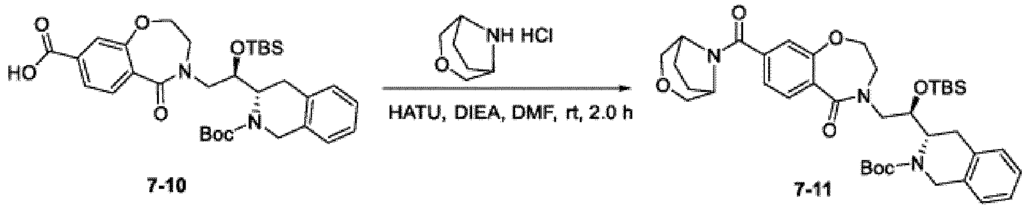


At room temperature, 4-((R)-2-((S)-2-(tert-butoxycarbonyl)-1,2,3,4-tetrahydroisoquinolin-3-yl)-2-((tert-butyl dimethylsilyl)oxy)ethyl)-5-oxo-2,3,4,5-tetrahydrobenzo[f][1,4]oxazepine-8-carboxylic acid (7-10) (20 mg, 0.033 mmol), 2-(7-azabenzotriazole)-N,N,N',N'-tetramethylurea hexafluorophosphate (25.5 mg, 0.067 mmol) and N,N-diisopropylethylamine (17.0 mg, 0.134 mmol) were added to ultradry N,N-dimethylformamide (1.0 mL). Then 3-oxa-8-azabicyclo[3.2.1]octane hydrochloride (6.5 mg, 0.044 mmol) was added, and the mixture was reacted at room temperature for 2.0 hours. Upon completion of reaction, saturated brine (30 mL) was added, and the resulting mixture was extracted with ethyl acetate (30 mL X 2). The organic phase was washed with saturated brine (30 mL X 2), dried over anhydrous sodium sulfate and filtered. The filtrate was concentrated under reduced pressure. The residue was purified by Prep-TLC (developing agent: petroleum ether/ethyl acetate = 1/1) to afford the target intermediate tert-butyl (3S)-3-((1R)-2-(8-(3-oxa-8-azabicyclo[3.2.1]octane-8-carbonyl)-5-oxo-2,3-dihydrobenzo[f][1,4]oxazepin-4(5H)-yl)-1-((tert-butyl dimethylsilyl)oxy)ethyl)-3,4-dihydroisoquinoline-2(1H)-carboxylate (7-11) (20.1 mg, yield: 87%).
**LCMS:** Rt: 1.813 min; MS m/z (ESI): 692.4 [M+H].

Preparation of compound 007:

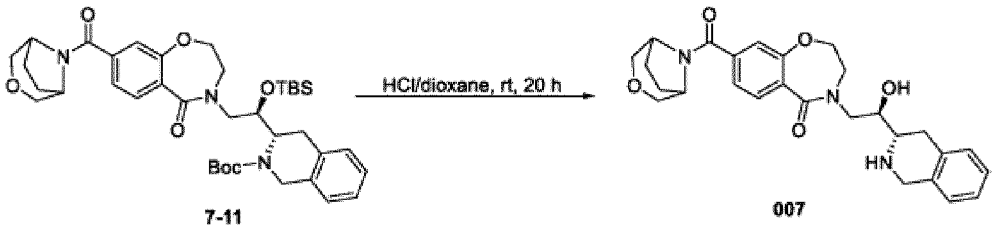


At 0°C, tert-butyl (3S)-3-((1R)-2-(8-(3-oxa-8-azabicyclo[3.2.1]octane-8-carbonyl)-5-oxo-2,3-dihydrobenzo[f][1,4]oxazepin-4(5H)-yl)-1-((tert-butyl dimethylsilyl)oxy)ethyl)-3,4-dihydroisoquinoline-2(1H)-carboxylate (7-11) (20.1 mg) was added to a hydrochloric acid/dioxane solution (4 mol/L, 5.0 mL), and the reaction solution was stirred at room temperature for 20 hours. Upon completion of reaction, the mixture was concentrated under reduced pressure. The residue was purified by high-performance liquid preparative chromatography using the eluent gradient below:

| Instruments | Waters 2767 | | |
|---|---|---|---|
| Mobile phase | A: 0.1% $NH_4HCO_3$ in $H_2O$ B: $CH_3CN$ | | |
| Chromatographic column | Waters sunfire C18 19*250 mm 10 μm | | |
| Injection volume | 500 μL | | |
| Table of running gradients | Time (min) | Mobile phase A (%) | Mobile phase B (%) |
| | 0 | 90 | 5 |
| | 1 | 80 | 20 |
| | 11 | 60 | 40 |
| | 11.2 | 5 | 95 |
| | 13 | 5 | 95 |
| | 13.2 | 95 | 5 |
| | 15 | 95 | 5 |

to afford the target compound 8-(3-oxa-8-azabicyclo[3.2.1]octane-8-carbonyl)-4-((R)-2-hydroxy-2-((S)-1,2,3,4-tetrahydroisoquinolin-3-yl)ethyl)-3,4-dihydrobenzo[f][1,4]oxazepin-5(2H)-one (compound 007) (7.09 mg, yield: 51%).

**$^1$H NMR** (400 MHz, $CD_3OD$): δ 7.79 (d, *J* = 8.0 Hz, 1H), 7.31-7.26 (m, 1H), 7.18-7.08 (m, 4H), 7.08-7.02 (m, 1H), 4.63 (s, 1H), 4.56-4.47 (m, 2H), 4.10-3.94 (m, 5 H), 3.84-3.63 (m, 6 H), 3.62-3.52 (m, 1 H), 2.99-2.82 (m, 3 H), 2.12-1.92 (m, 4 H).
**LCMS:** Rt: 1.525 min; MS m/z (ESI): 478.1 [M+H].

Example 9. Preparation of 4-((R)-2-hydroxy-2-((S)-1,2,3,4-tetrahydroisoquinolin-3-yl)ethyl)-8-(3-methoxy-8-azabicyclo[3.2.1]octane-8-carbonyl)-3,4-dihydrobenzo[f][1,4]oxazepin-5(2H)-one (compound 008)

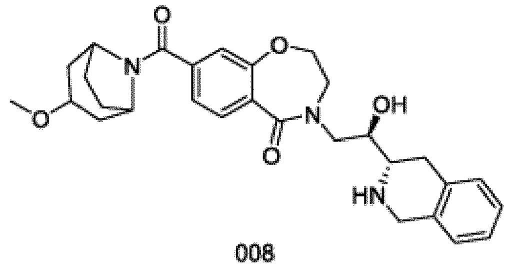

008

Reference can be made to the synthetic method in example 8 and 3-methoxy-8-azabicyclo[3.2.1]octane hydrochloride was used in place of 3-oxa-8-azabicyclo[3.2.1]octane hydrochloride to afford the target product 4-((R)-2-hydroxy-2-((S)-1,2,3,4-tetrahydroisoquinolin-3-yl)ethyl)-8-(3-methoxy-8-azabicyclo[3.2.1]octane-8-carbonyl)-3,4-dihydrobenzo[f][1,4]oxazepin-5(2H)-one (compound 008).

**$^1$H NMR** (400 MHz, $CD_3OD$): δ 7.78 (d, *J* = 8.0 Hz, 1H), 7.24 (dd, *J* = 8.0 Hz, 1.6 Hz, 1H), 7.13-7.03 (m, 5 H), 4.66 (s, 1 H), 4.53-4.50 (m, 2 H), 4.07-3.94 (m, 5 H), 3.76-3.66 (m, 3 H), 3.55 (s, 1 H), 3.29 (s, 3 H), 2.95-2.85 (m, 3 H), 2.19-2.07 (m, 4 H), 1.95-1.86 (m, 4 H).
**LCMS:** Rt: 1.305 min; MS m/z (ESI): 506.3 [M+H].

Example 10. Preparation of 4-((R)-2-hydroxy-2-((S)-1,2,3,4-tetrahydroisoquinolin-3-yl)ethyl)-8-(2-methoxy-7-azaspiro[3.5]nonane-7-carbonyl)-3,4-dihydrobenzo[f][1,4]oxazepin-5(2H)-one (compound 009)

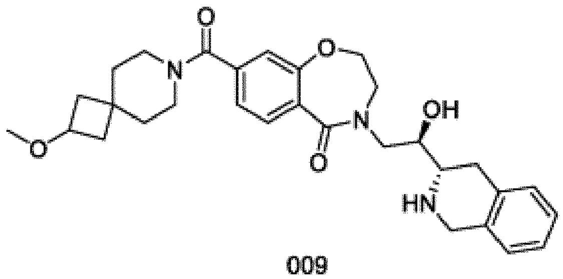

009

Reference can be made to the synthetic method in example 8 and 2-methoxy-7-azaspiro[3.5]nonane hydrochloride was used in place of 3-oxa-8-azabicyclo[3.2.1]octane hydrochloride to afford the target compound 4-((R)-2-hydroxy-2-((S)-1,2,3,4-tetrahydroisoquinolin-3-yl)ethyl)-8-(2-methoxy-7-azaspiro[3.5]nonane-7-carbonyl)-3,4-dihydrobenzo[f][1,4]oxazepin-5(2H)-one (compound 009).

**$^1$H NMR** (400 MHz, $CD_3OD$): δ 7.77 (d, *J* = 7.9 Hz, 1 H), 7.16 (d, *J* = 7.9 Hz, 1 H), 7.15-7.07 (m, 3 H), 7.07-6.99 (m, 2 H), 4.57-4.45 (m, 2 H), 4.10-3.85 (m, 5 H), 3.80-3.54 (m, 5 H), 3.36-3.30 (m, 2 H), 3.21 (s, 3 H), 3.00-2.82 (m, 3 H), 2.31-2.18 (m, 2 H), 1.79-1.60 (m, 4 H), 1.54 (s, 2H).
**LCMS:** Rt: 1.306 min; MS m/z (ESI): 520.3 [M+H].

Example 11. Preparation of 1-ethyl-4-((R)-2-hydroxy-2-((S)-1,2,3,4-tetrahydroisoquinolin-3-yl)ethyl)-8-(8-oxa-2-azaspiro[4.5]decane-2-carbonyl)-1,2,3,4-tetrahydro-5H-benzo[e][1,4]diazepin-5-one hydrochloride (compound 010)

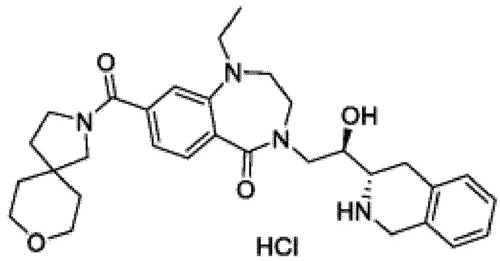

HCl

010

Reference can be made to the synthetic method in example 2 and 8-oxa-2-azaspiro[4.5]decane was used in place of 3-oxa-8-azabicyclo[3.2.1]octane hydrochloride to afford the target product 1-ethyl-4-((R)-2-hydroxy-2-((S)-1,2,3,4-tetrahydroisoquinolin-3-yl)ethyl)-8-(8-oxa-2-azaspiro[4.5]decane-2-carbonyl)-1,2,3,4-tetrahydro-5H-benzo[e][1,4]diazepin-5-one hydrochloride (compound 010).
**$^1$H NMR** (400 MHz, $CD_3OD$): $\delta$ 7.71 (dd, *J* = 7.9, 2.0 Hz, 1H), 7.42 - 7.19 (m, 6H), 4.52 - 4.31 (m, 3H), 4.07 - 3.97 (m, 1H), 3.80 - 3.62 (m, 9H), 3.62 - 3.49 (m, 4H), 3.49 - 3.33 (m, 3H), 3.29 - 3.20 (m, 2H), 1.96 (t, *J* =7.3 Hz, 1H), 1.89 (t, *J* = 6.9 Hz, 1H), 1.72 - 1.63 (m, 2H), 1.59 - 1.49 (m, 2H), 1.20 (t, *J* = 7.0 Hz, 3H).
**LCMS:** Rt: 1.230 min; MS m/z (ESI): 533.2 [M+H].

Example 12. Preparation of 1-ethyl-4-((R)-2-hydroxy-2-((S)-1,2,3,4-tetrahydroisoquinolin-3-yl)ethyl)-8-(7-methoxy-2-azaspiro[3.5]nonane-2-carbonyl)-1,2,3,4-tetrahydro-5H-benzo[e][1,4]diazepin-5-one hydrochloride (compound 011)

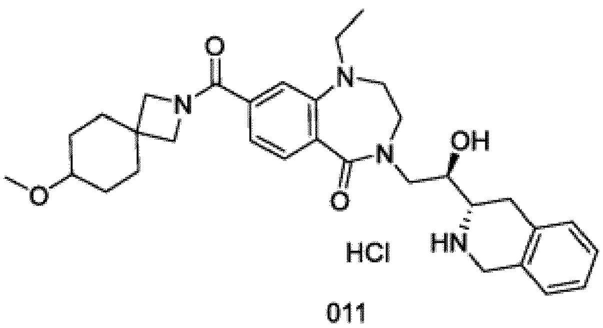

HCl

011

Preparation of compound 011-2:

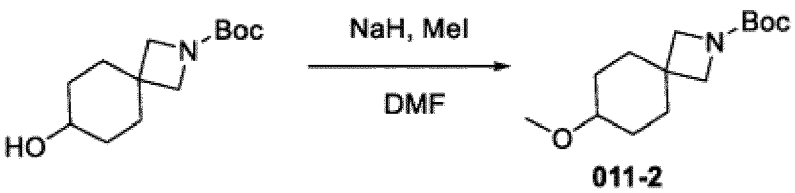


011-2

At room temperature, tert-butyl 7-hydroxy-2-azaspiro[3.5]nonane-2-carboxylate (50 mg, 0.207 mmol) was added to ultradry N,N-dimethylformamide (1.5 mL), and then sodium hydride (20 mg, 0.498 mmol) was added under ice bath. The mixture was reacted at room temperature for 0.5 hours. Then iodomethane (59 mg, 0.414 mmol) was added, and the resulting mixture was reacted at room temperature for 1.5 hours. Upon completion of reaction, the reaction was

quenched with saturated aqueous ammonium chloride solution and extracted with ethyl acetate (30 mL X 2). The organic phases were combined, washed with saturated brine (50 mL X 3), dried over anhydrous sodium sulfate and filtered. The filtrate was concentrated under reduced pressure to afford the target intermediate tert-butyl 7-methoxy-2-azaspiro[3.5]nonane-2-carboxylate (011-2) (51 mg). **LCMS:** Rt: 1.800 min; MS m/z (ESI): 256.0 [M+H].

Preparation of compound 011-3:

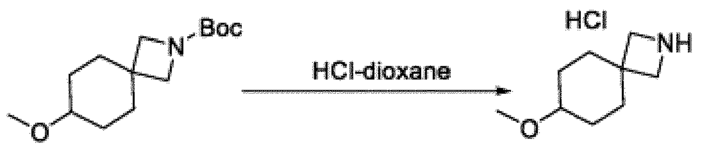


011-2 011-3

At room temperature, tert-butyl 7-methoxy-2-azaspiro[3.5]nonane-2-carboxylate (011-2) (51 mg, 0.2 mmol) was added to a hydrochloric acid-dioxane solution (4 N, 3.0 mL) and the mixture was reacted at room temperature for 1 hour. Upon completion of reaction, the reaction solution was concentrated under reduced pressure to afford the intermediate 7-methoxy-2-azaspiro[3.5]nonane hydrochloride (011-3) (33.7 mg). **LCMS:** Rt: 0.391 min; MS m/z (ESI): 156.3 [M+H].

Reference can be made to the synthetic method in example 2 for the other steps and 7-methoxy-2-azaspiro[3.5]nonane hydrochloride (011-3) was used in place of 3-oxa-8-azabicyclo[3.2.1]octane hydrochloride to afford the target product 1-ethyl-4-((R)-2-hydroxy-2-((S)-1,2,3,4-tetrahydroisoquinolin-3-yl)ethyl)-8-(7-methoxy-2-azaspiro[3.5]nonane-2-carbonyl)-1,2,3,4-tetrahydro-5H-benzo[e][1,4]diazepin-5-one hydrochloride (compound 011).

$^1$**H NMR** (400 MHz, $CD_3OD$): δ 7.70 (d, $J$ = 8.0 Hz, 1H), 7.51 - 7.46 (m, 2H), 7.31 - 7.22 (m, 4H), 4.49 - 4.33 (m, 3H), 4.05 - 3.88 (m, 3H), 3.85 (s, 2H), 3.72 - 3.58 (m, 6H), 3.47 - 3.40 (m, 2H), 3.35 - 3.31 (m, 3H), 3.30 - 3.21 (m, 3H), 1.94 - 1.38 (m, 4H), 1.62 - 1.56 (m, 2H), 1.44 - 1.38 (m, 2H), 1.20 (t, $J$ = 7.2 Hz, 3H).
**LCMS:** Rt: 1.302 min; MS m/z (ESI): 547.2 [M+H].

Example 13. Preparation of 8-(4,4-difluoro-6-azaspiro[2.5]octane-6-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 012)

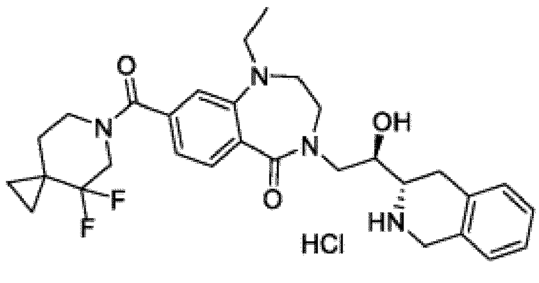

012

Reference can be made to the synthetic method in example 2 and 4,4-difluoro-6-azaspiro[2.5]octane hydrochloride was used in place of 3-oxa-8-azabicyclo[3.2.1]octane hydrochloride to afford the target product 8-(4,4-difluoro-6-azaspiro[2.5]octane-6-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 012).
$^1$**H NMR** (400 MHz, $CD_3OD$): δ7.74 (d, $J$ = 7.6 Hz, 1H), 7.31 - 7.22 (m, 6H), 4.50 - 4.35 (m, 3H), 4.04 - 4.00 (m, 2H), 3.87 (s, 1H), 3.74 - 3.63 (m, 7H), 3.55 - 3.35 (m, 3H), 3.28 - 3.22 (m, 2H), 1.73 - 1.66 (m, 2H), 1.21 (t, $J$ = 6.8 Hz, 3H), 0.91 (s, 2H), 0.57 (s, 2H). **LCMS:** Rt: 1.361 min; MS m/z (ESI): 539.3 [M+H].

Example 14. Preparation of 8-(1,1-difluoro-6-azaspiro[2.5]octane-6-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 013)

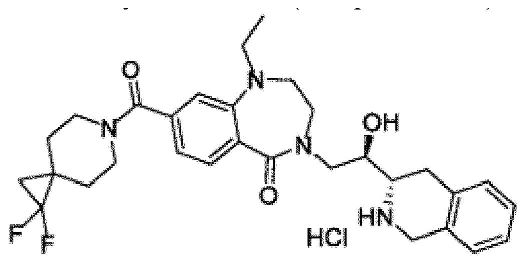

013

Reference can be made to the synthetic method in example 2 and 1,1-difluoro-6-azaspiro[2.5]octane hydrochloride was used in place of 3-oxa-8-azabicyclo[3.2.1]octane hydrochloride to afford the target product 8-(1,1-difluoro-6-azaspiro[2.5]octane-6-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 013).

**$^1$H NMR** (400 MHz, $CD_3OD$): $\delta$7.70 (d, *J* = 8.0 Hz, 1H), 7.31 - 7.22 (m, 6H), 4.49 - 4.33 (m, 3H), 4.01 (dd, *J* =4.4 Hz, 14.0 Hz, 1H), 3.80 (s, 2H), 3.73 - 3.58 (m, 6H), 3.48 - 3.35 (m, 4H), 3.27 - 3.21 (m, 2H), 1.78 - 1.611 (m, 4H), 1.26 - 1.25 (m, 2H), 1.20 (t, *J* = 6.8 Hz, 3H).
**LCMS:** Rt: 1.330 min; MS m/z (ESI): 539.3 [M+H].

Example 15. Preparation of 1-ethyl-4-((R)-2-hydroxy-2-((S)-1,2,3,4-tetrahydroisoquinolin-3-yl)ethyl)-8-(morpholine-4-carbonyl)-1,2,3,4-tetrahydro-5H-benzo[e][1,4]diazepin-5-one hydrochloride (compound 014)

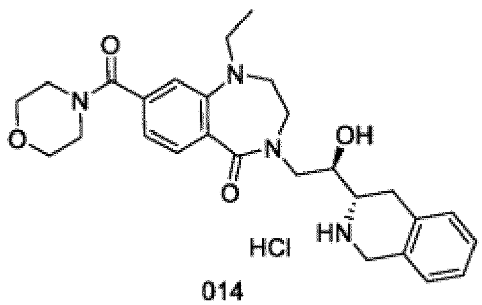

014

Reference can be made to the synthetic method in example 2 and morpholine was used in place of 3-oxa-8-azabicyclo[3.2.1]octane hydrochloride to afford the target product 1-ethyl-4-((R)-2-hydroxy-2-((S)-1,2,3,4-tetrahydroisoquinolin-3-yl)ethyl)-8-(morpholine-4-carbonyl)-1,2,3,4-tetrahydro-5H-benzo[e][1,4]diazepin-5-one hydrochloride (compound 014).

**$^1$H NMR** (400 MHz, $CD_3OD$): $\delta$ 7.62 (d, *J* = 7.7 Hz, 1H), 7.32 - 7.20 (m, 4H), 7.12 - 7.06 (m, 2H), 4.50 - 4.37 (m, 2H), 4.34 - 4.26 (m, 1H), 3.98 (dd, *J* = 14.2, 4.5 Hz, 1H), 3.84 - 3.53 (m, 12H), 3.50 - 3.39 (m, 3H), 3.30 - 3.20 (m, 3H), 1.16 (t,*J* = 7.0 Hz, 3H). **LCMS:** Rt: 1.336 min; MS m/z (ESI): 479.1 [M+H].

Example 16. Preparation of 8-(4-acetylpiperazine-1-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 015)

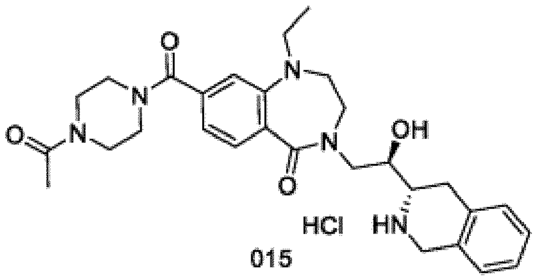

015

Reference can be made to the synthetic method in example 2 and 1-acetylpiperazine was used in place of 3-oxa-8-azabicyclo[3.2.1]octane hydrochloride to afford the target product 8-(4-acetylpiperazine-1-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 015).

**$^{1}$H NMR** (400 MHz, $CD_3OD$): δ 7.61 (d, *J* = 8.0 Hz, 1H), 7.31 - 7.20 (m, 4H), 7.10 - 7.03 (m, 2H), 4.50 - 4.36 (m, 2H), 4.33 - 4.22 (m, 1H), 3.97 (dd, *J* = 14.2, 4.4 Hz, 1H), 3.86 - 3.35 (m, 15H), 3.28 - 3.11 (m, 3H), 2.21 - 2.04 (m, 3H), 1.15 (t, J = 6.8 Hz, 3H).
**LCMS:** Rt: 1.574 min; MS m/z (ESI): 520.3 [M+H].

Example 17. Preparation of N-benzyl-1-ethyl-4-((R)-2-hydroxy-2-((S)-1,2,3,4-tetrahydroisoquinolin-3-yl)ethyl)-N-methyl-5-oxo-2,3,4,5-tetrahydro-1H-benzo[e][1,4]diaza-8-carboxamide hydrochloride (compound 016)

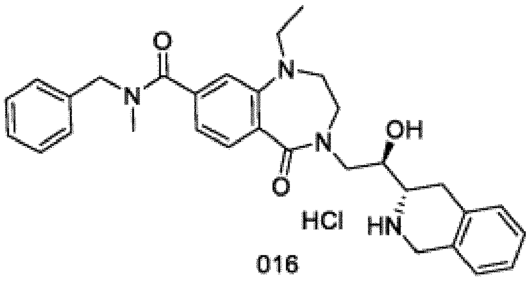

016

Reference can be made to the synthetic method in example 2 and N-methyl-1-phenylmethanamine was used in place of 3-oxa-8-azabicyclo[3.2.1]octane hydrochloride to afford the target product N-benzyl-1-ethyl-4-((R)-2-hydroxy-2-((S)-1,2,3,4-tetrahydroisoquinolin-3-yl)ethyl)-N-methyl-5-oxo-2,3,4,5-tetrahydro-1H-benzo[e][1,4]diaza-8-carboxamide hydrochloride (compound 016).
**$^{1}$H NMR** (400 MHz, $CD_3OD$): δ 7.67 - 7.57 (m, 1H), 7.42 - 7.17 (m, 9H), 7.16 - 6.99 (m, 2H), 4.76 (s, 1H), 4.55 (s, 1H), 4.47 - 4.36 (m, 2H), 4.33 - 4.24 (m, 1H), 4.04 - 3.87 (m, 1H), 3.76 - 3.34 (m, 7H), 3.27 - 3.16 (m, 2H), 3.11 - 2.86 (m, 4H), 1.23 - 0.90 (m, 3H). **LCMS:** Rt: 1.460 min; MS m/z (ESI): 513.1 [M+H].

Example 18. Preparation of 1-ethyl-N-(4-fluorophenethyl)-4-((R)-2-hydroxy-2-((S)-1,2,3,4-tetrahydroisoquinolin-3-yl)ethyl)-N-methyl-5-oxo-2,3,4,5-tetrahydro-1H-benzo[e][1,4]diazepine-8-carboxamide hydrochloride (compound 017)

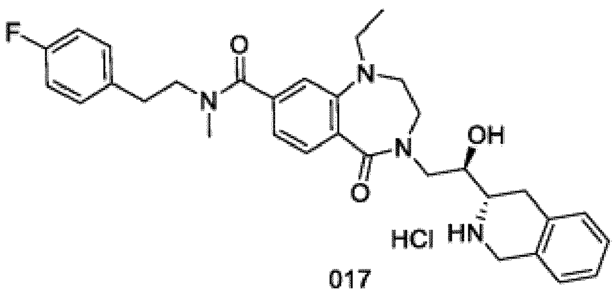

017

Reference can be made to the synthetic method in example 2 and 2-(4-fluorophenyl)-N-methyethylamine was used in place of 3-oxa-8-azabicyclo[3.2.1]octane hydrochloride to afford the target product 1-ethyl-N-(4-fluorophenethyl)-4-((R)-2-hydroxy-2-((S)-1,2,3,4-tetrahydroisoquinolin-3-yl)ethyl)-N-methyl-5-oxo-2,3,4,5-tetrahydro-1H-benzo[e][1,4]diazepine-8-carboxamide hydrochloride (compound 017).

**$^1$H NMR** (400 MHz, $CD_3OD$): δ 7.56 (dd, *J* = 35.7, 7.8 Hz, 1H), 7.38 - 7.16 (m, 5H), 7.09 - 6.84 (m, 4H), 6.77 - 6.67 (m, 1H), 4.52 - 4.38 (m, 2H), 4.35 - 4.26 (m, 4.5 Hz, 1H), 3.98 (dd, *J* = 14.2, 4.4 Hz, 1H), 3.87 - 3.50 (m, 7H), 3.49 - 3.38 (m, 1H), 3.30 - 3.09 (m, 6H), 3.06 - 2.95 (m, 1H), 2.89 - 2.79 (m, 2H), 1.20 - 1.06 (m, 3H).
**LCMS:** Rt: 1.274 min; MS m/z (ESI): 545.3 [M+H].

Example 19. Preparation of 1-ethyl-4-((R)-2-hydroxy-2-((S)-1,2,3,4-tetrahydroisoquinolin-3-yl)ethyl)-N-methyl-5-oxo-N-(2-(pyridin-3-yl)ethyl)-2,3,4,5-tetrahydro-1H-benzo[e][1,4]diazepine-8-carboxamide hydrochloride (compound 018)

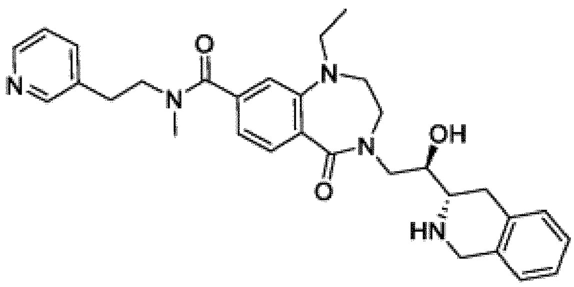

Reference can be made to the synthetic method in example 2 and N-methyl-2-(pyridin-3-yl)ethan-1-amine was used in place of 3-oxa-8-azabicyclo[3.2.1]octane hydrochloride to afford the target product 1-ethyl-4-((R)-2-hydroxy-2-((S)-1,2,3,4-tetrahydroisoquinolin-3-yl)ethyl)-N-methyl-5-oxo-N-(2-(pyridin-3-yl)ethyl)-2,3,4,5-tetrahydro-1H-benzo[e][1,4]diazepine-8-carboxamide hydrochloride (compound 018).

**$^1$H NMR** (400 MHz, $CD_3OD$): δ 9.07 - 8.56 (m, 3H), 8.19 - 7.96 (m, 1H), 7.77 - 7.52 (m, 1H), 7.39 - 7.18 (m, 4H), 7.17 - 6.52 (m, 2H), 4.53 - 4.28 (m, 3H), 4.13 - 3.82 (m, 3H), 3.79 - 3.42 (m, 6H), 3.41 - 3.31 (m, 3H), 3.29 - 2.93 (m, 6H), 1.17 (s, 3H).
**LCMS:** Rt: 1.654 min; MS m/z (ESI): 528.3 [M+H].

Example 20. Preparation of N,N,1-triethyl-4-((R)-2-hydroxy-2-((S)-1,2,3,4-tetrahydroisoquinolin-3-yl)ethyl)-5-oxo-2,3,4,5-tetrahydro-1H-benzo[e][1,4]diazepine-8-carboxamide hydrochloride (compound 019)

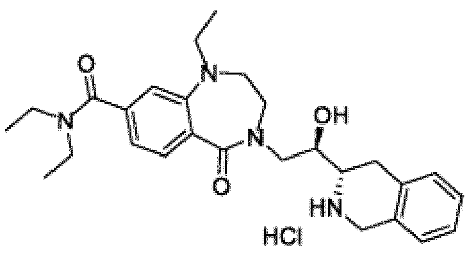
HCl

019

Reference can be made to the synthetic method in example 2 and diethylamine was used in place of 3-oxa-8-azabicyclo[3.2.1]octane hydrochloride to afford the target product N,N,1-triethyl-4-((R)-2-hydroxy-2-((S)-1,2,3,4-tetrahydroisoquinolin-3-yl)ethyl)-5-oxo-2,3,4,5-tetrahydro-1H-benzo[e][1,4]diazepine-8-carboxamide hydrochloride (compound 019).

**$^1$H NMR** (400 MHz, $CD_3OD$): δ 7.69 (d, *J* = 7.7 Hz, 1H), 7.34 - 7.21 (m, 4H), 7.20 - 7.12 (m, 2H), 4.51 - 4.38 (m,

2H), 4.38 - 4.31 (m, 1H), 4.06 - 3.96 (m, 1H), 3.75 - 3.61 (m, 5H), 3.60 - 3.49 (m, 3H), 3.47 - 3.33 (m, 3H), 3.29 - 3.20 (m, 3H), 1.26 (t, $J$ = 7.1 Hz, 3H), 1.22-1.10 (m, 6H).
**LCMS:** Rt: 1.274 min; MS m/z (ESI): 465.3 [M+H].

Example 21. Preparation of 8-(3-oxa-9-azabicyclo[3,3,1]nonane-9-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 020)

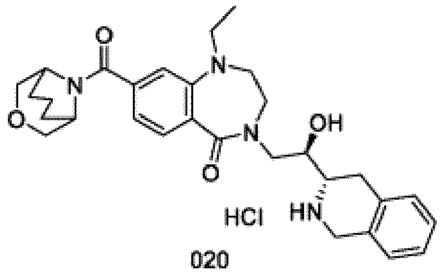

HCl

020

Reference can be made to the synthetic method in example 2 and 3-oxa-9-azabicyclo[3.3.1]nonane hydrochloride was used in place of 3-oxa-8-azabicyclo[3.2.1]octane hydrochloride to afford the target product 8-(3-oxa-9-azabicyclo[3.3.1]nonane-9-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 020).

**$^1$H NMR** (400 MHz, $CD_3OD$): $\delta$ 7.63 (d, $J$ = 8.0 Hz, 1H), 7.28-7.21 (m, 4H), 7.11 (t, $J$ = 6.4 Hz, 2H), 4.50 (s, 1H), 4.42-4.31 (m, 2H), 4.30-4.29 (m, 1H), 4.03-3.95 (m, 2H), 3.87-3.85 (m, 2H), 3.78-3.66 (m, 7H), 3.58 (s, 1H), 3.33-3.30 (m, 2H), 3.29-3.25 (m, 2H), 2.69-2.55 (m, 1H), 1.99-1.83 (m, 4H) 1.66-1.64 (m, 1H), 1.15 (t, $J$ = 7.0 Hz, 3H).
**LCMS:** Rt: 1.382 min; MS m/z (ESI): 519.5 [M+H].

Example 22. Preparation of 8-(2-(dimethylamino)-7-azaspiro[3.5]nonane-7-carbonyl)-1-ethyl-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 021)

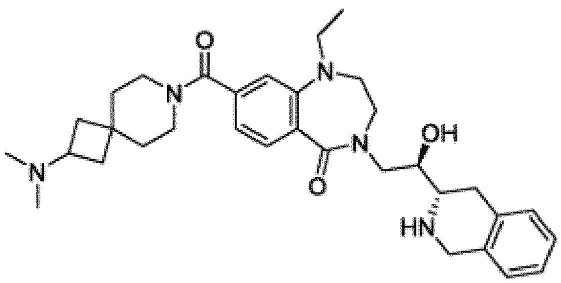

Preparation of compound 021-2:

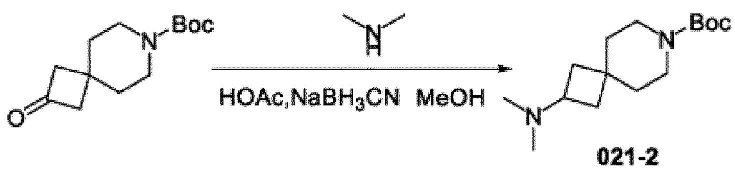


021-2

At room temperature, tert-butyl 2-oxo-7-azaspiro[3.5]nonane-7-carboxylate (1 g, 4.2 mmol) was added to methanol (10 mL) and then dissolved. Then acetic acid (1.5 mL, 20.9 mmol) and dimethylamine (10.5 mL, 20.9 mmol) were

added, and the resulting mixture was reacted at room temperature for 0.5 hours. Subsequently, sodium cyanoborohydride (1.3 g, 20.9 mmol) was added. Upon completion of reaction, the reaction solution was concentrated under reduced pressure to remove methanol, ethyl acetate (50 mL) was added for dissolution, and the mixture was washed with a sodium chloride solution (10 mL X 2), dried over anhydrous sodium sulfate and filtered. The filtrate was concentrated under reduced pressure to afford tert-butyl 2-(dimethylamino)-7-azaspiro[3.5]nonane-7-carboxylate (021-2) (800 mg).
**LCMS:** Rt: 1.054 min; MS m/z (ESI): 269.1 [M+H].

Preparation of compound 021-3 :

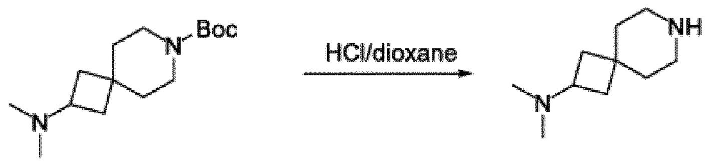


021-2 021-3

At room temperature, tert-butyl 2-(dimethylamino)-7-azaspiro[3.5]nonane-7-carboxylate (021-2) (800 mg, 2.9 mmol) was added to a hydrogen chloride-dioxane (2.5 mL, 4 mol/L) solution. The mixture was stirred and reacted at room temperature for 1.0 hour. Upon completion of reaction, the reaction solution was concentrated under reduced pressure and then slurried with ethyl acetate (3 mL). The product was collected to afford a hydrochloride (700 mg) of N,N-dimethyl-7-azaspiro[3.5]nonane-2-amine (021-3).
**LCMS:** Rt: 0.263 min; MS m/z (ESI): 169.1 [M+H].
Reference can be made to the synthesis in example 2 for the other steps and the hydrochloride of N,N-dimethyl-7-azaspiro[3.5]nonane-2-amine (021-3) was used in place of 3-oxa-8-azabicyclo[3.2.1]octane hydrochloride to afford the target product 8-(2-(dimethylamino)-7-azaspiro[3.5]nonane-7-carbonyl)-1-ethyl-4-((R)-2-hydroxy-2-((S)-1,2,3,4-tetrahydroisoquinolin-3 -yl)ethyl)-1,2,3,4-tetrahydro-5H-benzo[e][1,4] diazepin-5-one hydrochloride (compound 021).

**$^1$H NMR** (400 MHz, $CD_3OD$): δ 7.60 (d, *J* = 8.0 Hz, 1H), 7.33 - 7.19 (m, 4H), 7.07 - 6.99 (m, 2H), 4.49 - 4.36 (m, 2H), 4.34 - 4.27 (m, 1H), 3.98 (dd, *J* = 14.2, 4.6 Hz, 1H), 3.80 - 3.60 (m, 6H), 3.60 - 3.52 (m, 3H), 3.48 -3.37 (m, 2H), 3.29 - 3.20 (m, 4H), 2.78 (s, 6H), 2.38 (s, 2H), 2.06 (t, *J* = 10.3 Hz, 2H), 1.80 - 1.56 (m, 4H), 1.14 (t, *J* = 7.0 Hz, 3H).
**LCMS:** Rt: 0.517 min; MS m/z (ESI): 560.6 [M+H].

Example 23. Preparation of 1-ethyl-4-((R)-2-hydroxy-2-((S)-1,2,3,4-tetrahydroisoquinolin-3-yl)ethyl)-8-(3-hydroxy-8-azabicyclo[3.2.1]octane-8-carbonyl)-1,2,3,4-tetrahydro-5H-benzo[e][1,4]diazepin-5-one hydrochloride (compound 022)

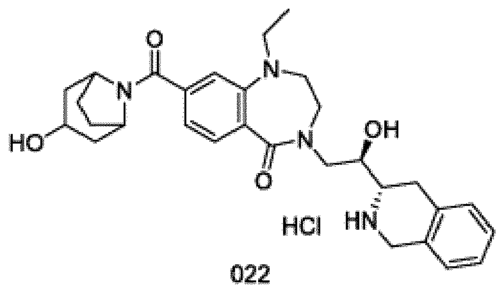

022

Reference can be made to the synthetic method in example 2 and 8-azabicyclo[3.2.1]octane-3-ol was used in place of 3-oxa-8-azabicyclo[3.2.1]octane hydrochloride to afford the target compound 1-ethyl-4-((R)-2-hydroxy-2-((S)-1,2,3,4-tetrahydroisoquinolin-3-yl)ethyl)-8-(3-hydroxy-8-azabicyclo[3.2.1]octane-8-carbonyl)-1,2,3,4-tetrahydro-5H-benzo[e][1,4]diazepin-5-one hydrochloride (compound 022).

**$^1$H NMR** (400 MHz, $CD_3OD$): δ 7.68 (d, *J* = 7.7 Hz, 1H), 7.37 - 7.17 (m, 6H), 4.70 (s, 1H), 4.51 - 4.37 (m, 2H), 4.37 - 4.28 (m, 1H), 4.15 - 3.93 (m, 3H), 3.76 - 3.50 (m, 6H), 3.47 - 3.33 (m, 2H), 3.28 - 3.15 (m, 2H), 2.47 -2.12(m, 2H),

2.08- 1.87 (m, 4H), 1.79 (d, *J* = 14.4 Hz, 1H), 1.39- 1.24 (m, 1H), 1.18 (t, *J* = 7.0 Hz, 3H).
**LCMS:** Rt: 1.310 min; MS m/z (ESI): 519.5 [M+H].

Example 24. Preparation of 1-ethyl-8-(hexahydro-1H-furo[3,4-c]pyrrole-5-carbonyl)-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 023)

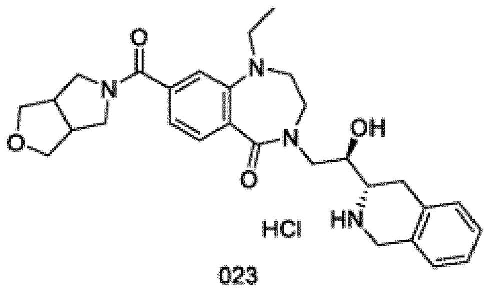

023

Reference can be made to the synthetic method in example 2 and hexahydro-1H-furo[3,4-c]pyrrole was used in place of 3-oxa-8-azabicyclo[3.2.1]octane hydrochloride to afford the target compound 1-ethyl-8-(hexahydro-1H-furo[3,4-c]pyrrole-5-carbonyl)-4-((R)-2-hydroxy-2-((S)-1,2,3,4-tetrahydroisoquinolin-3-yl)ethyl)-1,2,3,4-tetrahydro-5H-benzo[e][1,4]diazepin-5-one hydrochloride (compound 023).

**$^1$H NMR** (400 MHz, $CD_3OD$): δ 7.63 (d, *J* = 8.0 Hz, 1H), 7.30-7.17 (m, 6H), 4.43-4.42 (m, 2H),4.31-4.28 (m, 1H), 4.00-3.97 (m, 1H), 3.95-3.85 (m, 3H), 3.81-3.68 (m, 8H), 3.66-3.60 (m, 3H), 3.37-3.34 (m, 2H), 3.30-3.25 (m, 2H), 3.03-2.99 (m, 2H), 1.16 (t, *J* = 7.0 Hz 3H).
**LCMS:** Rt: 1.316 min; MS m/z (ESI): 505.5 [M+H].

Example 25. Preparation of 1-ethyl-4-((R)-2-hydroxy-2-((S)-1,2,3,4-tetrahydroisoquinolin-3-yl)ethyl)-8-(2-hydroxy-7-azaspiro[3.5]nonane-7-carbonyl)-1,2,3,4-tetrahydro-5H-benzo[e][1,4]diazepin-5-one (compound 024)

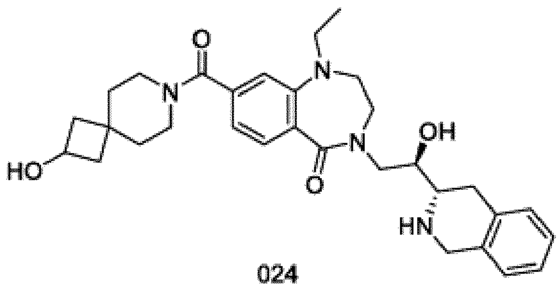

024

Reference can be made to the synthetic method in example 2 and 7-azaspiro[3.5]nonane-2-ol hydrochloride was used in place of 3-oxa-8-azabicyclo[3.2.1]octane hydrochloride to finally prepare a hydrochloride of the target compound 024. The resulting hydrochloride was dissolved with water and then adjusted with saturated sodium bicarbonate to the pH of 8. The aqueous phase was extracted with ethyl acetate 3 times, and the organic phase was washed with saturated sodium chloride solution 3 times and then dried over anhydrous sodium sulfate. After filtration, the filtrate was concentrated and then lyophilized following the addition of pure water to afford the target product 1-ethyl-4-((R)-2-hydroxy-2-((S)-1,2,3,4-tetrahydroisoquinolin-3-yl)ethyl)-8-(2-hydroxy-7-azaspiro[3.5]nonane-7-carbonyl)-1,2,3,4-tetrahydro-5H-benzo[e][1,4]diazepin-5-one (compound 024).

**$^1$H NMR** (400 MHz, $CD_3OD$): δ 7.56 (d, *J* = 7.7 Hz, 1H), 7.15 - 7.06 (m, 3H), 7.06 - 7.00 (m, 1H), 6.99 - 6.92 (m, 2H), 4.31 - 4.16 (m, 1H), 4.09 - 3.85 (m, 4H), 3.78 (dd, *J* = 14.0, 6.4 Hz, 1H), 3.71 - 3.56 (m, 4H), 3.55 -3.46 (m, 1H), 3.40 - 3.31 (m, 2H), 3.28 - 3.11 (m, 3H), 3.04 - 2.91 (m, 2H), 2.91 - 2.80 (m, 1H), 2.36 - 2.21 (m, 2H), 1.78 - 1.59 (m, 4H), 1.53 (s, 2H), 1.11 (t, *J* = 7.1 Hz, 3H).

**LCMS:** Rt: 1.016 min; MS m/z (ESI): 533.2 [M+H].

Example 26. Preparation of 1-ethyl-4-((R)-2-hydroxy-2-((S)-1,2,3,4-tetrahydroisoquinolin-3-yl)ethyl)-8-(2-oxa-6-azaspiro[3.3]heptane-6-carbonyl)-1,2,3,4-tetrahydro-5H-benzo[e][1,4]diazepin-5-one (compound 025)

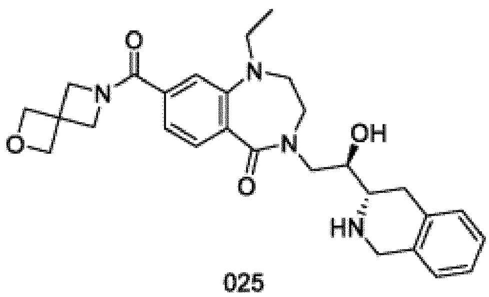

025

Reference can be made to the synthetic method in example 2 and 2-oxa-6-azaspiro[3.3]heptane was used in place of 3-oxa-8-azabicyclo[3.2.1]octane hydrochloride, and the final compound obtained was purified by preparative chromatography (Waters sunfire C18 column, 19*250 mm 10 μm; using a mixture of water (containing 0.1% aqueous ammonia) and acetonitrile with a decreasing polarity as the eluent) to afford the target product 1-ethyl-4-((R)-2-hydroxy-2-((S)-1,2,3,4-tetrahydroisoquinolin-3-yl)ethyl)-8-(2-oxa-6-azaspiro[3.3]heptane-6-carbonyl)-1,2,3,4-tetrahydro-5H-benzo[e][1,4]diazepin-5-one (compound 025).

**$^1$H NMR** (400 MHz, $CD_3OD$): δ 7.56 (d, *J* = 7.8 Hz, 1H), 7.25 - 7.18 (m, 2H), 7.19 - 7.02 (m, 4H), 4.83 - 4.75 (m, 4H), 4.51 (s, 2H), 4.32 (s, 2H), 4.14 - 3.95 (m, 3H), 3.90 (dd, *J* = 14.1, 3.8 Hz, 1H), 3.77 (dd, *J* = 14.1, 6.5 Hz, 1H), 3.65 - 3.56 (m, 2H), 3.55 - 3.44 (m, 1H), 3.40 - 3.32 (m, 1H), 3.28 - 3.15 (m, 2H), 3.15 - 2.86 (m, 3H), 1.13 (t, *J* = 7.0 Hz, 3H).
**LCMS:** Rt: 1.346 min; MS m/z (ESI): 491.4 [M+H].

Example 27. Preparation of 1-ethyl-4-((R)-2-hydroxy-2-((S)-1,2,3,4-tetrahydroisoquinolin-3-yl)ethyl)-8-(2-oxo-7-azaspiro[3. 5]nonane-7-carbonyl)-1,2,3,4-tetrahydro-5H-benzo[e][1,4]diazepin-5-one (compound 026)

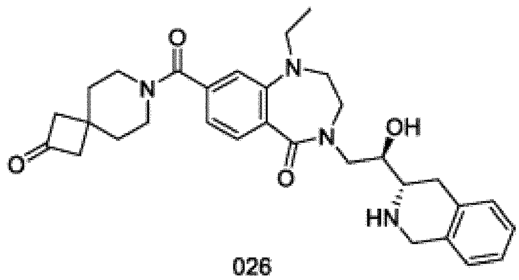

026

Reference can be made to the synthetic method in example 2 and 7-azaspiro[3.5]nonane-2-one hydrochloride was used in place of 3-oxa-8-azabicyclo[3.2.1]octane hydrochloride. The final compound was prepared and purified through a chromatographic column (referring to example 1 for the eluents and gradient). The resulting eluate was adjusted with saturated sodium bicarbonate to the pH of 8-9 and extracted with ethyl acetate 3 times. The organic phase was washed with saturated sodium chloride solution 3 times and then dried over anhydrous sodium sulfate. After filtration, the filtrate was concentrated and then lyophilized following the addition of pure water to afford the target product 1-ethyl-4-((R)-2-hydroxy-2-((S)-1,2,3,4-tetrahydroisoquinolin-3-yl)ethyl)-8-(2-oxo-7-azaspiro[3.5]nonane-7-carbonyl)-1,2,3,4-tetrahydro-5H-benzo[e][1,4]diazepin-5-one (compound 026).

**$^1$H NMR** (400 MHz, $CD_3OD$): δ 7.57 (d, *J* =7.6 Hz, 1H), 7.13-6.99 (m, 6H), 4.07-3.87 (m, 4H), 3.81-3.61 (m, 5H), 3.55-3.33 (m, 4H), 3.27-3.16 (m, 3H), 3.03-2.93 (m, 2H), 2.90-2.83 (m, 4H), 1.857 (s, 2H), 1.734 (s, 2H), 1.12(t, *J* =7.1 Hz, 3H).

**LCMS:** Rt: 1.067 min; MS m/z (ESI): 531.2 [M+H].

Example 28. Preparation of 1-ethyl-4-((R)-2-hydroxy-2-((S)-1,2,3,4-tetrahydroisoquinolin-3-yl)ethyl)-8-(2-(methoxy-*d*$_3$)-7-azaspiro[3.5]nonane-7-carbonyl)-1,2,3,4-tetrahydro-5H-benzo[e][1,4]diazepin-5-one hydrochloride (compound 027)

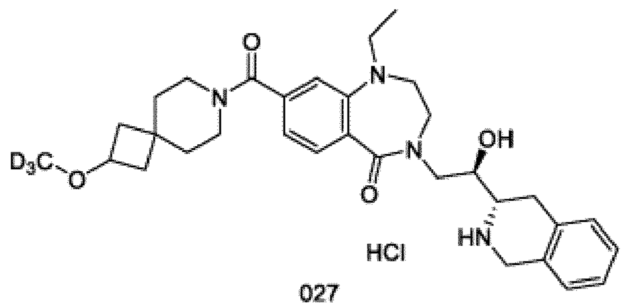

027

Preparation of compound 027-2:

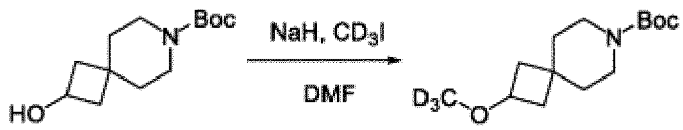


027-2

At room temperature, tert-butyl 2-hydroxy-7-azaspiro[3.5]nonane-7-carboxylate (1.5 g) was dissolved in N,N-dimethylformamide (15 mL). At 0°C, sodium hydride (300 mg) was added. The mixture was reacted at 0°C for 0.5 hours and then deuterated iodomethane (1 g) was added. The reaction solution was reacted at room temperature for 16 hours. The reaction was complete as detected by TLC and then quenched with aqueous ammonia (5 mL). The resulting mixture was diluted by adding water (15 mL), and the aqueous phase was extracted with ethyl acetate (20 mL) twice. The organic phase of the extraction solution was washed with saturated sodium chloride aqueous solution (20 mL) twice, then dried over anhydrous sodium sulfate, filtered and concentrated. The residue was purified using a normal phase silica gel chromatographic column (petroleum ether : ethyl acetate = 10 : 1) to collect the product, which was concentrated to afford tert-butyl 2-(methoxy-*d*$_3$)-7-azaspiro[3.5]nonane-7-carboxylate (027-2) (1.5 g).

Preparation of compound 027-3:

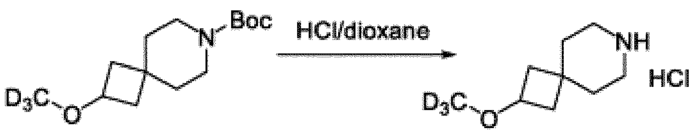


027-2 027-3

At room temperature, tert-butyl 2-(methoxy-*d*$_3$)-7-azaspiro[3.5]nonane-7-carboxylate (027-2) (1.5 g) was dissolved in a hydrogen chloride-dioxane (15 mL, 4M) solution, and then the mixture was reacted and stirred at room temperature for 2 hours. The reaction was complete as detected by TLC. The reaction solution was directly concentrated under reduced pressure. The residue was slurried with ethyl acetate (5 mL) and filtered. The solid was dried to afford 2-(methoxy-*d*$_3$)-7-azaspiro[3.5]nonane hydrochloride (027-3) (1.0 g).

Reference can be made to the synthetic method of compound 002 in example 3 for the other steps and 2-(methoxy-*d*$_3$)-7-azaspiro[3.5]nonane hydrochloride (027-3) was used in place of 2-methoxy-7-azaspiro[3.5]nonane hydrochloride to afford the target product 1-ethyl-4-((R)-2-hydroxy-2-((S)-1,2,3,4-tetrahydroisoquinolin-3-yl)ethyl)-8-(2-(methoxy-$d_3$)-7-azaspiro[3.5]nonane-7-carbonyl)-1,2,3,4-tetrahydro-5H-benzo[e][1,4]diazepin-5-one hydrochloride (compound 027).

**$^1$H NMR** (400 MHz, $CD_3OD$): δ 7.67 (d, *J* = 7.6 Hz, 1 H), 7.29-7.23 (m, 4 H), 7.21-714 (m, 2 H), 4.48-4.32 (m, 3 H), 4.00-3.91 (m, 2 H), 3.73-3.61 (m, 7 H), 3.53-3.42 (m, 1 H), 3.34-3.29 (m, 4 H), 3.27-3.21 (m, 2 H), 2.26-2.24 (m, 2 H), 1.68-1.55 (m, 6 H), 1.19 (t, *J* = 7.0 Hz, 3 H).
**LCMS:** Rt: 1.469 min; MS m/z (ESI): 550.4 [M+H].

**Test Examples of Biological Activity and Related Properties**

Test Example 1: PRMT5 Enzymatic Activity Inhibition Experiment

**Materials:** PRMTS/MEP50 proteins were purchased from BPS Bioscience Inc. (USA); Histone H4 Peptide substrate was purchased from Sangon Biotech (Shanghai) Co., Ltd.; Anti-Histone H4 (symmetric dimethyl R3) antibody - ChIP Grade was purchased from Abeam PLC. (USA); S-(5'-Adenosyl)-L-methionine chloride dihydrochloride was purchased from Sigma PLC. (USA); 384-well plate, AlphaScreen Streptavidin Donor beads, AlphaScreen Protein A Acceptor beads, and Envision 2104 multi-label Reader were purchased from Perkin-Elmer Instruments (USA) Corporation; and Echo 550 Liquid Handler was purchased from Labcyte Inc. (USA).

**Enzymatic activity assay:** The compounds were added into a 384-well plate by using the Echo 550 Liquid Handler with the final concentration of 0-1000 nM (initial concentration: 1000 nM, 3-fold dilution, for 10 points), and the DMSO content was 0.5%. 10 μL of a 2X PRMTS/MEP50 solution was added into each well and incubated at room temperature for 30 minutes. 10 μL of 2X PRMTS/MEP50 substrate solution was added to each well to start the reaction and incubated at room temperature for 60 minutes. A 6X detection reagent containing AlphaScreen Protein A Acceptor beads and Anti-Histone H4 (symmetric dimethyl R3) antibodies was prepared and 5 μL was added to each well. The plate was incubated at room temperature for 60 minutes. A 6X detection reagent containing AlphaScreen Streptavidin Donor beads was prepared and 5 μL was added to each well. The plate was incubated at room temperature for 60 minutes. The signal values were detected by using the Envision. The test results are shown in Table 2.

Test Example 2: Inhibitory Activity Experiment of the Compounds on Tumor Cell Proliferation

**Materials and cells:** Z-138 cells were purchased from ATCC (USA); IMDM medium and penicillin-streptomycin were purchased from Sigma PLC. (USA); horse serum was purchased from Hyclone (USA); 96-well plate was purchased from Corning (USA); and Cell-Titer Glo reagent was purchased from Promega (USA).

**Cell culture:** Z-138 cells were cultured in an IMDM culture solution containing 10% Horse serum + 1% penicillin - streptomycin at 37°C, 5% $CO_2$. Cells in logarithmic growth phase were suitable for the experiment.

**Detection of cell proliferation activity:** The inhibitory activities of the compounds on the proliferation of Z-138 cells were detected by using the Cell-Titer Glo reagent. The concentrations of the cells were adjusted, and 180 μL was inoculated in each well of the 96-well plate (500/well) and equilibrated for 10-15 minutes at 37°C, 5% $CO_2$. 20 μL of a culture solution containing the compounds was added into each well with the final concentration reaching 0-300 nM (initial concentration: 300 nM, 3-fold dilution, for 10 points), and the DMSO content was 0.1%. The cell plate was placed at 37°C, 5% $CO_2$ and incubated for 8 days. Culture solution was replaced at day 4: 100 μL of the supernatant was slowly aspirated and 100 μL of a fresh culture solution containing the compounds was provided as a supplement while the concentrations of the compounds were held constant. The cell viability was detected by using the Cell-Titer Glo reagent. The test results are shown in Table 2.

Test Example 3: Inhibitory Activity Experiment of Compounds on SDMA

**Materials and cells:** Z-138 cells were purchased from ATCC (USA); IMDM medium and penicillin-streptomycin were purchased from Sigma PLC. (USA); horse serum was purchased from Hyclone (USA); Hoechst antibodies were purchased from Invitrogen (USA); Alexa Fluor 488 goat anti-rabbit IgG antibodies were purchased from Invitrogen (USA); Anti-dimethyl-Arginine symmetric (SYM11) antibodies were purchased from Merck & Co., Inc. (USA); DPBS was purchased from Gibco (USA); Nonfat Dry milk was purchased from Cell Signaling Technology (USA); paraformaldehyde was purchased from Beijing Solarbio Science & Technology Co., Ltd.; 384-well plate and Echo 550 Liquid Handler were purchased from Labcyte Inc. (USA); and an ImageXpress Nano was purchased from Molecular Devices (USA).

**Cell culture:** Z-138 cells were cultured in an IMDM culture solution containing 10% Horse serum + 1% penicillin - streptomycin at 37°C, 5% $CO_2$. Cells in logarithmic growth phase were suitable for the experiment.

**Immunofluorescent assay:** The effects of the compounds on SDMA in Z-138 cells were detected by using

immunofluorescence. The cells were adjusted to the concentration of 1*$10^5$/ mL and 40 μL was inoculated in each well of the 384-well plate (4000/well) and equilibrated for 10-15 minutes at 37°C, 5% $CO_2$. The compound was added into a 384-well plate by using the Echo 550 Liquid Handler with the final concentration of 0-300 nM (initial concentration: 300 nM, 3-fold dilution, for 10 points), and the DMSO content was 0.1%. The cell plate was placed at 37°C, 5% $CO_2$ and incubated for 2 days. 40 μL of 8% paraformaldehyde was added into each well and incubated at room temperature for 30 minutes. The supernatant was discarded, the plate was washed with DPBS, and 40 μL of 0.5% PBST (phosphate buffer with Tween) was added into each well and incubated at room temperature for 60 minutes. The supernatant was discarded, the plate was washed with 0.05% PBST, and 40 μL of a blocking solution (1% Nonfat milk in 0.05% PBST) was added into each well and incubated at room temperature for 60 minutes. The supernatant was discarded, and 20 μL of the primary antibody (SYM11, diluted with a blocking solution at 1 : 500) was added into each well and maintained at 4°C overnight. The supernatant was discarded, the plate was washed with 0.05% PBST, 20 μL of the secondary antibody (Alexa Fluor 488 goat anti-rabbit and Hoechst, diluted with a blocking solution at 1 : 1000 and 1 : 5000, respectively) was added to each well and incubated at room temperature for 60 minutes. The supernatant was discarded, the plate was washed with 0.05% PBST, and the fluorescent intensity was detected with ImageXpress Nano. The test results are shown in the table below.

Table 2

| Compounds | Inhibition of enzymatic activity $IC_{50}$ (nM) | Inhibition of cell proliferation Z-138 $IC_{50}$ (nM) | Inhibition of SDMA in cells $IC_{50}$ (nM) |
|---|---|---|---|
| 001 | 4.56 | 3.47 | 3.80 |
| 002 | 4.05 | 2.19 | 1.40 |
| 003 | 2.02 | 1.21 | 0.80 |
| 004 | 4.53 | 1.87 | 1.40 |
| 005 | 6.73 | 4.27 | 1.50 |
| 006 | 3.62 | 4.80 | 1.80 |
| 007 | 7.66 | 7.55 | NA |
| 008 | 12.24 | 6.04 | NA |
| 009 | 4.96 | 2.97 | 6.10 |
| 010 | NA | 1.93 | NA |
| 011 | NA | 2.20 | NA |
| 012 | NA | 4.09 | NA |
| 013 | NA | 2.37 | NA |
| 014 | NA | 11.69 | NA |
| 015 | NA | 11.14 | NA |
| 016 | NA | 45.10 | NA |
| 017 | NA | 27.59 | NA |
| 018 | NA | 25.99 | NA |
| 019 | NA | 20.44 | NA |
| 020 | NA | 3.71 | NA |
| 021 | NA | 8.35 | NA |
| 022 | NA | 14.72 | NA |
| 023 | NA | 16.78 | NA |
| 024 | NA | 10.61 | NA |
| 025 | NA | 138.47 | NA |
| 026 | NA | 5.62 | NA |

(continued)

| Compounds | Inhibition of enzymatic activity $IC_{50}$ (nM) | Inhibition of cell proliferation Z-138 $IC_{50}$ (nM) | Inhibition of SDMA in cells $IC_{50}$ (nM) |
|---|---|---|---|
| 027 | NA | 2.36 | NA |
| Notes: "NA" means that no test is performed. | | | |

Test Example 4: Pharmacokinetic Experiment in Mice

**Experimental materials:** CB17-SCID mice were purchased from Beijing Vital River Laboratory Animal Technology Co., Ltd.; DMSO, HP-β-CD (Hydroxypropyl-β-cyclodextrin), MC (methyl cellulose) and acetonitrile were purchased from Merck & Co., Inc. (USA), and $K_2$EDTA anticoagulation tubes were purchased from Jiangsu Xinkang Medical Instrument Co., Ltd.

**Experiment method:** Six female CB17-SCID mice (20-30 g, 4-6 weeks) were randomized into 2 groups, 3 mice/group. Group 1 was given compound **002** via tail vein injection at a dose of 2 mg/kg, and the vehicle was 5% of DMSO + 95% of 10% HP-β-CD aqueous solution; and group 2 was given compound **002** orally at a dose of 10 mg/kg, and the vehicle was 0.5% MC aqueous solution. Prior to the experiment, the animals were given food and water as usual. At pre-dose and 0.083 (only for the vein injection group), 0.25, 0.5, 1, 2, 4, 6, 8 and 24 h post-dose, blood was sampled from the mice in each group from the vein. The whole blood sample collected was placed in a $K_2$EDTA anticoagulation tube and centrifuged for 5 min (4000 rpm, 4°C) and then the plasma was taken for detection.

10 μL of the mouse plasma sample was taken and 150 μL of acetonitrile solvent (wherein internal standard compound was contained) was added to precipitate proteins. After the mixture was vortexed for 0.5 min, centrifugation (4700 rpm, 4°C) was performed for 15 min. The supernatant was diluted 2-fold with water containing 0.05% (v/v) formic acid and 3 μL was injected into the LC-MS/MS system (AB Sciex Triple Quad 6500+) for the quantitative detection. The concentrations of the samples were determined with the present of plasma standard curve (linear range: 0.5-1000 ng/mL) of CB17-SCID mice and quality control samples. For the preparation of 10-fold diluted samples, 2 μL of mouse plasma sample was taken and 18 μL of blank plasma was added. After the mixture was vortexed for 0.5 min, 300 μL of acetonitrile solvent (wherein internal standard compound was contained) was added to precipitate proteins. The other processing steps were identical to those for the samples not diluted.

Pharmacokinetic test results are as shown in Table 3.

Table 3 Results of pharmacokinetic tests in mice

| | PK parameters | Compound **002** |
|---|---|---|
| Mice IV (2 mpk) | Cl_obs (mL/min/kg) | 61.1 ± 6.2 |
| | $T_{1/2}$ (h) | 3.56 ± 0.55 |
| | $V_{ss}$_obs (L/kg) | 8.20 ± 0.60 |
| Mice PO (10 mpk) | $T_{max}$ (h) | 0.33 ± 0.14 |
| | $C_{max}$ (ng/mL) | 827 ± 79 |
| | $T_{1/2}$ (h) | 2.68 ± 0.20 |
| | $AUC_{last}$ (h*ng/mL) | 2646 ± 563 |
| | F (%) | 96.5 ± 20.5 |

Test Example 5: Hepatocellular Metabolic Stability Test

**Experimental materials:** Human hepatocytes were purchased from Biopredic; mouse hepatocytes were purchased from BioIVT; acetonitrile and methanol were purchased from Merck & Co., Inc.; AOPI dyes were purchased from Nexcelom; dexamethasone was purchased from NIFDC; DMSO was purchased from Beijing Solarbio Science & Technology Co., Ltd.; DPBS (10x), GlutaMAX™-1 (100x) and recombinant human insulin were purchased form Gibco by Life Technologies; fetal bovine serum was purchased from Corning; formic acid was purchased from DIKMAPURE; Isotonic Percoll was purchased from GE Healthcare; alprazolam was purchased from Supelco; caffeine was purchased from ChromaDex.inc; and HEPES, tolbutamide and Williams' Medium E were purchased from Sigma.

**Experiment preparation:**

The powder of a test substance was formulated with DMSO into a stock solution with a high concentration, which was diluted to a working solution of 100 μM with acetonitrile prior to use. The final concentration of the test substance was 1 μM.

The specific preparation information of the hepatocyte thawing media is shown in Table 4 below. 49.5 mL of Williams'E Medium and 0.5 mL of GlutaMAX were mixed as an incubation media. Hepatocyte thawing media and incubation media were placed in a 37°C water-bath and pre-heated for at least 15 minutes prior to use. A tube of hepatocytes stored at an ultra-low temperature was taken and the hepatocytes were ensured to be at a frozen state at a low temperature prior to thawing. The hepatocytes were rapidly placed in a 37°C water-bath and gently shaken until all the ice crystals were dispersed, and then after 70% ethanol was sprayed, the hepatocytes were transferred to a biosafety cabinet. The content of the tube was poured into a centrifugal tube containing 50 mL of thawing media and centrifuged at 100 g for 10 minutes. After centrifugation, the thawing media was aspirated, and a sufficient amount of incubation media was added to obtain a cell suspension with a cell density of about $1.5 \times 10^6$ cells/mL. The hepatocytes were counted, and the viable cell density was determined using Cellometer Vision, and the survival rate of the hepatocytes must be higher than 75%. The hepatocyte suspension was diluted by using the incubation media to a viable cell density of $0.5 \times 10^6$ viable cells/mL.

Table 4 Preparation of hepatocyte thawing media

| Reagents | Initial concentration | Final concentration | Volume of reagent |
|---|---|---|---|
| Williams' Medium E | - | - | 31.2 mL |
| Isotonic Percoll | - | 30% | 13.5 mL |
| DPBS (10x) | - | - | 1.5 mL |
| GlutaMAX™-1 (100x) | 200 mM/100x | 2 mM | 500 μL |
| HEPES | 1 M | 15 mM | 750 μL |
| FBS | - | 5% | 2.5 mL |
| Recombinant human insulin | 4 mg/mL | 4 μg/mL | 50 μL |
| Dexamethasone | 10 mM | 1 μM | 5μL |

**Experiment method:**

247.5 μL of viable cell (human hepatocytes or mouse hepatocytes) suspension or media were transferred into a 96-well deep-well plate, and the deep-well plate was placed in a vortex incubator and pre-heated for 10 minutes. Incubation of all the samples was performed in parallel duplicate. Test substance (2.5 μL, 100 μM) was added into each well to start the reaction and the deep-well plate was placed in the vortex incubator again. The samples were incubated, and 25 μL of the suspension was taken at 0, 15, 30, 60, 90 and 120 minutes, respectively. 125 μL of acetonitrile containing internal standard (100 nM alprazolam, 200 nM caffeine, and 100 nM tolbutamide) was added to terminate the reaction. The mixture was vortexed for 10 minutes and centrifuged at 3220 g, 4°C for 30 minutes. After centrifugation, 100 μL of the supernatant was transferred into the loading plate and 150 μL of pure water was added and mixed uniformly for LC-MS/MS analysis.

All the data calculation was performed by the Microsoft Excel software. The peak area was detected by extracting the ion chromatogram. The *in vitro* half-life ($t_{1/2}$) of the parent drug was detected by performing linear fitting of the natural logarithm of elimination percent of the parent drug and time.

The *in vitro* half-life ($t_{1/2}$) was calculated by means of the slope:

*in vitro* $t_{1/2} = 0.693/k$.

The experimental results are as shown in Table 5.

Table 5 Results of hepatocellular metabolic stability test

| Compounds | Human $t_{1/2}$ (min) | Mouse $t_{1/2}$ (min) |
|---|---|---|
| **001** | 1471.30 | 122.34 |
| **002** | 252.37 | 94.62 |
| **003** | 7862.70 | 118.05 |
| **004** | 671.53 | 52.26 |

(continued)

| Compounds | Human $t_{1/2}$ (min) | Mouse $t_{1/2}$ (min) |
|---|---|---|
| **005** | 336.63 | 77.84 |
| **024** | 772.45 | 35.29 |

Test Example 6: *In Vivo* Drug Efficacy Experiment in Mice

**Experimental materials:** Z138 cells were purchased from ATCC; IMDM culture solution, penicillin-streptomycin and 0.25% trypsin-EDTA were purchased from Gibco; horse serum and PBS were purchased from Hyclone; and Matrigel was purchased from Corning.

**Animal information:** CB17-SCID mice (female, 6-7 weeks, body weight: about 14-20 g) were purchased from Shanghai Lingchang Biotechnology Co., Ltd. The mice were fed in SPF environment and each cage position was individually ventilated. All animals had free access to standard certified commercial laboratory food and water.

**Experiment method:**

Cell culture: Human mantle cell lymphoma Z-138 cell lines were cultured *in vitro* and the culture conditions involve: IMDM (cell culture solution) supplemented with 10% horse serum and 1% penicillin-streptomycin solution, 37°C, 5% $CO_2$ incubator. Conventional digestion treatment with 0.25% trypsin-EDTA digestion solution was carried out twice a week for passage. When the cell saturation was 85%-90%, and the number reached the requirement, the cells were collected and counted.

Cell inoculation: 0.1 ml/ (containing $1 \times 10^7$) Z-138 cell suspension (PBS : Matrigel = 1 : 1) was inoculated subcutaneously on the right back of each mouse. At day 18 post inoculation, when the average tumor volume measured has reached about 125 $mm^3$, grouping and administration were performed by using the randomized stratification and grouping method based on the tumor volume and animal body weight. PBS was a phosphate buffer free of Ca and Mg ions, and Matrigel was a matrix gel.

Administration: Compound **002** was administered at the doses of 1.5 mg/kg, 5 mg/kg, or 15 mg/kg, PO, twice daily (BID) $\times$ 3 weeks. 6 mice per group.

**Tumor measurement and experimental index:**

The diameter of the tumor was measured with vernier calipers twice a week. The calculation formula of tumor volume was $V = 0.5a \times b^2$, wherein a and b represented the long and short diameters of the tumor, respectively. The body weights of the mice were weighed twice a week.

The tumor-inhibiting efficacy of the compound was evaluated using tumor growth inhibition (TGI) (%).

TGI (%) = [(1-(average tumor volume at the end of administration in a certain treatment group - average tumor volume at the beginning of administration in the treatment group)/ (average tumor volume at the end of administration in the solvent control group - average tumor volume at the beginning of administration in the solvent control group)] $\times$ 100%.

**Experimental results:**

See table 6, FIG. 2 and FIG. 3. No morbidity or death of the mice occurred during the experiment.

**Table 6 Tumor volumes of Z-138 subcutaneous tumor models**

| Groups | **Test compounds** | **Dose** | **Frequency of administration** | **Average tumor volume ($mm^3$)** | | | | | | | **TGI (%)** |
|---|---|---|---|---|---|---|---|---|---|---|---|
| | | | | **Day 0** | **Day 4** | **Day 7** | **Day 11** | **Day 14** | **Day 18** | **Day 21** | |
| Group 1 | Solvent control | / | BID | 125 | 216 | 286 | 422 | 543 | 741 | 1007 | / |
| Group 2 | Compound **002** | 1.5 mg/kg | BID | 125 | 151 | 203 | 374 | 436 | 567 | 689 | 36.0% |
| Group 3 | Compound **002** | 5 mg/kg | BID | 124 | 157 | 197 | 281 | 310 | 452 | 585 | 47.8% |
| Group 4 | Compound **002** | 15 mg/kg | BID | 126 | 140 | 113 | 59 | 34 | 18 | 16 | 112.5% |

**Experiment conclusion:**

In Z-138 models of mice with subcutaneous transplantation tumor, compound **002** of the present disclosure has significant inhibitory effects on tumor growth when it is administered twice daily at 1.5 mg/kg, 5 mg/kg and 15 mg/kg and shows a good dose-response relationship. When administered twice daily at 15 mg/kg, the compound shows an effect of shrinking tumor. In this efficacy experiment, the tested doses of compound **002** do not significantly affect the body weights of the mice, nor did they cause any death in mice, and the mice can tolerate the doses.

The embodiments of the present disclosure are illustrated as above. However, the present disclosure is not limited to the above-mentioned embodiments. Any modifications, equivalent substitutions, improvements, etc. made within the spirit and principles of the present disclosure shall be included within the scope of protection of the present disclosure.

## Claims

1. A compound of formula (I), or a pharmaceutically acceptable salt thereof:

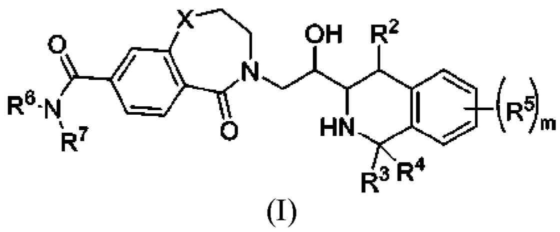

(I)

wherein

X is selected from $NR^1$, $CR^8R^9$, or O;
$R^1$ is selected from H, $C_1$-$C_{10}$ alkyl, $C_2$-$C_{10}$ alkenyl, $C_2$-$C_{10}$ alkynyl, $C_6$-$C_{10}$ aryl, 5- to 10-membered heteroaryl, $C_3$-$C_{10}$ cycloalkyl, or 3- to 10-membered heterocyclyl, wherein $C_1$-$C_{10}$ alkyl, $C_2$-$C_{10}$ alkenyl, $C_2$-$C_{10}$ alkynyl, $C_6$-$C_{10}$ aryl, 5- to 10-membered heteroaryl, $C_3$-$C_{10}$ cycloalkyl, or 3- to 10-membered heterocyclyl is optionally substituted with one or more $R^a$;
$R^2$ and $R^5$ are independently selected from H, halogen, CN, or the following groups which are optionally substituted with one or more $R^b$: $NH_2$, $C_1$-$C_{10}$ alkyl, $C_1$-$C_{10}$ alkoxy, $C_3$-$C_{10}$ cycloalkyl, or 3- to 10-membered heterocyclyl;
$R^3$ and $R^4$ are independently selected from H, deuterium, halogen, CN, or the following groups which are optionally substituted with one or more $R^b$: $NH_2$, $C_1$-$C_{10}$ alkyl, $C_1$-$C_{10}$ alkoxy, $C_3$-$C_{10}$ cycloalkyl, or 3- to 10-membered heterocyclyl;
or, $R^3$ and $R^4$ together with the C to which they are attached form $C_3$-$C_{10}$ cycloalkyl or 3-to 10-membered heterocyclyl, wherein the $C_3$-$C_{10}$ cycloalkyl or 3- to 10-membered heterocyclyl is optionally substituted with one or more $R^e$;
$R^8$ and $R^9$ are independently selected from H, OH, CN, $NO_2$, $C_1$-$C_{10}$ alkyl, $C_1$-$C_{10}$ alkoxy, $C_3$-$C_{10}$ cycloalkyl, or 3- to 10-membered heterocyclyl, wherein the $C_1$-$C_{10}$ alkyl, $C_1$-$C_{10}$ alkoxy, $C_3$-$C_{10}$ cycloalkyl, or 3- to 10-membered heterocyclyl is optionally substituted with one or more $R^f$;
m is selected from 0, 1, 2, 3 or 4;
$R^a$ is selected from halogen, =O, OH, CN, $NO_2$, $C_1$-$C_{10}$ alkyl, $C_1$-$C_{10}$ alkoxy, $C_6$-$C_{10}$ aryl, 5- to 10-membered heteroaryl, $C_3$-$C_{10}$ cycloalkyl, or 3- to 10-membered heterocyclyl;
$R^b$ is selected from halogen, OH, CN, =O, $NO_2$, $C_1$-$C_{10}$ alkyl, $C_3$-$C_{10}$ cycloalkyl, 3- to 10-membered heterocyclyl, $C_1$-$C_{10}$ alkoxy, $C_3$-$C_{10}$ cycloalkyloxy, 3- to 10-membered heterocyclyloxy, $C_2$-$C_{10}$ alkenyl, $C_2$-$C_{10}$ alkynyl, $C_6$-$C_{10}$ aryl, 5- to 10-membered heteroaryl, $C_6$-$C_{10}$ aryloxy, or 5- to 10-membered heteroaryloxy;
$R^e$ and $R^f$ are independently selected from halogen, =O, OH, CN, $NO_2$, $C_1$-$C_{10}$ alkyl, or $C_1$-$C_{10}$ alkoxy;
$R^6$ and $R^7$ are independently selected from H, $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl, wherein the $C_1$-$C_{10}$ alkyl, $C_6$-$C_{10}$ aryl, 5- to 10-membered heteroaryl, $C_3$-$C_{10}$ cycloalkyl, or 3- to 10-membered heterocyclyl is optionally substituted with one or more $R^c$;
$R^c$ is selected from halogen, OH, CN, =O, $C_1$-$C_{10}$ alkoxy, $C_6$-$C_{10}$ aryl, $C_6$-$C_{10}$ aryloxy, 5- to 10-membered heteroaryl, 5- to 10-membered heteroaryloxy, $C_3$-$C_{10}$ cycloalkyl, $C_3$-$C_{10}$ cycloalkyloxy, 3- to 10-membered heterocyclyl, or 3- to 10-membered heterocyclyloxy, wherein the $C_1$-$C_{10}$ alkoxy, $C_6$-$C_{10}$ aryl, $C_6$-$C_{10}$ aryloxy,

5- to 10-membered heteroaryl, 5-to 10-membered heteroaryloxy, $C_3$-$C_{10}$ cycloalkyl, $C_3$-$C_{10}$ cycloalkyloxy, 3- to 10-membered heterocyclyl, or 3- to 10-membered heterocyclyloxy is optionally substituted with one or more $R^{c1}$;
$R^{c1}$ is selected from halogen, OH, =O, CN, $NO_2$, $C_1$-$C_{10}$ alkyl, or $C_1$-$C_{10}$ alkoxy;
or, $R^6$ and $R^7$ together with the N to which they are attached form 6- to 13-membered spiroheterocycloalkyl, 6- to 13-membered fused heterocycloalkyl, 6- to 13-membered bridged heterocycloalkyl, 3- to 8-membered monocyclic heterocycloalkyl, or 5- to 10-membered heteroaryl, wherein the 6- to 13-membered spiroheterocycloalkyl, 6- to 13-membered fused heterocycloalkyl, 6- to 13-membered bridged heterocycloalkyl, 3- to 8-membered monocyclic heterocycloalkyl, or 5- to 10-membered heteroaryl is optionally substituted with one or more $R^d$;
$R^d$ is selected from halogen, OH, =O, CN, $NO_2$, $C_1$-$C_{10}$ alkyl, $C_1$-$C_{10}$ alkoxy, $C_1$-$C_{10}$ alkyl-NH-, ($C_1$-$C_{10}$ alkyl)$_2$-N-, $C_6$-$C_{10}$ aryl, 5- to 10-membered heteroaryl, $C_3$-$C_{10}$ cycloalkyl, $C_3$-$C_{10}$ cycloalkyloxy, $C_3$-$C_{10}$ cycloalkyl-NH-, ($C_3$-$C_{10}$ cycloalkyl)$_2$-N-, or 3- to 10-membered heterocyclyl, wherein the $C_1$-$C_{10}$ alkyl, $C_1$-$C_{10}$ alkoxy, $C_1$-$C_{10}$ alkyl-NH-, ($C_1$-$C_{10}$ alkyl)$_2$-N-, $C_6$-$C_{10}$ aryl, 5- to 10-membered heteroaryl, $C_3$-$C_{10}$ cycloalkyl, $C_3$-$C_{10}$ cycloalkyloxy, $C_3$-$C_{10}$ cycloalkyl-NH-, ($C_3$-$C_{10}$ cycloalkyl)$_2$-N-, or 3- to 10-membered heterocyclyl is optionally substituted with one or more $R^{d1}$;
$R^{d1}$ is selected from deuterium, halogen, OH, =O, CN, $NO_2$, or $C_1$-$C_{10}$ alkoxy.

2. The compound of formula (I) or the pharmaceutically acceptable salt thereof according to claim 1, wherein $R^2$, $R^3$, $R^4$ and $R^5$ are independently selected from H, halogen, CN, or the following groups which are optionally substituted with one or more $R^b$: $NH_2$, $C_1$-$C_{10}$ alkyl, $C_1$-$C_{10}$ alkoxy, $C_3$-$C_{10}$ cycloalkyl, or 3- to 10-membered heterocyclyl; or, $R^3$ and $R^4$ together with the C to which they are attached form $C_3$-$C_{10}$ cycloalkyl or 3-to 10-membered heterocyclyl, wherein the $C_3$-$C_{10}$ cycloalkyl or 3- to 10-membered heterocyclyl is optionally substituted with one or more $R^e$.

3. The compound of formula (I) or the pharmaceutically acceptable salt thereof according to claim 1 or 2, wherein $R^d$ is selected from halogen, OH, =O, CN, $NO_2$, $C_1$-$C_{10}$ alkyl, $C_1$-$C_{10}$ alkoxy, $C_1$-$C_{10}$ alkyl-NH-, ($C_1$-$C_{10}$ alkyl)$_2$-N-, $C_6$-$C_{10}$ aryl, 5- to 10-membered heteroaryl, $C_3$-$C_{10}$ cycloalkyl, $C_3$-$C_{10}$ cycloalkyloxy, $C_3$-$C_{10}$ cycloalkyl-NH-, ($C_3$-$C_{10}$ cycloalkyl)$_2$-N-, or 3- to 10-membered heterocyclyl, wherein the $C_1$-$C_{10}$ alkyl, $C_1$-$C_{10}$ alkoxy, $C_6$-$C_{10}$ aryl, 5- to 10-membered heteroaryl, $C_3$-$C_{10}$ cycloalkyl, or 3- to 10-membered heterocyclyl is optionally substituted with one or more $R^{d1}$; $R^{d1}$ is selected from halogen, OH, =O, CN, $NO_2$, or $C_1$-$C_{10}$ alkoxy.

4. The compound of formula (I) or the pharmaceutically acceptable salt thereof according to any one of claims 1 to 3, wherein $R^6$ and $R^7$ are independently selected from H, $C_1$-$C_{10}$ alkyl, $C_3$-$C_{10}$ cycloalkyl, or 3- to 10-membered heterocyclyl, wherein the $C_1$-$C_{10}$ alkyl, $C_3$-$C_{10}$ cycloalkyl, or 3- to 10-membered heterocyclyl is optionally substituted with one or more $R^c$, and more preferably, the $R^6$ and $R^7$ are independently selected from H, or $C_1$-$C_{10}$ alkyl optionally substituted with one or more $R^c$.

5. The compound of formula (I) or the pharmaceutically acceptable salt thereof according to any one of claims 1 to 3, wherein $R^6$ and $R^7$ together with the N to which they are attached form 6- to 13-membered spiroheterocycloalkyl, 6- to 13-membered fused heterocycloalkyl, 6- to 13-membered bridged heterocycloalkyl, 3- to 8-membered monocyclic heterocycloalkyl, or 5- to 10-membered heteroaryl, wherein the 6- to 13-membered spiroheterocycloalkyl, 6- to 13-membered fused heterocycloalkyl, 6- to 13-membered bridged heterocycloalkyl, 3- to 8-membered monocyclic heterocycloalkyl, or 5-to 10-membered heteroaryl is optionally substituted with one or more $R^d$, and more preferably, the $R^6$ and $R^7$ together with the N to which they are attached form 6- to 13-membered spiroheterocycloalkyl optionally substituted with one or more $R^d$.

6. The compound of formula (I) or the pharmaceutically acceptable salt thereof according to any one of claims 1 to 3, wherein $R^6$ and $R^7$ together with the N to which they are attached form

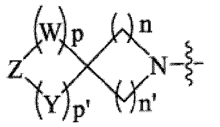

optionally substituted with one or more $R^d$, wherein:

n, n', p, and p' are independently selected from 1, 2, 3, or 4, and n + n' + p + p' ≤ 10;
W and Y are independently selected from $CH_2$, NH, or O; and
Z is selected from $CH_2$, NH, O, or a bond.

**7.** The compound of formula (I) or the pharmaceutically acceptable salt thereof according to claim 6, wherein n, n', p, and p' are independently selected from 1 or 2, and n + n' + p + p' ≤ 8; W and Y are selected from $CH_2$; and Z is selected from O, $CH_2$, or a bond.

**8.** The compound of formula (I) or the pharmaceutically acceptable salt thereof according to any one of claims 1 to 3, wherein $R^6$ and $R^7$ together with the N to which they are attached form 6- to 13-membered bridged heterocycloalkyl optionally substituted with one or more $R^d$.

**9.** The compound of formula (I) or the pharmaceutically acceptable salt thereof according to any one of claims 1 to 3, wherein $R^6$ and $R^7$ together with the N to which they are attached form

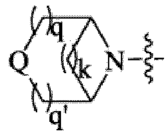

optionally substituted with one or more $R^d$, wherein:

q, q', and k are independently selected from 1, 2 or 3; and
Q is selected from $CH_2$, NH, O, or a bond.

**10.** The compound of formula (I) or the pharmaceutically acceptable salt thereof according to any one of claims 1 to 3, wherein $R^6$ and $R^7$ together with the N to which they are attached form 6- to 13-membered fused heterocycloalkyl, 3- to 8-membered monocyclic heterocycloalkyl, or 5- to 10-membered heteroaryl optionally substituted with one or more $R^d$, and more preferably, $R^6$ and $R^7$ together with the N to which they are attached form a morpholine ring or piperazine ring optionally substituted with one or more $R^d$.

**11.** The compound of formula (I) or the pharmaceutically acceptable salt thereof according to any one of claims 1 to 3, wherein the compound of formula (I) or the pharmaceutically acceptable salt thereof is selected from a compound of formula (II), or a pharmaceutically acceptable salt thereof:

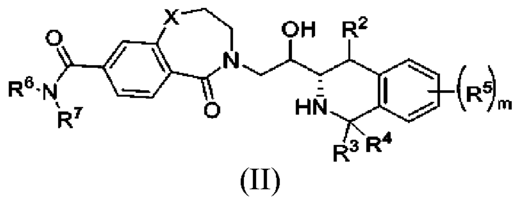

(II)

wherein X, $R^2$, $R^3$, $R^4$, $R^5$, $R^6$, $R^7$, and m are as defined in any one of claims 1 to 3.

**12.** The compound of formula (I) or the pharmaceutically acceptable salt thereof according to any one of claims 1 to 3, wherein the compound of formula (I) or the pharmaceutically acceptable salt thereof is selected from a compound of formula (III), or a pharmaceutically acceptable salt thereof:

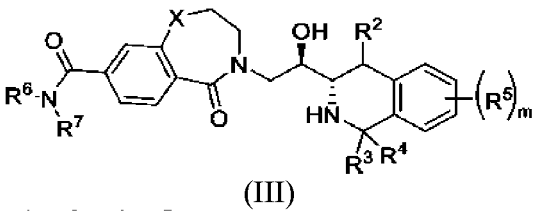

(III)

wherein X, $R^2$, $R^3$, $R^4$, $R^5$, $R^6$, $R^7$, and m are as defined in any one of claims 1 to 3.

**13.** The compound of formula (I) or the pharmaceutically acceptable salt thereof according to claim 1, wherein the compound has a structure selected from one of the following:

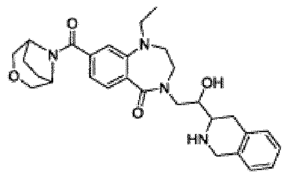
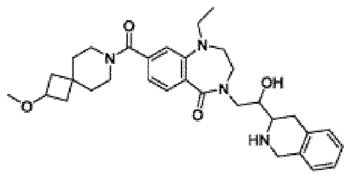
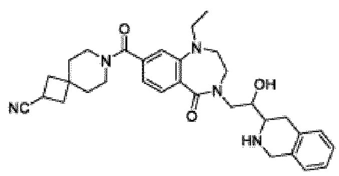

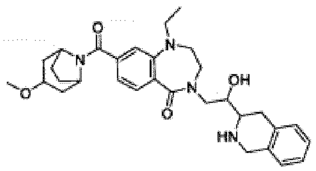
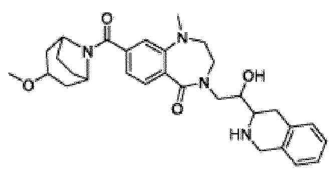
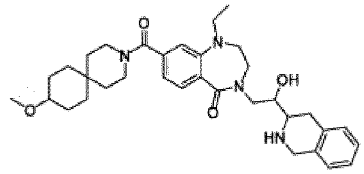

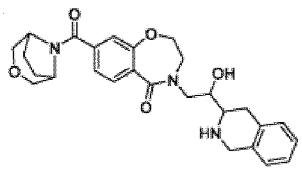
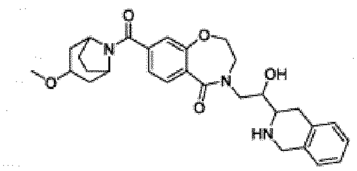
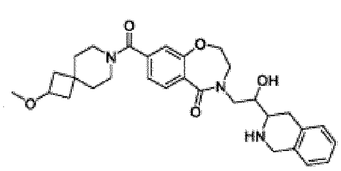

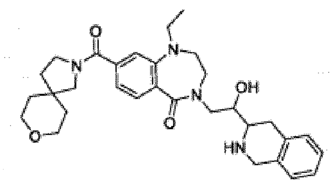
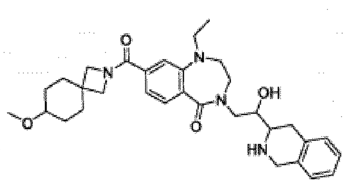
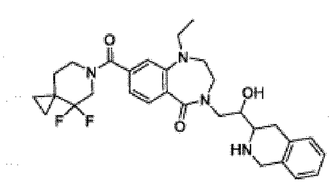

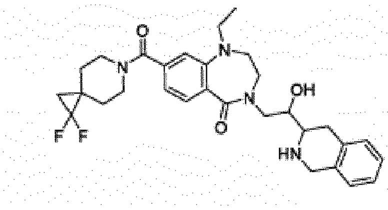
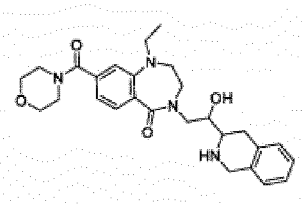
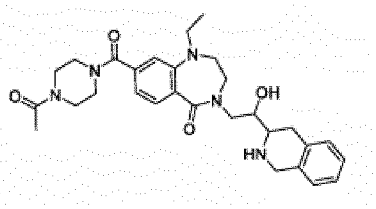

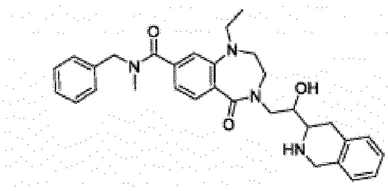
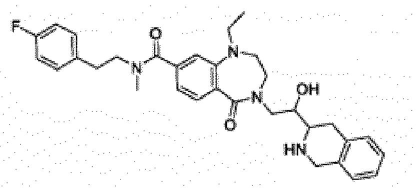

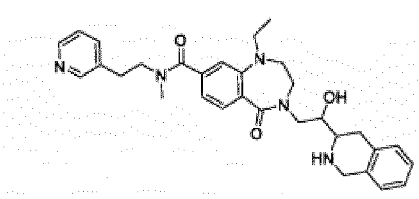
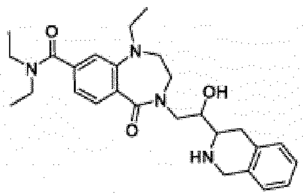
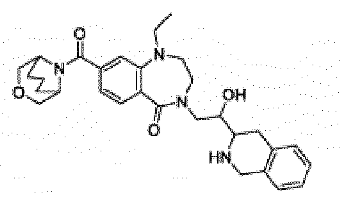

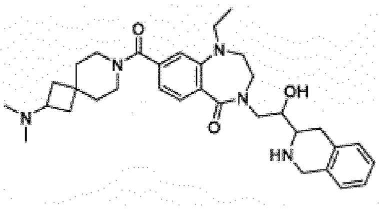
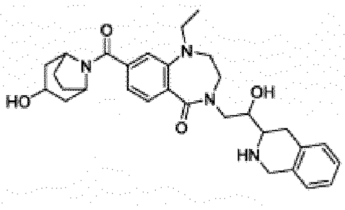
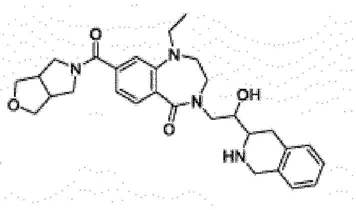

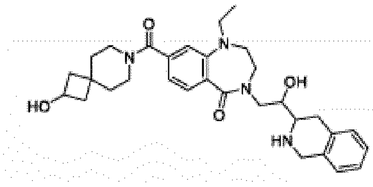
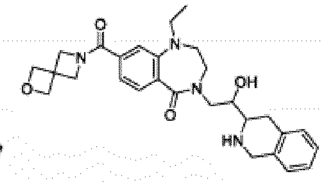
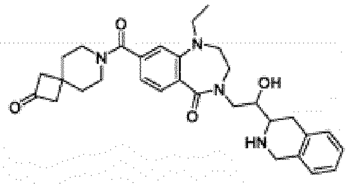

or

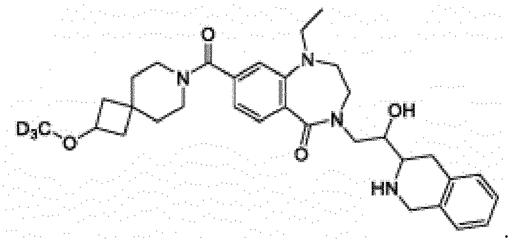

.

**14.** The compound of formula (I) or the pharmaceutically acceptable salt thereof according to claim 1, wherein the compound has a structure selected from one of the following:

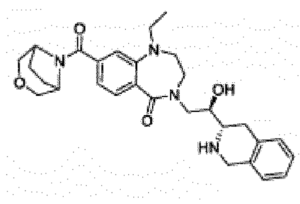
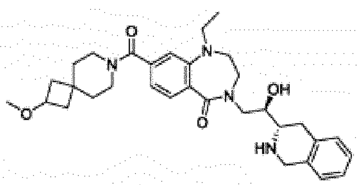
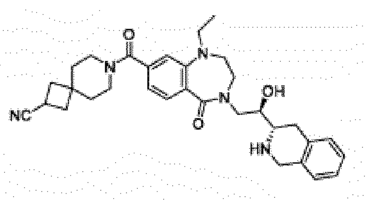

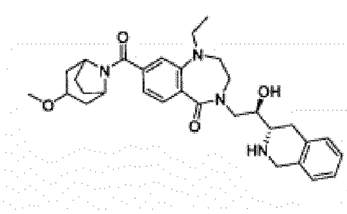
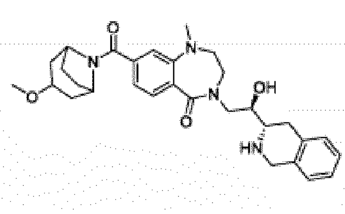
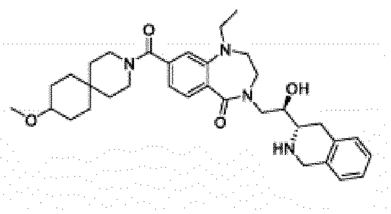

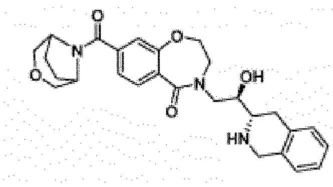
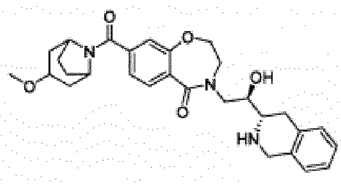
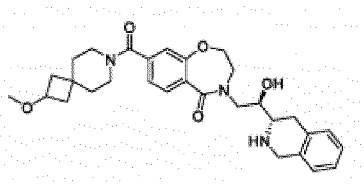

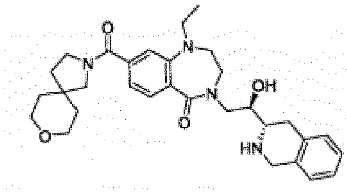
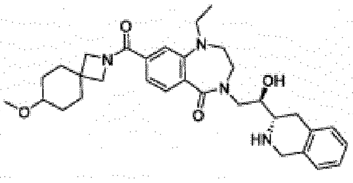
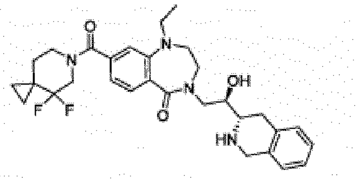

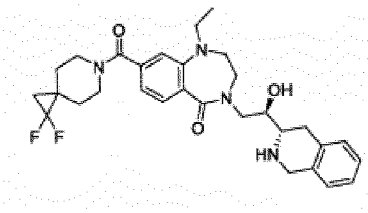
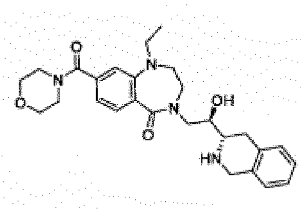
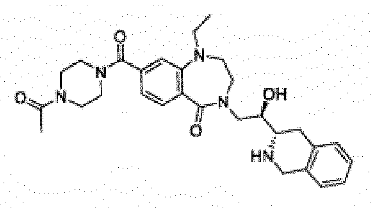

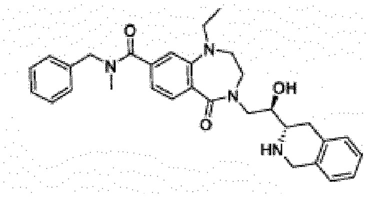
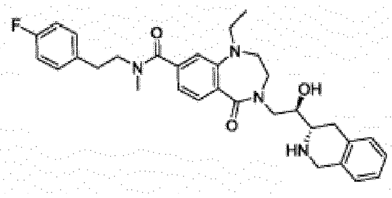

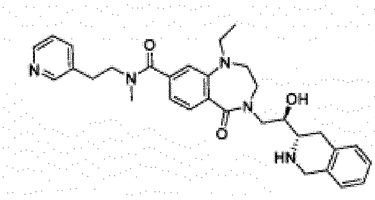
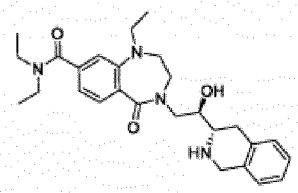
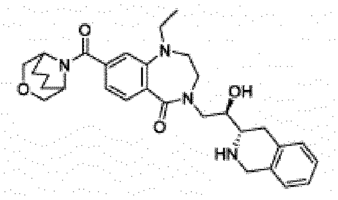

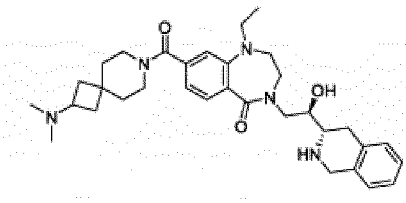
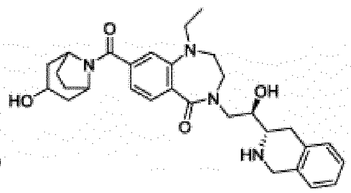
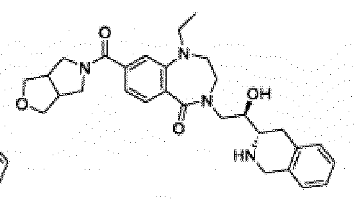

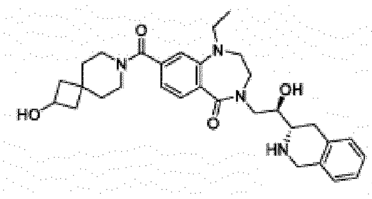
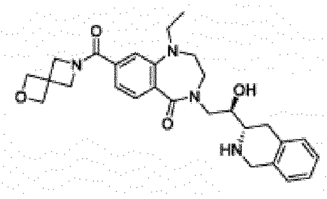
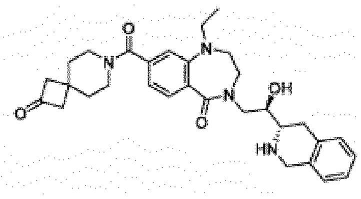

or

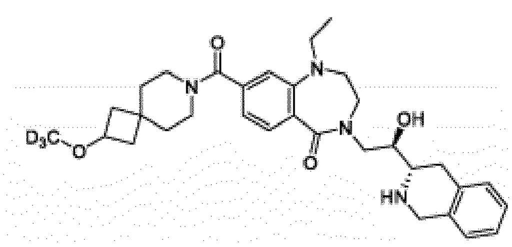

.

**15.** A pharmaceutical composition comprising the compound or the pharmaceutically acceptable salt thereof according to any one of claims 1 to 14, and a pharmaceutically acceptable adjuvant.

**16.** Use of the compound or the pharmaceutically acceptable salt thereof according to any one of claims 1-14 or the pharmaceutical composition according to claim 15 for preventing or treating a disease mediated by PRMT5, wherein the disease mediated by PRMT5 is preferably cancer.

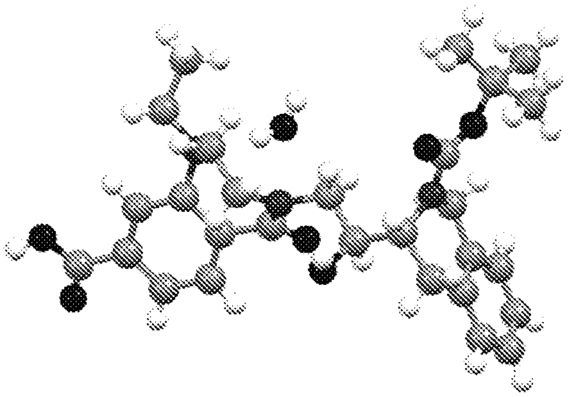

*Fig. 1*

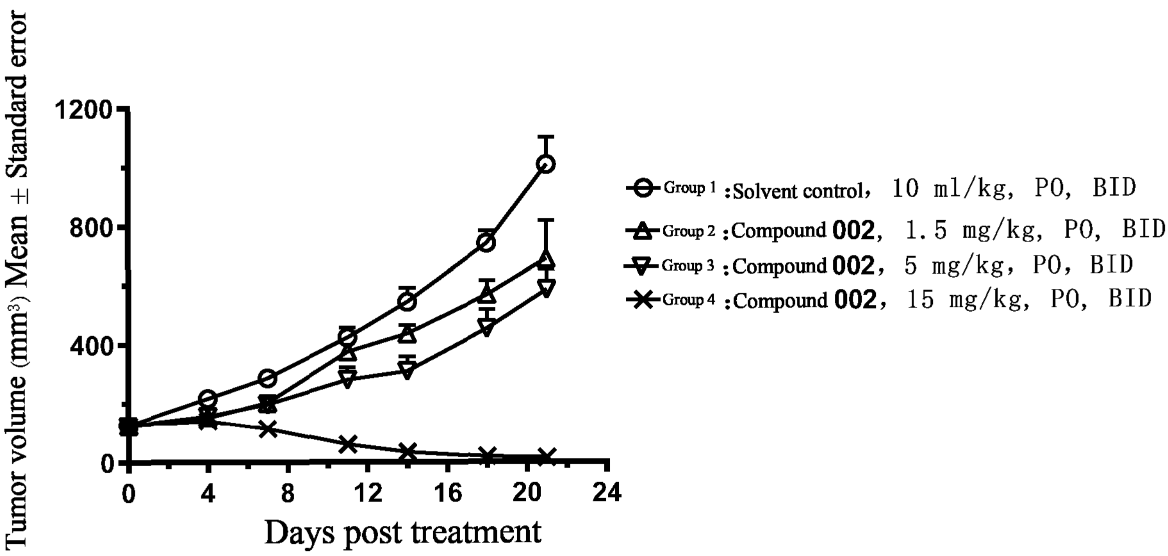


*Fig. 2*

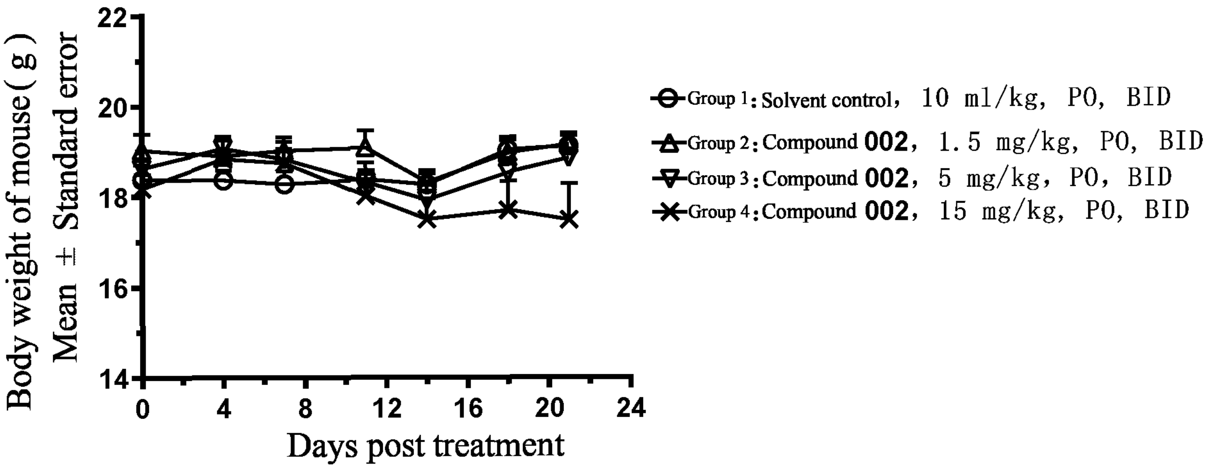


*Fig. 3*

**INTERNATIONAL SEARCH REPORT**

International application No.
**PCT/CN2021/103597**

**A. CLASSIFICATION OF SUBJECT MATTER**

C07D 413/12(2006.01)i; A61P 3/10(2006.01)i; A61P 35/00(2006.01)i; C07D 401/12(2006.01)i; C07D 405/14(2006.01)i; C07D 413/14(2006.01)i; A61K 31/428(2006.01)i; A61P 7/06(2006.01)i; C07D 405/12(2006.01)i; A61K 31/4439(2006.01)i; C07D 471/04(2006.01)i; C07D 471/08(2006.01)i; A61K 31/502(2006.01)i; A61K 31/517(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNKI, VEN, SIPOABS, STN(CAPLUS, REG), PRMT5, 癌, 瘤, 四氢异喹啉, 苯并, 氮杂cancer, tumor, tumour, +tetrahydroisoquinoline+, + benzo+, +aze+, structural formula

**C. DOCUMENTS CONSIDERED TO BE RELEVANT**

| Category* | Citation of document, with indication, where appropriate, of the relevant passages | Relevant to claim No. |
|---|---|---|
| A | WO 2019173804 A1 (PHARMABLOCK SCIENCES NANJING INC.) 12 September 2019 (2019-09-12)<br>entire document | 1-15 |
| A | CN 110650950 A (ARGONAUT THERAPEUTICS LIMITED) 03 January 2020 (2020-01-03)<br>entire document | 1-15 |
| A | CN 110325188 A (INDIANA UNIVERSITY RESEARCH AND TECHNOLOGY CORPORATION) 11 October 2019 (2019-10-11)<br>entire document | 1-15 |
| A | WO 2017153520 A1 (CTXT PTY LTD) 14 September 2017 (2017-09-14)<br>entire document | 1-15 |
| A | WO 2016034673 A1 (CTXT PTY LTD) 10 March 2016 (2016-03-10)<br>entire document | 1-15 |
| A | WO 2019219805 A1 (CTXONE PTY LTD.) 21 November 2019 (2019-11-21)<br>entire document | 1-15 |

☐ Further documents are listed in the continuation of Box C.　　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 September 2021** | **29 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/103597**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

[1] Claim 16 relates to a method for treatment of human or animal bodies. Therefore, the subject matter of claim 16 belongs to a subject matter for which no international search is required. (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/103597**

| Patent document cited in search report | Publication date (day/month/year) | Patent family member(s) | Publication date (day/month/year) |
|---|---|---|---|
| WO 2019173804 A1 | 12 September 2019 | EP 3761978 A1 | 13 January 2021 |
| | | US 2020399261 A1 | 24 December 2020 |
| | | KR 20200119859 A | 20 October 2020 |
| | | CA 3090836 A1 | 12 September 2019 |
| CN 110650950 A | 03 January 2020 | WO 2018167269 A1 | 20 September 2018 |
| | | US 2020325128 A1 | 15 October 2020 |
| | | KR 20190129936 A | 20 November 2019 |
| | | AU 2018235139 A1 | 17 October 2019 |
| | | IL 269354 D0 | 28 November 2019 |
| | | CA 3056724 A1 | 20 September 2018 |
| | | SG 11201908598 P A | 30 October 2019 |
| | | MX 2019011061 A | 19 December 2019 |
| | | GB 201704327 D0 | 03 May 2017 |
| | | JP 2020510091 A | 02 April 2020 |
| | | BR 112019019358 A2 | 14 April 2020 |
| | | EP 3596061 A1 | 22 January 2020 |
| CN 110325188 A | 11 October 2019 | WO 2018081451 A1 | 03 May 2018 |
| | | EP 3532051 A1 | 04 September 2019 |
| | | US 2020062745 A1 | 27 February 2020 |
| | | EP 3532051 A4 | 12 August 2020 |
| WO 2017153520 A1 | 14 September 2017 | US 10787434 B2 | 29 September 2020 |
| | | EP 3426351 B1 | 09 December 2020 |
| | | US 2020010451 A1 | 09 January 2020 |
| | | GB 201604030 D0 | 20 April 2016 |
| | | EP 3426351 A1 | 16 January 2019 |
| WO 2016034673 A1 | 10 March 2016 | EP 3189041 A1 | 12 July 2017 |
| | | US 2017298075 A1 | 19 October 2017 |
| | | EP 3189041 B1 | 28 April 2021 |
| | | US 10494376 B2 | 03 December 2019 |
| WO 2019219805 A1 | 21 November 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010614735
- CN 202110296535
- US 10494376 B2


### Non-patent literature cited in the description


- **MAEHR.** *J. Chem. Ed.,* 1985, vol. 62, 114-120
- Greene's Protective Groups in Organic Synthesis. John Wiley & Sons, Inc